# EUROPEAN PATENT APPLICATION

(11) **EP 4 635 976 A1**
(43) Date of publication of application: **22.10.2025**
(21) Application number: 24199974.7
(22) Date of filing: 12.09.2024
(51) Int. Cl.: C07K 14/705, A61K 39/00, A61K 40/00, A61K 40/32, A61K 47/64, C12N 5/0783, C12N 15/90

(54) **CONSTANT UNI-TABS**

(30) Priority: 19.04.2024 EP 24171426
(71) Applicant: Medigene Immunotherapies GmbH, 82152 Planegg-Martinsried (DE)
(72) Inventor: The designation of the inventor has not yet been filed
(74) Representative: Maiwald GmbH

(57) **Abstract**

The present invention covers a T cell receptor (TCP) comprising at least one epitope tag. An isolated polypeptide comprising at least one epitope tag and functional portion of the TCP is also described. Moreover, a multivalent TCP complex, nucleic acid molecules, vectors, cells as well as medical uses and pharmaceutical compositions relating to the TCP as well as a method of modifying a TCP or a fragment thereof comprising such that the TCP comprises at least one epitope tag are defined.

## Description

### FIELD OF THE INVENTION

The present invention relates to a T cell receptor (TCR) comprising at least one epitope tag and to a polypeptide comprising at least one epitope tag and a functional portion of the TCR. Further implicated are a multivalent TCR complex, a nucleic acid encoding a TCR, a vector comprising said nucleic acid, a cell expressing the TCR, and to the use of any of the aforementioned as a medicament, a screening agent, or for the treatment of cancer. In a further aspect, the present invention also relates to a pharmaceutical composition comprising any of the aforementioned. In yet another aspect, the present invention also relates to a method of modifying a TCR or a fragment thereof comprising the constant region of the TCR, said method comprising introduction of at least one epitope tag into the constant region of the TCR.

### BACKGROUND OF THE INVENTION

T lymphocytes are part of the adaptive immune response and originate from hematopoietic stem cells located in the bone marrow. T lymphocytes express a unique antigen binding receptor on their membrane, the T cell receptor (TCR), which recognizes antigens in association with major histocompatibility complex (MHC) molecules.

With their central role in the immune system, T cells typically provide protection from pathogens or malignant cells. Each T cell expresses a single form of a T cell receptor, a structure which is used by the T cell to recognize infected or altered cells.

The concept of immunotherapy is based on the specificity of the adaptive immune response for the recognition and elimination of pathogens as well as tumor cells. The aim of a successful immunotherapy is the manipulation or reprogramming of the patient's immune response in order to specifically target pathogen-infected cells or tumor cells for destruction by the immune system.

Therapeutic approaches used to reprogram the immune system in the treatment of infectious diseases and cancer include active immunotherapy comprising the use of vaccination strategies, including dendritic cell (DC) vaccines, as well as passive immunotherapy comprising the application of specific antibodies or genetically engineered lymphocytes or the adoptive transfer of T cells specifically recognizing target antigens displayed by pathogen-infected cells or cancers.

In the realm of clinical products, adoptively transferred rTCR T cells must undergo rigorous quality control procedures. These procedures serve a dual purpose: to confirm the expression and identity of the rTCR. Existing methods such as pHLA-multimer staining, utilization of Vß-specific antibodies, or the inclusion of tagging molecules like RQR8 have been employed, but each presents significant drawbacks.

The production of pHLA-multimers is intricate, with molecules often having short half-lives and binding affected by various factors, rendering standardization challenging. Similarly, utilizing TCR-Vß specific antibodies is complex due to the lack of commercial antibodies for all TCR-Vß variants. Moreover, since the human T cell repertoire already includes all TCR-Vß variants, staining with TCR-Vß antibodies not only labels the rTCR but also endogenous TCRs, complicating the evaluation of rTCR-T cell levels in the final product.

The alternative approach involving the expression of a separate molecule like RQR8 alongside the rTCR introduces additional sequences to the vector system. Furthermore, disparities in the expression levels between the rTCR and the tagging molecule may lead to inaccurate assessments of the rTCR-carrying T cells.

Hence, there is a pressing need for a system that enables direct and distinct tagging of the rTCR with minimal addition of amino acids, ensuring accurate evaluation and minimizing the complexities associated with current methods.

TCRs can be modified to introduce additional domains which aid in identification, tracking, purification and/or isolation of the respective molecule (tags), useful examples of which are epitope tags. Epitope tags are short stretches of amino acids that allow for binding of a specific antibody and therefore enable identification and tracking of the binding and movement of soluble TCRs or host cells within a patient's body or cultivated (host) cells. Therefore, epitope-tagged TCRs represent invaluable tools for both research and clinical applications in adoptive immune therapy.

In the realm of research and development, epitope-tagged TCRs enable precise visualization and tracking of recombinant TCRs, facilitating a deeper understanding of their behavior and functionality.

In the context of clinical adoptive immune therapy, epitope-tagged rTCRs provide significant benefits, allowing for the visualization, isolation and distinctive identification of specific T-cell populations, thereby greatly enhancing the precision and efficacy of therapeutic interventions. This technology enables the identification and enrichment of highly functional T cells that possess the ability to recognize and eliminate cancer cells or pathogens. Epitope-tagged rTCRs are also useful for product characterization and release. Additionally, epitope-tagged rTCRs offer the potential for real-time monitoring of T-cell localization and persistence *in vivo,* optimizing treatment protocols and improving patient outcomes. In addition to their primary applications, epitope-tagged rTCRs hold the potential to serve as a vital safety switch in adoptive immune therapy. They offer a promising avenue for the depletion of malfunctional or overly potent T cells that have been transferred to patients. Particularly, epitope-tagged rTCRs may eventually function as a safeguard, allowing clinicians to selectively remove undesirable T-cell populations, thereby minimizing the risk of adverse effects and optimizing the overall safety and efficacy of adoptive T-cell therapies.

Overall, the application of epitope-tagged rTCRs in adoptive immune therapy represents a powerful approach that combines both research insights and clinical benefits. By leveraging the capabilities of such tagged receptors, researchers and clinicians can advance understanding of T-cell behavior and unlock new opportunities for targeted and personalized therapeutic interventions.

However, in designing epitope-tagged rTCRs, one encounters various challenges. For instance, in order to minimize the probability for the development of immune responses in patients against artificially introduced epitopes in transferred TCRs, unique sequence compositions representing natural T cell receptors are needed. A further prerequisite on epitope tags is a low influence on the tertiary TCR structure, especially on the CDR regions, thereby allowing natural TCR-pMHC contact. Most importantly, epitope tags may not negatively affect TCR specificity, affinity and avidity for the respective target antigen and should have no negative impact on TCR expression as well as TCR interaction with co-receptor molecules or activation of the T cell.

### OBJECTIVES AND SUMMARY OF THE INVENTION

Against this background, one objective of the present invention was the provision of T cell receptors comprising at least one epitope tag.

The inventors surprisingly found that placing of tagging epitopes in certain adaptable positions within the constant chain of recombinant T cell receptors (rTCRs), particularly as secondary structures in the constant region, maintains the structural integrity and functionality of the rTCR.

The present invention thus relates, in a first aspect, to T cell receptors (TCRs) or fragments thereof comprising the constant region of the TCR, and further comprising, in said constant region, at least one epitope tag, wherein the insert position of the epitope tag is located in the alpha constant region (TRAC) and/or the beta constant region (TRBC).

The TCR according to the invention may be isolated and/or purified and may be soluble or membrane bound or a single chain TCR comprising one of the TCR constant regions.

In some embodiments, the insert position of the at least one epitope tag is comprised in the alpha constant region (TRAC) and the beta constant region (TRBC).

In some embodiments, the insert position of the at least one epitope tag is comprised in the alpha constant region (TRAC) or the beta constant region (TRBC).

In some embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and/or the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and/or the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

In some embodiments, the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149; and/or the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151; and/or the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 153.

In some embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 1, SEQ ID NO: 2, SEQ ID NO: 3, and SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 5, SEQ ID NO: 6, SEQ ID NO: 7, and SEQ ID NO: 8 of the TCR beta constant region; and/or the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 9, SEQ ID NO: 10, and SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 12, SEQ ID NO: 13, and SEQ ID NO: 14 of the TCR beta constant region; and/or the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15, SEQ ID NO: 16, and SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18, SEQ ID NO: 19, and SEQ ID NO: 20 of the TCR alpha constant region.

In some embodiments, the epitope tag is inserted by means of linker sequences, preferably short glycine-rich linker sequences, more preferably glycine-rich linker sequences consisting of 2 to 6, preferably 3 to 5 amino acids, more preferably comprising or consisting of the amino acid sequence GSG.

In some embodiments, the epitope tag comprises 4 to 17 amino acids, preferably 5 to 15 amino acids, more preferably 6 to 14 amino acids.

In some embodiments, the epitope tag, which can be recognized by a tagging antibody specific for the epitope tag, comprises an amino acid sequence having at least 80 %, preferably at least 85 %, more preferably at least 90 % sequence identity to an amino acid sequence selected from the group consisting of SEQ ID NO 47-53 and 154.

In some embodiments, the epitope tag, which can be recognized by a tagging antibody specific for the epitope tag, consists of an amino acid sequence having at least 80 %, preferably at least 85 %, more preferably at least 90 % sequence identity to an amino acid sequence selected from the group consisting of SEQ ID NO 47-53 and 154.

In various embodiments, integration of the epitope tag does not interfere with structural integrity and/or functionality of the TCR.

In some embodiments, the amino acid sequence of the TCR may comprise one or more phenotypically silent substitutions. In addition, the TCRs of the invention can be labelled. Useful labels are known in the art and can be coupled to the TCR or TCR variant using routine methods, optionally *via* linkers of various lengths. The term "label" or "labelling group" refers to any detectable label, but does not refer to the epitope tag, as herein described and defined. Additionally, or alternatively, the amino acid sequence may be modified to comprise a therapeutic agent or pharmacokinetic modifying moiety. The therapeutic agent may be selected from the group consisting of an immune effector molecule, a cytotoxic agent and a radionuclide. The immune effector molecule may for example be a cytokine. The pharmacokinetic modifying moiety may be at least one polyethylene glycol repeating unit, at least one glycol group, at least one sialyl group or a combination thereof.

The TCR, in particular a soluble form of the TCR according to the invention, can be modified by attaching additional functional moieties, *e.g.,* for reducing immunogenicity, increasing hydrodynamic size (size in solution) solubility and/or stability (*e.g.,* by enhanced protection to proteolytic degradation) and/or extending serum half-life. Other useful functional moieties and modifications include "suicide" or "safety switches" that can be used to shut off or turn on effector host cells carrying an inventive TCR in a patient's body.

TCRs with an altered glycosylation pattern are also envisaged herein.

It is also conceivable to add a drug or a therapeutic entity, such as a small molecule compound to the TCR, in particular to a soluble form of the inventive TCR.

In some embodiments, the TCR is of the single chain type, wherein the TCR α chain and the TCR β chain are linked by a linker sequence, with the at least one epitope tag being present in the alpha constant region (TRAC) and/or the beta constant region (TRBC). The single chain TCR mostly only contains either the TRAC or the TRBC, but can contain parts of both.

In another aspect, the present invention relates to an isolated polypeptide comprising a functional portion of the TCR of the present invention, including the at least one epitope tag.

In a further aspect, the present invention also relates to a multivalent TCR complex, comprising a least two TCRs according to the present invention and as described herein. In some such embodiments, at least one of said TCRs is associated with a therapeutic agent.

In another aspect, the present invention relates to a nucleic acid encoding a TCR according to the present invention or encoding the polypeptide according to the present invention.

A further aspect of the present invention relates to a plasmid or vector comprising a nucleic acid of the present invention as described herein. Preferably, the vector is an expression vector or a vector suitable for the transduction or transfection of cells, especially eukaryotic cells. The vector may be for example a retroviral vector, for example a gamma-retroviral or lentiviral vector.

Some embodiments refer to the inventive TCR expressed on an effector cell, especially on an immune effector cell as a functional polypeptide or functional multivalent polypeptide.

In a further aspect, the present invention thus also relates to a cell expressing the TCR of the present invention. The cell may be isolated or non-naturally occurring.

In a further aspect, the present invention relates to a cell comprising the nucleic acid as described herein or the plasmid or vector as described herein. More specifically, the cell may comprise:
a) an expression vector which comprises at least one nucleic acid as described herein, or
b) a first expression vector which comprises a nucleic acid encoding the alpha chain of the TCR as described herein, and a second expression vector which comprises a nucleic acid encoding the beta chain of a TCR as described herein.

In such embodiments b), the at least one epitope tag may be present in said alpha chain and/or in said beta chain.

The cell may be a peripheral blood lymphocyte (PBL) or a peripheral blood mononuclear cell (PBMC). Typically, the cell is an immune effector cell, especially a T cell. Other suitable cell types include gamma-delta T cells, NK cells and NK-like T cells.

Another aspect of the invention refers to a pharmaceutical composition comprising the epitope-tagged TCR of the present invention, the polypeptide of the present invention, the multivalent TCR complex of the present invention, the nucleic acid of the present invention, the vector of the present invention, or the cell of the present invention.

Typically, in some embodiments, the pharmaceutical composition comprises at least one pharmaceutically acceptable carrier.

The invention also relates to the corresponding epitope-tagged TCRs, the polypeptide of the present invention, the multivalent TCR complex of the present invention, the nucleic acid of the present invention, the vector of the present invention, or the cell of the present invention for use as a medicament, particularly the epitope-tagged TCRs of the present invention, for use as a screening agent, or for the use in the therapy of malignancies.

Specifically, in one such aspect, the present invention relates to the epitope-tagged TCRs of the present invention, the polypeptide of the present invention, the multivalent TCR complex of the present invention, the nucleic acid of the present invention, the vector of the present invention, or the cell of the present invention of the present invention, particularly the epitope-tagged TCRs of the present invention, for use as a medicament.

In another such aspect, the present invention relates to the TCR of the present invention, the polypeptide of the present invention, the multivalent TCR complex of the present invention, the nucleic acid of the present invention, the vector of the present invention, or the cell of the present invention of the present invention, particularly the TCRs of the present invention, for use as screening agent.

In a yet further aspect, the present invention also relates to the TCR of the present invention, the polypeptide of the present invention, the multivalent TCR complex of the present invention, the nucleic acid of the present invention, the vector of the present invention, or the cell of the present invention of the present invention, particularly the TCRs of the present invention, for use in the treatment of cancer.

Finally, in a further aspect, the present invention also relates to a method for modifying a TCR or a fragment thereof comprising the constant region of the TCR, the method comprising introduction, into the constant region, of at least one epitope tag, wherein the insert position of the epitope tag is located in the alpha constant region (TRAC) and/or the beta constant region (TRBC).

### FIGURE LEGENDS

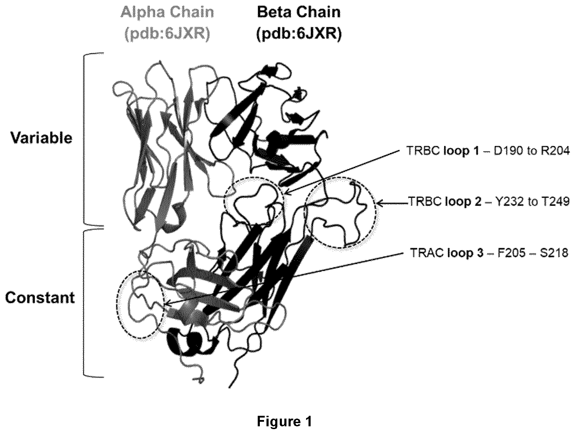

**Figure 1** **Shows the positioning of the UNI-TABS epitope in the constant region of a rTCR.** Two loops in the TRBC (Loop 1 & 2) and one loop in the TRAC (Loop 3) in the constant region of a TCR were identified for insertion of the UNI-TABs epitope.

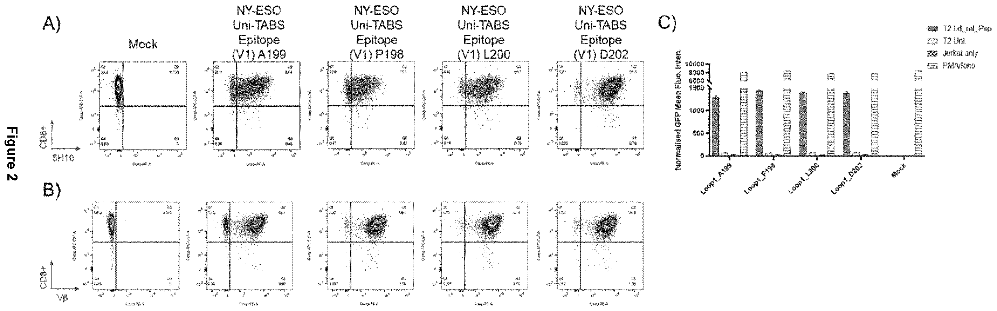

**Figure 2A-C**show the analysis results of expression and functionality of UNI-TAB-TCR inserted with the epitope tag set forth in SEQ ID NO: 47 in TRBC-loop 1 in Jurkat 76 -/-CD8+ ieGFP. The binding analysis of anti-TRBC MDG827 (2A) and anti-TRBV12-3/4 (Beckman Coulter; #Cat PN IM1148) (2B) antibody to UNI-TABS carrying NY-ESO specific TCR (T11.8-10-17) at position A199, A198, L200, and D202 in loop 1 of TRBC on Jurkat biosensor cells. Flow cytometric evaluation of Jurkat biosensor cells transduced with respective Uni-TABS (epitope tag set forth in SEQ ID NO: 47 flanked with GSG linker sequences as set forth in SEQ ID NO: 54 herein denoted "V1") modified cells **(2C):** Functional evaluation of Jurkat biosensor cells expressing Uni-TABS epitope V1 (epitope tag set forth in SEQ ID NO: 47 flanked with GSG linker sequences as set forth in SEQ ID NO: 54) modified NY-ESO specific TCR (T11.8-10-17). For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

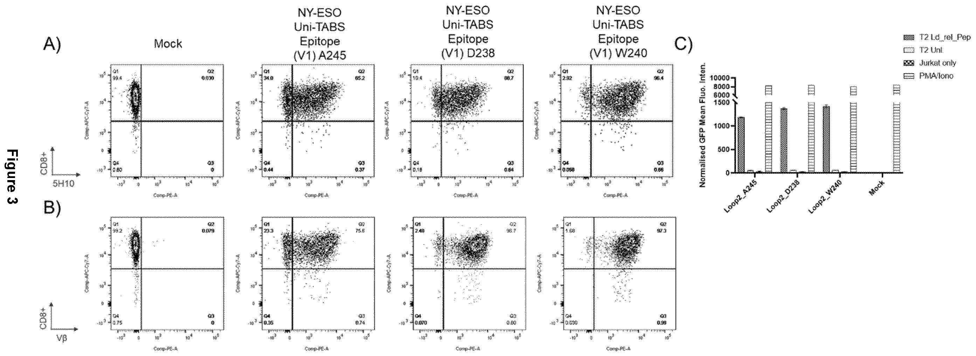

**Figures 3A-C** show the analysis results of expression and functionality of UNI-TAB-TCR inserted with the epitope tag set forth in SEQ ID NO: 47 in TRBC-loop 2 in Jurkat 76 -/-CD8+ ieGFP. The binding analysis of anti-TRBC MDG827 **(3A)** and anti-TRBV12-3/4 (Beckman Coulter; #Cat PN IM1148) **(2B)** antibody to UNI-TABS carrying NY-ESO specific TCR (T11.8-10-17) at position A245, D238, & W240 in loop 2 of TRBC on Jurkat biosensor cells. Flow cytometric evaluation of Jurkat biosensor cells transduced with respective Uni-TABS (epitope tag set forth in SEQ ID NO: 47 flanked with GSG linker sequences as set forth in SEQ ID NO: 54 denoted "V1") modified cells **(3C):** Functional evaluation of Jurkat biosensor cells expressing Uni-TABS epitope V1 (epitope tag set forth in SEQ ID NO: 47 flanked with GSG linker sequences as set forth in SEQ ID NO: 54) modified NY-ESO specific TCR (T11.8-10-17). For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

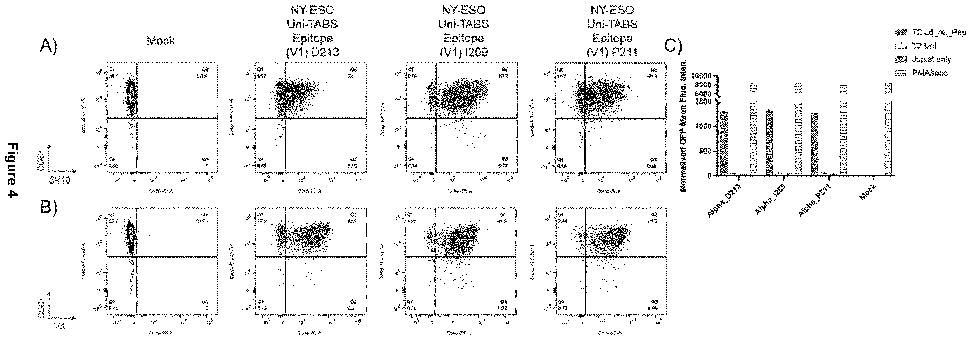

**Figures 4A-C**show the analysis results of expression and functionality of UNI-TAB-TCR inserted with the epitope tag set forth in SEQ ID NO: 47 in TRAC-loop 3 in Jurkat 76 -/-CD8+ ieGFP. The binding analysis of anti-TRBC MDG827 (4A) and anti-TRBV12-3/4 (Beckman Coulter; #Cat PN IM1148) **(4B)** antibody to UNI-TABS carrying NY-ESO specific TCR (T11.8-10-17) at position D213, I209, & P211 in loop 3 of TRAC on Jurkat biosensor cells. Flow cytometric evaluation of Jurkat biosensor cells transduced with respective Uni-TABS (epitope tag set forth in SEQ ID NO: 47 flanked with GSG linker sequences as set forth in SEQ ID NO: 54 denoted "V1") modified cells **(4C):** Functional evaluation of Jurkat biosensor cells expressing Uni-TABS epitope V1 (epitope tag set forth in SEQ ID NO: 47 flanked with GSG linker sequences as set forth in SEQ ID NO: 54) modified NY-ESO specific TCR (T11.8-10-17) . For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

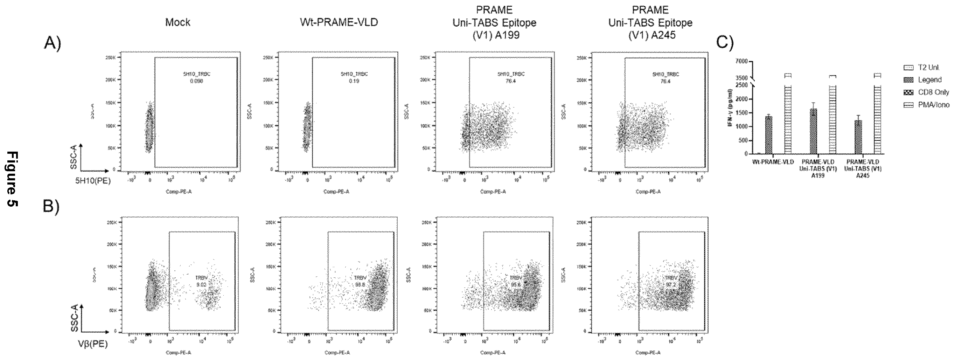

**Figures 5A-C**show the analysis results of expression and functionality of UNI-TAB-TCR in PBMC. The binding analysis of anti-TRBC MDG827 **(5A)** and anti-TRBV9 (Beckman Coulter; #Cat IM2002) **(5B)** antibody to PRAME specific TCR (T4.8-1-29) on PRAME-Uni-TABS-TCR transduced PBMCs. Flow cytometric evaluation of PBMCs, transduced with various WT or Uni-TABS V1 (epitope tag set forth in SEQ ID NO: 47 flanked by GSG linker sequences as set forth in SEQ ID NO: 54) -modified TCRs in loop1 (A199) & loop2 (A245). **(5C):** Functional evaluation of CD8-enriched PBMCs expressing wildtype or Uni-TABS epitope V1 (epitope tag set forth in SEQ ID NO: 47 flanked by GSG linker sequences as set forth in SEQ ID NO: 54) modified PRAME specific TCR (T4.8-1-29). For the experiment, CD8⁺ cells were co-cultured with (T2_Ld_rel_Pep) or without (T2 unl.) relevant peptide-loaded APC (T2) in an E:T ratio of 1:2 along with controls (negative control: CD8 only; positive control for complete activation: PMA/lonomycin treated) and subsequently INF-γ release was determined using ELISA, PMA/lono: PMA & lonomycin treated.

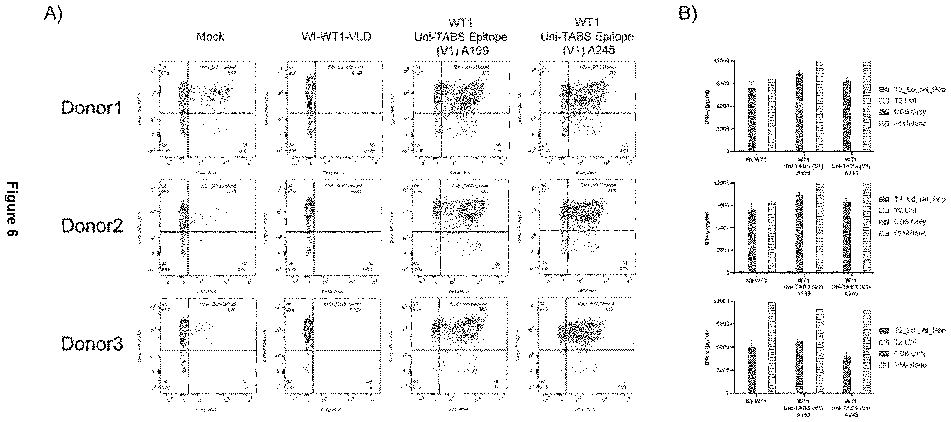

**Figures 6A and 6B** show the analysis results of expression and functionality of UNI-TAB-TCR in PBMC. Binding analysis of anti-TRBC MDG827 **(6A)** antibody to WT1 specific TCR on WT1-Uni-TABS-TCR transduced PBMCs of three donors. Flow cytometric evaluation of PBMCs, transduced with various WT or Uni-TABS V1 (epitope tag set forth in SEQ ID NO: 47 flanked by GSG linker sequences as set forth in SEQ ID NO: 54) WT1 specific TCR modified in loop1 (A199) & loop2 (A245); **(6B):** Functional evaluation of CD8-enriched PBMCs expressing wildtype or Uni-TABS epitope V1 (epitope tag set forth in SEQ ID NO: 47 flanked by GSG linker sequences as set forth in SEQ ID NO: 54) modified WT1 specific TCR. For the experiment, CD8⁺ cells were co-cultured with (T2_Ld_rel_Pep) or without (T2 unl.) relevant peptide-loaded APC (T2) in an E:T ratio of 1:2 along with controls (negative control: CD8 only; positive control for complete activation: PMA/lonomycin treated) and subsequently INF-y release was determined using ELISA, PMA/lono: PMA & lonomycin treated.

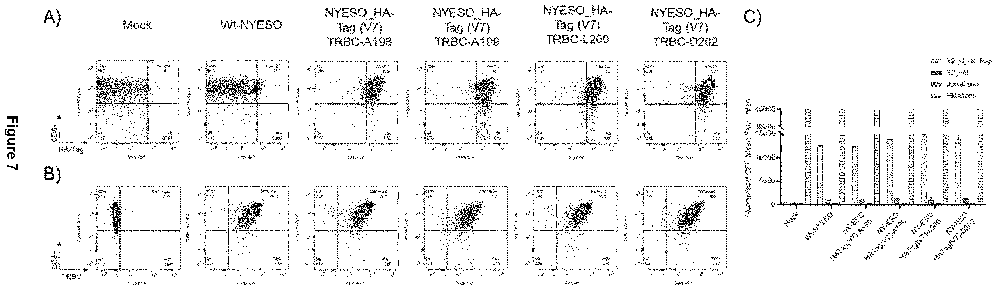

**Figures 7A-C**show the analysis results of expression and functionality NY-ESO specific TCR inserted with HA-Tag (epitope tag set forth in SEQ ID NO: 53 flanked by GSG linker sequences as set forth in SEQ ID NO: 60 herein denoted "V7") in TRBC-loop 1 in Jurkat 76 -/-CD8+ ieGFP cells. Binding analysis of anti-HA-Tag antibody **(7A)** & anti-TRBV12-3/4 (Beckman Coulter; #Cat PN IM1148) **(7B)** to NY-ESO specific TCR (T11.8-10-17) on Jurkat biosensor. Flow cytometric evaluation of Jurkat biosensor cells transduced with various NY-ESO specific WT or HA-Tag epitope inserted in TRBC-loop1 at position A198, A199, L200, & D202; (7C): Functional evaluation of Jurkat biosensor cells expressing wildtype or HA-Tag modified NY-ESO specific TCR (T11.8-10-17). For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

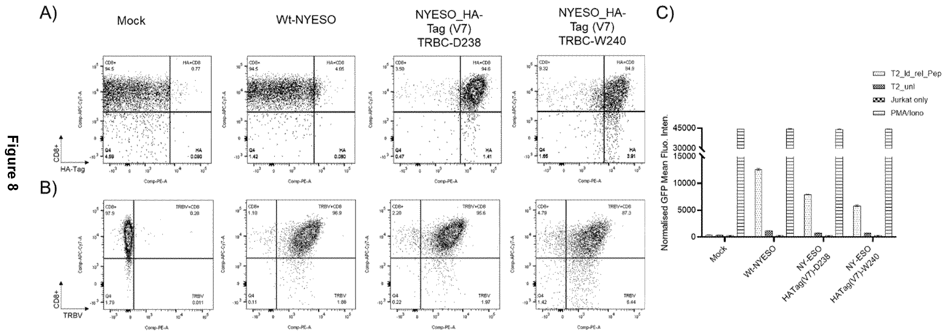

**Figures 8A-C**show the analysis results of expression and functionality NY-ESO specific TCR inserted with HA-Tag (epitope tag set forth in SEQ ID NO: 53 flanked by GSG linker sequences as set forth in SEQ ID NO: 60 denoted "V7") in TRBC-loop 2 in Jurkat 76 -/-CD8+ ieGFP cells. Binding analysis of anti-HA-Tag antibody **(8A)** & anti-TRBV12-3/4 (Beckman Coulter; #Cat PN IM1148) **(8B)** NY-ESO specific TCR (T11.8-10-17) on Jurkat biosensor. Flow cytometric evaluation of Jurkat biosensor cells transduced with various NY-ESO specific WT or HA-Tag epitope inserted in TRBC loop2 at position D238 & W240; **(8C):** Functional evaluation of Jurkat biosensor cells expressing wildtype or HA-Tag modified NY-ESO specific TCR (T11.8-10-17). For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

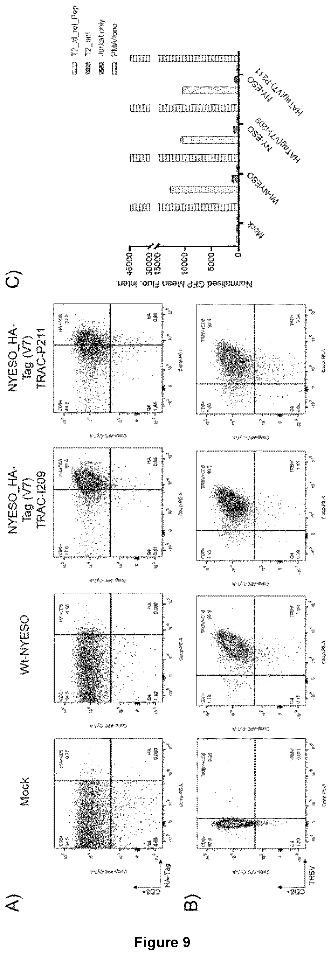

**Figures 9A-C**show the analysis results of expression and functionality NY-ESO specific TCR inserted with HA-Tag (epitope tag set forth in SEQ ID NO: 53 flanked by GSG linker sequences as set forth in SEQ ID NO: 60 denoted "V7") in TRAC-loop 3 in Jurkat 76 -/-CD8+ ieGFP cell. Binding analysis of anti-HA-Tag antibody **(9A)** & anti-TRBV12-3/4 (Beckman Coulter; #Cat PN IM1148) **(9B)** NY-ESO specific TCR (T11.8-10-17) on Jurkat biosensor. Flow cytometric evaluation of Jurkat biosensor cells transduced with various NY-ESO specific WT or HA-Tag epitope inserted in TRAC loop3 at position I209 & P211; **(9C):** Functional evaluation of Jurkat biosensor cells expressing wildtype or HA-Tag modified NY-ESO specific TCR (T11.8-10-17). For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, PMA/lono: PMA & lonomycin treated.

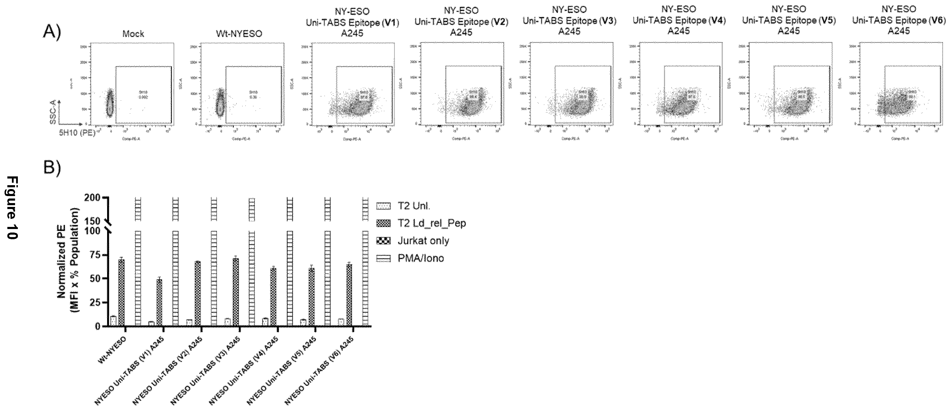

**Figures 10A and 10B** show the analysis results of expression and functionality of shortened UNI-TAB-epitopes. Binding analysis of anti-TRBC MDG827 antibody to NY-ESO specific TCR (T11.8-10-17) on Jurkat biosensor **(10A).** Flow cytometric evaluation of Jurkat biosensor cells transduced with various WT or various Uni-TABS version (epitopes tag set forth in SEQ ID NOs 47-52 flanked by GSG linker sequences as set forth in SEQ ID NOs 54-59 denoted "V1", "V2", "V3", "V4", "V5", and "V6", respectively) modified NY-ESO TCR inserted in TRBC loop 2 at A245 position; **(10B):** Functional evaluation of Jurkat biosensor cells expressing wildtype or Uni-TABS epitopes set forth in SEQ ID NOs 47-52 flanked by GSG linker sequences as set forth in SEQ ID NOs 54-59 herein denoted "V1, V2, V3, V4, V5, and V6", respectively, modified NY-ESO specific TCR (T11.8-10-17). For the experiment, Jurkat biosensor cells were co-cultured with or without relevant peptide-loaded APC (T2) and subsequently GFP signal was analyzed as a read for the TCR activation.MFI: Mean Fluorescent Intensity, PMA/lono: PMA & lonomycin treated.

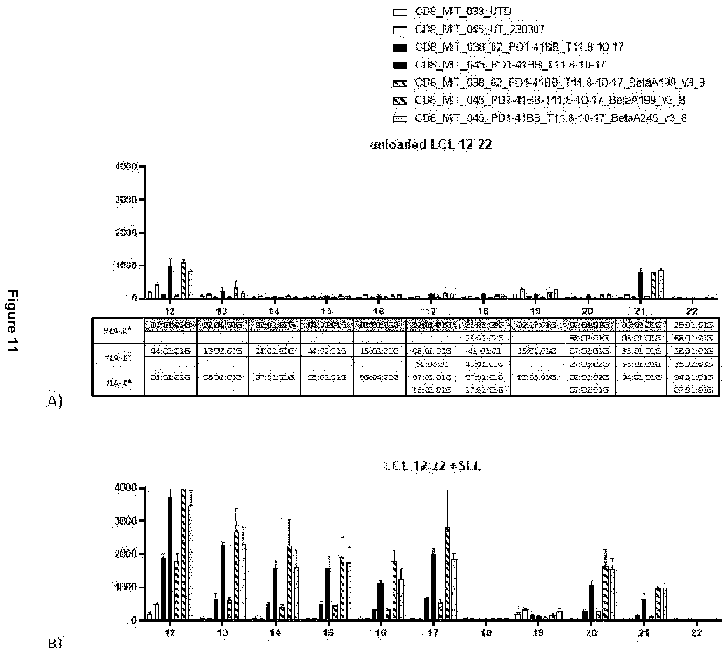

**Figures 11A and 11B** show the LCL Library analysis of UNI-TAB-variant TCRs. IFN-g-ELISA results of co-culture of UNI-TAB TCRs with given LCL library cell line (including HLA typing) **(11A).** IFN-g-ELISA results of co-culture of UNI-TAB TCRs with given LCL library cell line loaded with relevant peptide (11**B).** BetaA199: the epitope tag set forth in SEQ ID NO: 47 inserted in loop1 at position A199 flanked by GSG linker sequences; BetaA245: the epitope tag set forth in SEQ ID NO: 47 epitope inserted in loop2 at position A245 flanked by GSG linker sequences.

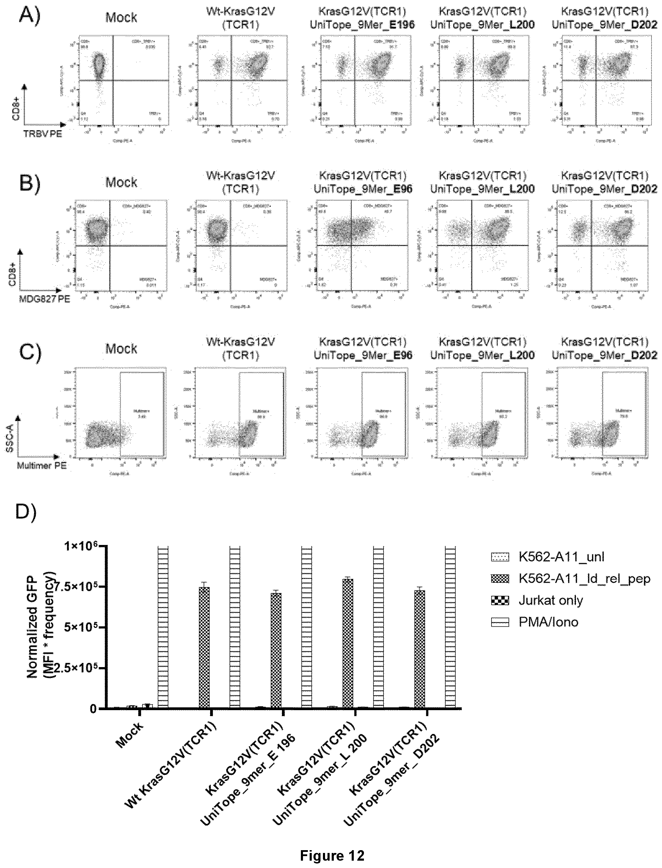

**Figures 12A-D**show results of analysis of expression and functionality of rTCR inserted with 9mer-epitope tag ((referred to in the Figure as "UniTope") with linker (GSGGEVPKDRFSGSG)) in TRBC-loop 1 in Jurkat biosensor cells: flow cytometric evaluation demonstrates the successful staining of the Jurkat biosensorcells transduced with KRASG12V-TCR1 when inserted with 9-mer epitope tag at position E96 or L200 or D202 in loop 1 of TRBC using anti-TRBV5-5-PE antibody **(A),** MDG827-PE antibody **(B),** and Multimer-PE **(C).** The 9-mer epitope tag-modified KRASG12V-TCR1 transduced Jurkat biosensorcells is detected specifically by the MDG827 antibody and does not stain untransduced as well as wild type KrasG12V-TCR1 whereas anti-TRBV5-5 and Multimer stained all transduced cells; **(D):** functional evaluation shows no significant difference in TCR activation of Jurkat biosensor cells expressing 9-mer-epitope tag modified KRASG12V-TCR1 specific TCR (T47.8-041-071) compared to wild type KRASG12V-TCR1. For the experiment, untransduced & transduced Jurkat biosensor cells were co-cultured with (K562-A11_Ld_rel_Pep) or without relevant peptide-loaded (K562-A11 unl.) APC (K562-A11) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, frequency: GFP positive population, PMA/lono: PMA & lonomycin treated, wild type: TCR without UniTope (epitope tag) sequence.

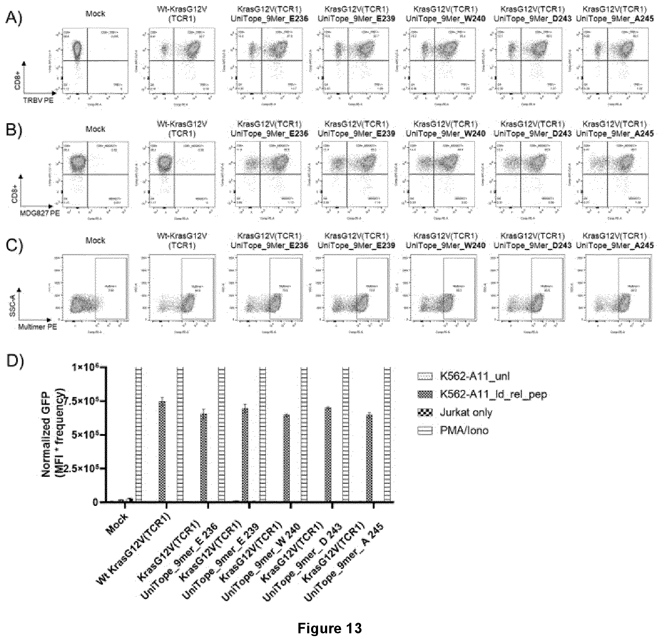

**Figures 13A-D**show results of analysis of expression and functionality of rTCR inserted with 9mer-epitope tag ((referred to in the Figure as "UniTope") with linker (GSGGEVPKDRFSGSG)) in TRBC-loop 2 in biosensor cells: flow cytometric evaluation shows the successful staining of the Jurkat biosensor cells transduced with KRASG12V-TCR1 when inserted with 9-mer epitope tag at position E236, E239, W240, D243, and A245 in loop 2 of TRBC using anti-TRBV5-5-PE antibody **(A),** MDG827-PE antibody **(B),** and Multimer-PE **(C);** the 9-mer epitope tag-modified KRASG12V-TCR1 transduced Jurkat biosensor cells is detected specifically by the MDG827 antibody and does not stain untransduced as well as wild type KRASG12V-TCR1 whereas anti-TRBV5-5 & Multimer stained all transduced cells; (D): functional evaluation shows no significant difference in TCR activation of Jurkat biosensor cells expressing 9-mer- epitope tag modified KRASG12V-TCR1 specific TCR (T47.8-041-071) compared to wild-type KRASG12V-TCR1. For the experiment, untransduced and transduced Jurkat biosensor cells were co-cultured with (K562-A1 1_Ld_rel_Pep) or without relevant peptide-loaded (K562-A1 1 unl.) APC (K562-A11) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, frequency: GFP positive population, PMA/lono: PMA & lonomycin treated, wild type: TCR without UniTope (epitope tag) sequence.

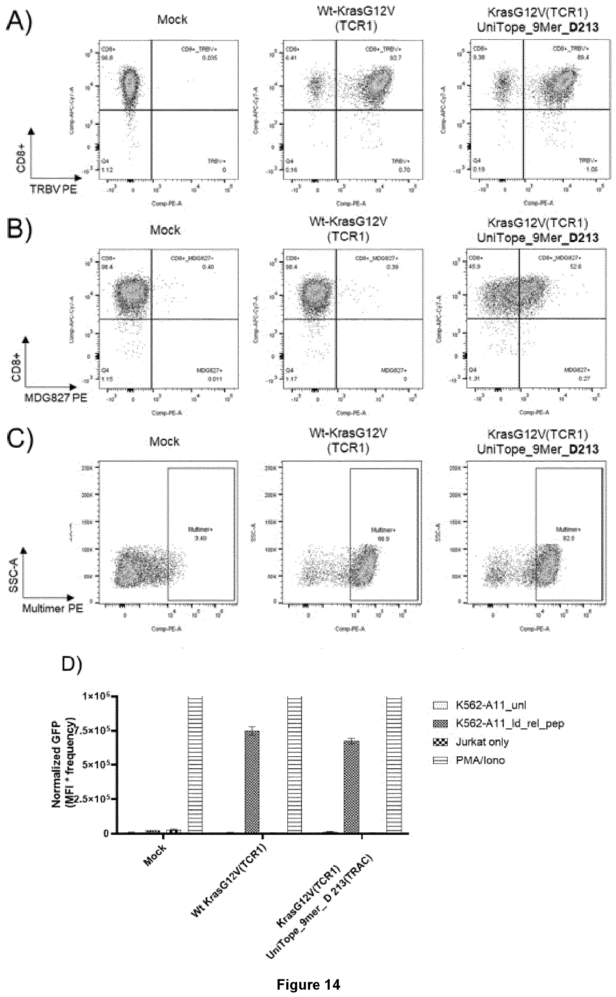

**Figures 14A-D**show results of analysis of expression and functionality of rTCR inserted with 9mer-epitope tag ((referred to in the Figure as "UniTope") with linker (GSGGEVPKDRFSGSG)) in TRBC-loop 3 in Jurkat biosensor cells: flow cytometric evaluation shows the successful staining of the Jurkat biosensor cells transduced with KRASG12V-TCR1 when inserted with 9-mer epitope tag at position D213 in loop 3 of TRBC using anti-TRBV5-5-PE antibody **(A),** MDG827-PE antibody **(B),** and Multimer-PE **(C);** the 9-mer epitope tag-modified KRASG12V-TCR1 transduced Jurkat biosensorcells is detected specifically by the MDG827 antibody and does not stain untransduced as well as wild type KRASG12V-TCR1 whereas anti-TRBV5-5 and Multimer stained all transduced cells; (D): functional evaluation shows no significant difference in TCR activation of Jurkat biosensor cells expressing 9-mer- epitope tag modified KRASG12V-TCR1 specific TCR (T47.8-041-071) compared to wild type KRASG12V-TCR1. For the experiment, untransduced & transduced Jurkat biosensor cells were co-cultured with (K562-A11_Ld_rel_Pep) or without relevant peptide-loaded (K562-A11 unl.) APC (K562-A11) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, frequency: GFP positive population, PMA/lono: PMA & lonomycin treated, wild type: TCR without UniTope (epitope tag) sequence.

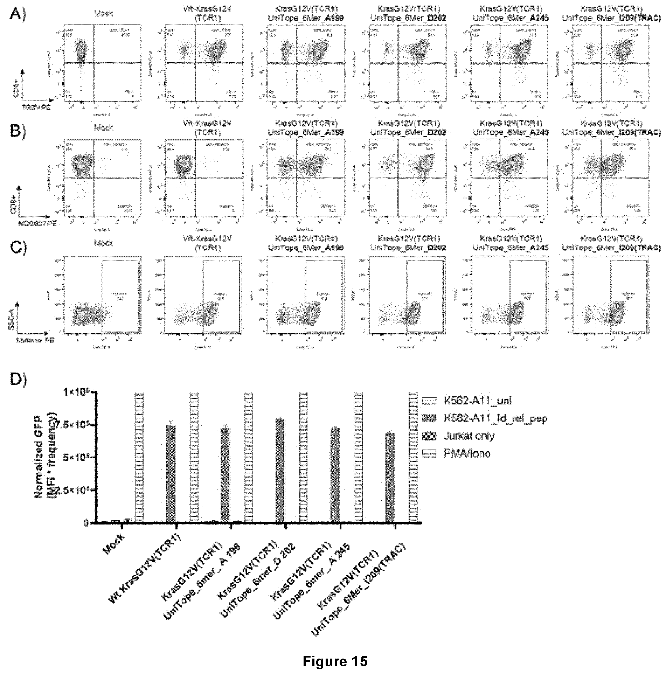

**Figures 15A-D**show results of analysis of expression and functionality of rTCR inserted with 6mer-epitope tag ((referred to in the Figure as "UniTope") with linker (GSGEVPKDRGSG)) in TRBC in Jurkat biosensor cells: flow cytometric evaluation shows the successful staining of the Jurkat biosensor cells transduced with KRASG12V-TCR1 inserted with 6-mer epitope tag with linker in loop1 (Position A199 or D202) or loop2 (position A245) or loop3 (position I209) in TCR constant chain on Jurkat biosensor cells using anti-TRBV5-5-PE antibody **(A),** MDG827-PE antibody **(B),** and Multimer-PE **(C).** The 6-mer epitope tag modified KRASG12V-TCR1 transduced Jurkat biosensor cells is detected specifically by the MDG827 antibody and does not stain untransduced as well as wild type KRASG12V-TCR1 whereas anti-TRBV5-5 & Multimer stained all transduced cells; **(D):** functional evaluation shows no significant difference in TCR activation of Jurkat biosensor cells expressing 6-mer-epitope tag modified KRASG12V-TCR1 specific TCR (T47.8-041-071) compared to wild type KRASG12V-TCR1. For the experiment, untransduced & Transduced Jurkat biosensor cells were co-cultured with (K562-A11_Ld_rel_Pep) or without relevant peptide-loaded (K562-A11 unl.) APC (K562-A11) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, frequency: GFP positive population, PMA/lono: PMA & lonomycin treated, wild type: TCR without UniTope (epitope tag) sequence.

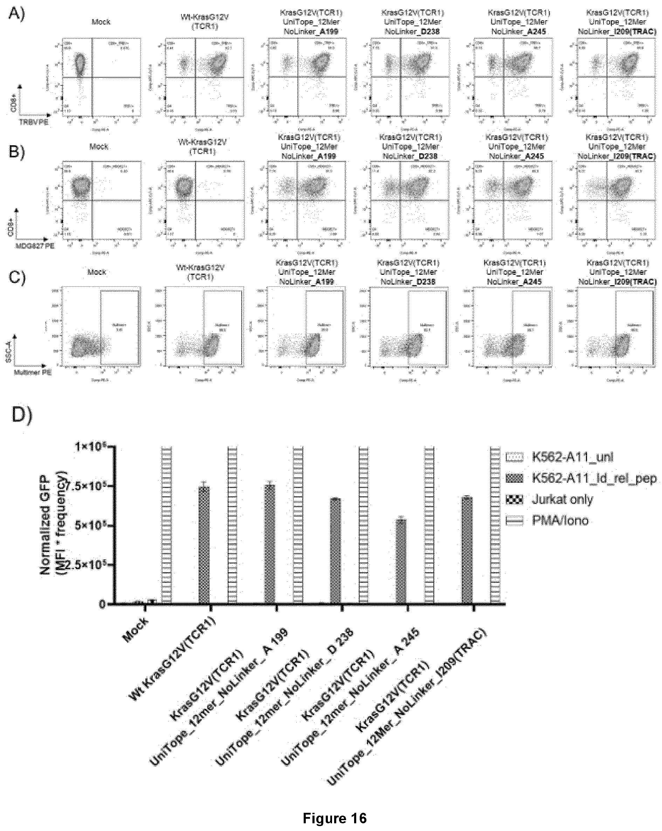

**Figures 16A-D**show results of analysis of expression and functionality of rTCR inserted with 12mer-epitope tag without 'GSG' linker ((referred to in the Figure as "UniTope" (DKGEVPKDRFSA)) in TRBC in Jurkat biosensor cells: flow cytometric evaluation shows the successful staining of Jurkat biosensor cells transduced with KRASG12V specific TCR1 (T47.8-041-071) containing 12mer-epitope tag without 'GSG' linker in loop1 (Position A199) or loop2 (position D238 or A245) or loop3 (position I209) in TCR constant chain on Jurkat biosensor cells using anti-TRBV5-5-PE antibody **(A),** MDG827-PE antibody **(B),** and Multimer-PE **(C);** the 12-mer without linker epitope tag-modified KRASG12V-TCR1 transduced Jurkat biosensor cells is detected specifically by the MDG827 antibody and does not stain untransduced as well as wild type KrasG12V-TCR1 whereas anti-TRBV5-5 & Multimer stained all transduced cells; **(D):** functional evaluation shows no significant difference in TCR activation of Jurkat biosensor cells expressing 12mer-epitope tag without 'GSG' linker modified KRASG12V-TCR1 specific TCR (T47.8-041-071) compared to wild type KRASG12V-TCR1. For the experiment, untransduced and transduced Jurkat biosensor cells were co-cultured with (K562-A1 1_Ld_rel_Pep) or without relevant peptide-loaded (K562-A1 1 unl.) APC (K562-A11) along with controls (negative control: Jurkat only; positive control for complete activation: PMA/lonomycin treated) and subsequently eGFP signal was analyzed by flow cytometry as a read for the TCR activation. MFI: Mean Fluorescent Intensity, frequency: GFP positive population, PMA/lono: PMA & lonomycin treated, wild type: TCR without UniTope (epitope tag) sequence.

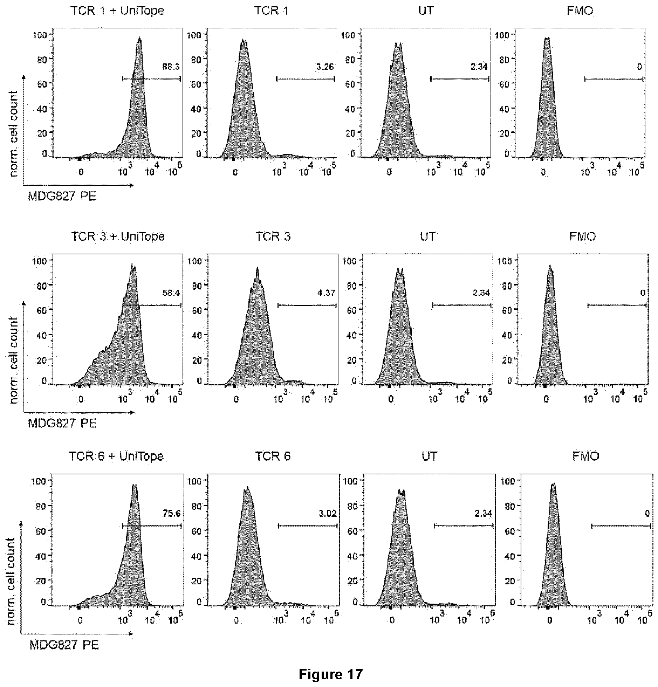

**Figure 17** shows the successful cell surface expression results of the 9mer-epitope tag (referred to in the Figure as "UniTope") with linker (GSGGEVPKDRFSGSG) inserted into KRASG12V specific TCRs (TCR 1 / TCR 3 / TCR 6; referred to in the Figure as "TCR 1 / TCR 3 / TCR 6 + UniTope") following retroviral transduction of CD8⁺ T cells. Untransduced CD8⁺ T cells (UT) were prepared in the same manner and used as controls. To assess transduction efficiency and expression levels of the transgenes, T cell samples were stained with anti-UniTope MDG827, anti-CD3 and anti-CD8 antibodies and analyzed by flow cytometry. The epitope tag is detected specifically by the MDG827 antibody and does not label TCR 1 / TCR 3 / TCR 6 -bearing CD8⁺ T cells above UT background. Populations shown are pre-gated on live single CD3⁺CD8⁺ T cells. A fluorescence minus one (FMO) control containing all antibodies but MDG827 was performed additionally.

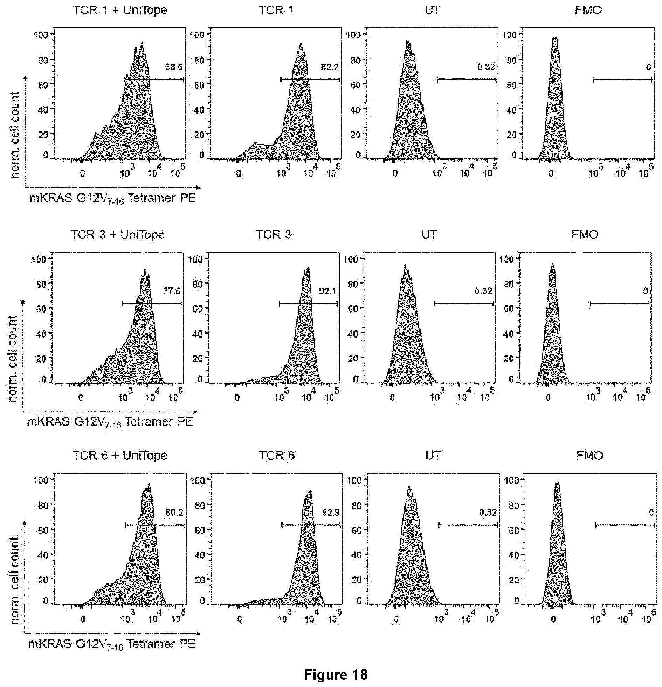

**Figure 18** shows the successful cell surface expression results of the 9mer-epitope tag (referred to in the Figure as "UniTope") with linker (GSGGEVPKDRFSGSG) inserted into KRASG12V specific TCRs (TCR 1 / TCR 3 / TCR 6; referred to in the Figure as "TCR 1 / TCR 3 / TCR 6 + UniTope") following retroviral transduction of CD8⁺ T cells. TCR 1 / TCR 3 / TCR 6 + UniTope tag and TCR 1 / TCR 3 / TCR 6 are bound by a mKRAS7-16 G12V (10-mer) HLA-A*11:01 tetramer. To assess transduction efficiency and expression levels of the transgenes, T cell samples were stained with anti-CD3 and anti-CD8 antibodies, mKRAS7-16 G12V (10-mer) HLA-A*11:01 tetramer and analyzed by flow cytometry. Untransduced CD8⁺ T cells (UT) were stained and analyzed in parallel as control. Populations shown are pre-gated on live single CD3⁺CD8⁺ T cells. Both TCR 1 / TCR 3 / TCR 6 and TCR 1 / TCR 3 / TCR 6 + epitope tag (referred to in the Figure as "TCR 1 / TCR 3 / TCR 6 + UniTope") were successfully surface-expressed in human CD8⁺ T cells, as demonstrated by tetramer staining. Thus, correct expression and folding of TCR 1 / TCR 3 / TCR 6 + epitope tag were verified. A fluorescence minus one (FMO) control containing all antibodies but not the tetramer was performed additionally.

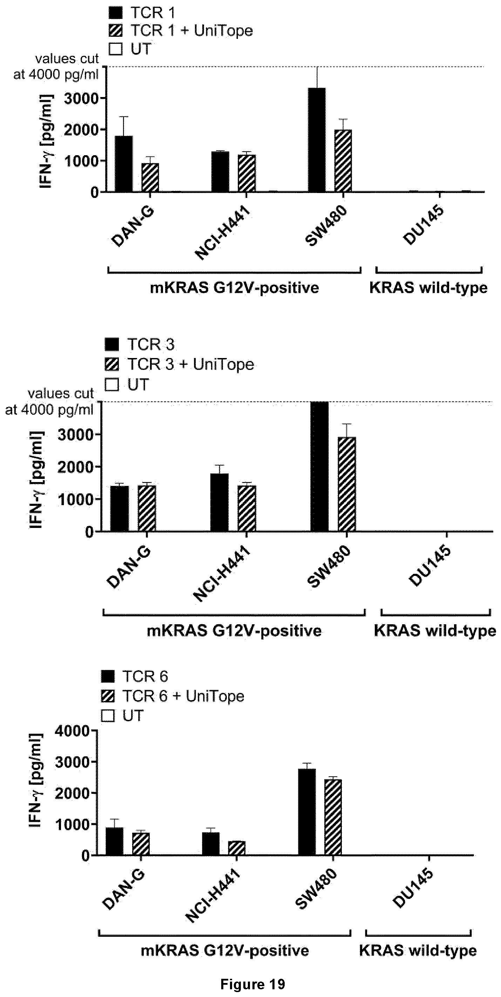

**Figure 19** shows comparably high and specific IFN-γ secretion by T cells transduced with KRASG12V specific TCRs (TCR 1 / TCR 3 / TCR 6 and TCR 1 / TCR 3 / TCR 6 + Unitope (GSGGEVPKDRFSGSG)) in response to tumor cells. Tumor cell lines derived from various tumor indications expressing either endogenous levels of mKRAS G12V antigen (DAN-G, NCI-H441, SW480) or KRAS wild type (DU145) were selected for this co-culture experiment. The co-culture experiment was set up by using the described tumor cell lines and TCR-transduced CD8⁺ T cells (TCR 1 / TCR 3 / TCR 6) and TCR + UniTope transduced CD8+ T cells (referred to in the Figure as "TCR 1 / TCR 3 / TCR 6 + UniTope"). Untransduced (UT) CD8⁺ T cells served as negative control. IFN-γ secretion was assessed 20 h after setting up the co-culture. The mean value of triplicates is shown with standard deviations. One representative experiment is shown

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

The terms "functional TCR" and "functional TCR types" refer to TCRs that contain at least TCR variable chains that are expressed on T cells. Correspondingly, the terms "functional TCR Vα chains" or "functional TCR Vβ chains" or "functional TCR variable chains" relate to TCR variable chains that are expressed on T cells. That means that this term does not include TCR variable chains that are not expressed, such as pseudogenes, i.e., genes with frameshift mutations or defects in the recombination signal. The annotation whether a TCR variable chain is functional or rather a pseudogene can be found for example in Folch ("The Human T cell Receptor Beta Variable (TRBV) Genes", Folch Géraldibem Lefranc Maire-Paule, Exp Clin Immunogenet 2000;17:42-54) or Su et al. (Chen Su and Masatoshi Nei, Mol.- Biol. Evol. 2001 ;18(4):505-513).

Technical terms are used by their common sense or meaning to the person skilled in the art. If a specific meaning is conveyed to certain terms, definitions of terms will be given in the following in the context of which the terms are used.

### TCR background

A TCR is composed of two different and separate protein chains, namely the TCR alpha (α) and the TCR beta (b) chain. The TCR α chain comprises variable (V), joining (J) and constant (C) regions. The TCR b chain comprises variable (V), diversity (D), joining (J) and constant (C) regions. The rearranged V(D)J regions of both the TCR α and the TCR β chain contain hypervariable regions (CDR, complementarity determining regions), among which the CDR3 region determines the specific epitope recognition. At the C-terminal region both TCR α chain and TCR β chain contain a hydrophobic transmembrane domain and end in a short cytoplasmic tail.

Typically, the TCR is a heterodimer of one α chain and one β chain. This heterodimer can bind to MHC molecules presenting a peptide.

The term *"variable TCR a region"* or *"TCR a variable chain"* in the context of the invention refers to the variable region of a TCR α chain. The term *"variable TCR β region"* or *"TCR* β *variable chain"* in the context of the invention refers to the variable region of a TCR β chain.

The TCR loci and genes are named using the International Immunogenetics (IMGT) TCR nomenclature (IMGT Database, www. IMGT.org; Giudicelli, V., et al.,IMGT/LIGM-DB, the IMGT® comprehensive database of immunoglobulin and T cell receptor nucleotide sequences, Nucl. Acids Res., 34, D781-D784 (2006). PMID: 16381979; T cell Receptor Factsbook, LeFranc and LeFranc, Academic Press ISBN 0-12- 441352-8).

### Epitope-tagged TCR

A first aspect of the invention relates to a TCR or a fragment thereof comprising the constant region of the TCR, and further comprising, in said constant region, at least one epitope tag, wherein the insert position of the epitope tag is located in the alpha constant region (TRAC) and/or the beta constant region (TRBC).

Epitope tags are generally known in the art and aid in identification, tracking, purification and/or isolation of the respective molecule (tags). Epitope tags are short stretches of amino acids that allow for binding of a specific antibody and therefore enable identification and tracking of the binding and movement of soluble TCRs or host cells within the patient's body or cultivated (host) cells. Detection of the epitope tag, and hence, the tagged TCR, can be achieved using a number of different techniques. Tags can further be employed for stimulation and expansion of host cells carrying the respective TCR by cultivating the cells in the presence of binding molecules (antibodies) specific for said tag.

In some embodiments, the insert position of the at least one epitope tag is comprised in the alpha constant region (TRAC) and the beta constant region (TRBC). Consequently, it is envisaged that a TCR (or fragment thereof, as herein defined) according to suchlike embodiments of the present invention comprises at least two epitope tags, as herein described and defined, such as 3, 4, 5 or 6 or more epitope tags. In some other embodiments, the insert position of the at least one epitope tag is comprised in the alpha constant region (TRAC) or the beta constant region (TRBC). Also in suchlike embodiments it is possible that the TCR (or fragment thereof, as herein defined) of the present invention comprises, in its alpha constant region or in its beta constant region, more than one epitope tag, as herein described and defined, such as 2, 3, 4, 5 or 6 or more epitope tags.

Particularly, in some embodiments, it is envisaged that the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR.

In some other embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR.

In various embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

Thus, in some embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and/or
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and/or
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

For instance, in some embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; or
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; or
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

In some other embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

Thus, in suchlike embodiments, the TCR (or fragment thereof, as herein defined) comprises at least three epitope tags, as herein described and defined.

In various other embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR.

Consequently, in suchlike embodiments, the TCR (or fragment thereof, as herein defined) of the present invention comprises at least two epitope tags, as herein described and defined.

In some other embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

In other embodiments, the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and
the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

In some embodiments, the insert position is located at any position within the amino acid sequence set forth in SEQ ID NO: 145 and is located within the alpha constant region of the TCR; and/or
the insert position is located at any position within the amino acid sequence set forth in SEQ ID NO: 146 and is located within the beta constant region of the TCR; and/or
the insert position is located at any position within the amino acid sequence set forth in SEQ ID NO: 147 and is located within the beta constant region of the TCR.

In various embodiments, the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 149.

In various embodiments, the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 151.

In various embodiments, the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.

In various such embodiments, the amino acid sequence that is preceded by certain amino acid sequences, as defined herein above, and/or followed by certain amino acid sequences, as herein defined above, is from 12 to 20, preferably from 14 to 18, such as 14, 15, 16, 17, or 18 amino acids of length, most preferably 14, 15, or 18 amino acids of length.

For instance, in various embodiments, wherein the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149, the amino acid sequence that is preceded and/or followed as defined is 12 to 16 amino acids of length, preferably 14 amino acids of length.

In other embodiments, wherein the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 151, the amino acid sequence that is preceded and/or followed as defined is 13 to 17 amino acids of length, preferably 15 amino acids of length.

In other embodiments, wherein the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153, the amino acid sequence that is preceded and/or followed as defined is 16 to 20 amino acids of length, preferably 18 amino acids of length.

In various embodiments:
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 151; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.

In various embodiments:
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 151; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.

In various embodiments:
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 148 and/or followed by an amino acid sequence consisting of an amino acid sequence having at least 80 %, preferably at least 85 %, more preferably at least 90 %, such as about 100 % sequence identity to the amino acid sequence set forth in SEQ ID NO: 149; and/or
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 150 and/or followed by an amino acid sequence consisting of an amino acid sequence having at least 80 %, preferably at least 85 %, more preferably at least 90 %, such as about 100 % sequence identity to the amino acid sequence set forth in SEQ ID NO: 151; and/or
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.

In various embodiments:
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 148 and followed by an amino acid sequence consisting of an amino acid sequence having at least 80 %, preferably at least 85 %, more preferably at least 90 %, such as about 100 % sequence identity to the amino acid sequence set forth in SEQ ID NO: 149; and/or
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 150 and followed by an amino acid sequence consisting of an amino acid sequence having at least 80 %, preferably at least 85 %, more preferably at least 90 %, such as about 100 % sequence identity to the amino acid sequence set forth in SEQ ID NO: 151; and/or
- the insertion position is located within an amino acid sequence that is preceded by an amino acid sequence consisting of an amino acid sequence having at least 80 %, preferably at least 85 %, more preferably at least 90 % sequence identity to the amino acid sequence set forth in SEQ ID NO: 152 and followed by an amino acid sequence consisting of an amino acid sequence having at least 80 %, preferably at least 85 %, more preferably at least 90 %, such as about 100 % sequence identity to the amino acid sequence set forth in SEQ ID NO: 153.

The insert position may, in some embodiments, also be further characterized in terms of the flanking amino acid sequences, i.e., in terms of the amino acid sequences sequentially preceding as well as following the amino acid sequence of the inserted epitope tag.

Therefore, in some embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 155, SEQ ID NO: 1, SEQ ID NO: 2, SEQ ID NO: 3, and SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 159, SEQ ID NO: 5, SEQ ID NO: 6, SEQ ID NO: 7, and SEQ ID NO: 8 of the TCR beta constant region.

For instance, in some embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 155 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 159 of the TCR beta constant region.

Alternatively or additionally, in some embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 1 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 5 of the TCR beta constant region.

Alternatively or additionally, in some other embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 2 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 6 of the TCR beta constant region.

Alternatively or additionally, in other embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 3 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 7 of the TCR beta constant region.

Alternatively or additionally, in further embodiments, the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 8 of the TCR beta constant region.

In various further embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 156, SEQ ID NO: 9, SEQ ID NO: 157, SEQ ID NO: 10, SEQ ID NO: 158, and SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 160, SEQ ID NO: 12, SEQ ID NO: 161, SEQ ID NO: 13, SEQ ID NO: 162, and SEQ ID NO: 14 of the TCR beta constant region.

For instance, in some embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 156 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 160 of the TCR beta constant region.

Alternatively or additionally, in some embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 9 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 12 of the TCR beta constant region.

Alternatively or additionally, in some embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 157 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 161 of the TCR beta constant region.

Alternatively or additionally, in other embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 10 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 13 of the TCR beta constant region.

Alternatively or additionally, in some embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 158 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 162 of the TCR beta constant region.

Alternatively or additionally, in other embodiments, the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 14 of the TCR beta constant region.

In various further embodiments, the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15, SEQ ID NO: 16, and SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18, SEQ ID NO: 19, and SEQ ID NO: 20 of the TCR alpha constant region.

For instance, in some embodiments, the epitope tag is preceded by an amino acid sequence comprising or consisting of an amino acid sequence having at least 80 %, preferably at least 85 %, more preferably at least 90 % sequence identity an amino acid sequence selected from the amino acid sequence set forth in SEQ ID NO: 15 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequence set forth in SEQ ID NO: 18 of the TCR alpha constant region.

In other embodiments, the epitope tag is preceded by an amino acid sequence comprising or consisting of an amino acid sequence having at least 80 %, preferably at least 85 %, more preferably at least 90 % sequence identity an amino acid sequence selected from the amino acid sequence set forth in SEQ ID NO: 16 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequence set forth in SEQ ID NO: 19 of the TCR alpha constant region.

In other embodiments, the epitope tag is preceded by an amino acid sequence comprising or consisting of an amino acid sequence having at least 80 %, preferably at least 85 %, more preferably at least 90 % sequence identity an amino acid sequence selected from the amino acid sequence set forth in SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequence set forth in SEQ ID NO: 20 of the TCR alpha constant region

In various further embodiments, the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 21 and SEQ ID NO: 22 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 23 and SEQ ID NO: 24 of the TCR alpha constant region.

In some embodiments, the insert position is located within the amino acid sequence ranging from about positions 190 to 204 of the TCR beta chain. In some embodiments, the insert position is located within the amino acid sequence ranging from about positions 232 to 249 of the TCR beta chain. In some further embodiments, the insert position is located within the amino acid sequence ranging from about positions 205 to 218 of the TCR alpha chain.

In this context, the amino acid position numbering of the alpha chain refers to the amino acid positions of the amino acid sequence set forth in SEQ ID NO: 143, which provides an exemplary TCR alpha full length chain amino acid sequence, and the amino acid position numbering of the beta chain refers to the amino acid positions of the amino acid sequence set forth in SEQ ID NO: 144, which provides an exemplary TCR beta full length chain amino acid sequence.

In other words, in the context of the present invention, the term "amino acid sequence ranging from about positions 190 to 204 of the TCR beta chain" refers to a sequence that corresponds to the amino acid sequence ranging from positions 190 to 204 of the TCR beta full length amino acid sequence set forth in SEQ ID NO: 144; the term "amino acid sequence ranging from about positions 232 to 249 of the beta chain" refers to a sequence that corresponds to the amino acid sequence ranging from positions 232 to 249 of the TCR beta full length amino acid sequence set forth in SEQ ID NO: 144; and the term "amino acid sequence ranging from about positions 205 to 218 of the TCR alpha chain" refers to a sequence that corresponds to the amino acid sequence ranging from positions 205 to 218 of the TCR alpha full length amino acid sequence set forth in SEQ ID NO: 143.

Consequently, in the context of the present invention, it is intended that said insert positions for the epitope tag are likewise applicable to TCR sequences differing from the amino acid sequences set forth in SEQ ID NO: 143 and SEQ ID NO: 144, respectively, since, within a given differing TCR sequence, the respective amino acids and amino acid regions corresponding to the referenced amino acids and amino acid regions, respectively, can easily be identified by the person skilled in the art. In other words, and as a non-limiting example, the alpha constant region of the TCR sequence set forth in SEQ ID NO: 143 corresponds to or is identical to alpha constant regions of differing TCR sequences and the beta constant region of the TCR sequence set forth in SEQ ID NO: 144 corresponds to or is identical to beta constant regions of differing TCR sequences, which thus also applies to specific amino acid regions within the alpha and beta constant regions, respectively.

Therefore, in the context of the present invention, positions 205 to 218 of the TCR sequence set forth in SEQ ID NO: 143 correspond to positions 76 to 89 of the TCR alpha constant region, as set forth in SEQ ID NO: 67; positions 190 to 204 of the TCR sequence set forth in SEQ ID NO: 144 correspond to positions 59 to 73 of the TCR beta constant region, as set forth in SEQ ID NO: 67; and positions 232 to 249 of the TCR sequence set forth in SEQ ID NO: 144 correspond to positions 101 to 118 of the TCR beta constant region, as set forth in SEQ ID NO: 141.

Non-limiting examples of a TCR alpha constant region are set forth in SEQ ID NOs: 67, 68, and 69 provided herein. A non-limiting example of a TCR beta constant region is set forth in SEQ ID NO: 141 provided herein.

The insert positions disclosed in the context of the present invention may further be provided in IMGT unique numbering-conform format, based on which the person skilled in the field is able to translate other positions of the TCR alpha / beta constant regions:

| **TCR constant allele** | **loops** | **position numbering across full length alpha/beta chain** | **position numbering across full length alpha/beta constant chains** | **IMGT unique numbering** |
|---|---|---|---|---|
| | Loop1 | Glu(E) 196 | Glu(E) 65 | Glu(E) 84.3 |
| | | Pro(P) 198 | Pro(P) 67 | Pro(P) 84.5 |
| | | Ala(A) 199 | Ala(A) 68 | Ala(A) 84.6 |
| | | Leu(L) 200 | Leu(L) 69 | Leu(L) 847 |
| | | Asp(D) 202 | Asp(D) 71 | Asp(D) 85.5 |
| TRBC1 | Loop2 | Glu(E) 236 | Glu(E) 105 | Glu(E) 111.2 |
| | | Asp(D) 238 | Asp(D) 107 | Asp(D) 111.4 |
| | | Glu(E) 239 | Glu(E) 108 | Glu(E) 111.5 |
| | | Trp(W) 240 | Trp(W) 109 | Trp(W) 111.6 |
| | | Asp(D) 243 | Asp(D) 112 | Asp(D) 111.4 |
| | | Ala(A) 245 | Ala(A) 114 | Ala(A) 112.2 |
| TRAC | Loop3 | Ile(I) 209 | Ile(I) 80 | Ile(I) 114 |
| | | Pro(P) 211 | Pro(P) 82 | Pro(P) 116 |
| | | Asp(D) 213 | Asp(D) 84 | Asp(D) 118 |

In some embodiments, the insert position is located within the amino acid sequence ranging from positions 190 to 204 of the TCR beta chain. In some embodiments, the insert position is located within the amino acid sequence ranging from positions 232 to 249 of the TCR beta chain. In some further embodiments, the insert position is located within the amino acid sequence ranging from positions 205 to 218 of the TCR alpha chain.

Consequently, for instance, applying the alpha / beta constant region internal numbering, the insert position is, in some embodiments, located within the amino acid sequence ranging from positions 59 to 73 of the TCR beta constant chain; in some embodiments, the insert position is located within the amino acid sequence ranging from positions 101 to 118 of the TCR beta constant chain; in some further embodiments, the insert position is located within the amino acid sequence ranging from positions 76 to 89 of the TCR alpha constant chain.

In some embodiments, the insert position is located
- within the amino acid sequence ranging from positions 190 to 204, preferably positions 192 to 203 of the TCR beta chain; and/or
- within the amino acid sequence ranging from positions 232 to 249, preferably positions 234 to 247 of the TCR beta chain; and/or
- within the amino acid sequence ranging from positions 205 to 218, preferably positions 207 to 215 of the TCR alpha chain.

Consequently, for instance, applying the alpha / beta constant region internal numbering, the insert position is, in some embodiments, located
- within the amino acid sequence ranging from positions 59 to 73, preferably positions 61 to 72 of the TCR beta constant chain; and/or
- within the amino acid sequence ranging from positions 101 to 118, preferably positions 103 to 116 of the TCR beta constant chain; and/or
- within the amino acid sequence ranging from positions 76 to 89, preferably positions 78 to 86 of the TCR alpha constant chain.

In some embodiments, the insert position is located
- within the amino acid sequence ranging from positions 190 to 204, preferably positions 192 to 203 of the TCR beta chain; and
- within the amino acid sequence ranging from positions 232 to 249, preferably positions 234 to 247 of the TCR beta chain; and
- within the amino acid sequence ranging from positions 205 to 218, preferably positions 207 to 215 of the TCR alpha chain.

In other embodiments, the insert position is located
- within the amino acid sequence ranging from positions 190 to 204, preferably positions 192 to 203 of the TCR beta chain; or
- within the amino acid sequence ranging from positions 232 to 249, preferably positions 234 to 247 of the TCR beta chain; or
- within the amino acid sequence ranging from positions 205 to 218, preferably positions 207 to 215 of the TCR alpha chain.

Particularly, in various embodiments, the insert position is located within the amino acid sequence ranging from positions 196 to 202 of the TCR beta chain. In some other embodiments, the insert position is located within the amino acid sequence ranging from positions 236 to 245 of the TCR beta chain. In some other embodiments, the insert position is located within the amino acid sequence ranging from positions 209 to 213 of the TCR alpha chain.

Consequently, for instance, applying the alpha / beta constant region internal numbering, the insert position is, in some embodiments, located within the amino acid sequence ranging from positions 65 to 71 of the TCR beta constant chain; in some embodiments, the insert position is located within the amino acid sequence ranging from positions 105 to 114 of the TCR beta constant chain; in some further embodiments, the insert position is located within the amino acid sequence ranging from positions 80 to 84 of the TCR alpha constant chain.

For instance, in some embodiments, the insert position is located
- within the amino acid sequence ranging from positions 196 to 202 of the TCR beta chain; and/or
- within the amino acid sequence ranging from positions 236 to 245 of the TCR beta chain; and/or
- within the amino acid sequence ranging from positions 209 to 213 of the TCR alpha chain.

Consequently, for instance, applying the alpha / beta constant region internal numbering, the insert position is, in some embodiments, located
- within the amino acid sequence ranging from positions 65 to 71 of the TCR beta constant chain; and/or
- within the amino acid sequence ranging from positions 105 to 114 of the TCR beta constant chain; and/or
- within the amino acid sequence ranging from positions 80 to 84 of the TCR alpha constant chain.

In some other embodiments, the insert position is located
- within the amino acid sequence ranging from positions 196 to 202 of the TCR beta chain; and
- within the amino acid sequence ranging from positions 236 to 245 of the TCR beta chain; and
- within the amino acid sequence ranging from positions 209 to 213 of the TCR alpha chain.

In various other embodiments, the insert position is located
- within the amino acid sequence ranging from positions 196 to 202 of the TCR beta chain; or
- within the amino acid sequence ranging from positions 236 to 245 of the TCR beta chain; or
- within the amino acid sequence ranging from positions 209 to 213 of the TCR alpha chain.

For instance, in some specific embodiments, the insert position is located at a position selected from the group consisting of positions 196, 198, 199, 200, and 202 of the TCR beta chain. In some embodiments, the insert position is located at a position selected from the group consisting of positions 236, 238, 239, 240, 243, and 245 of the TCR beta chain. In some embodiments, the insert position is located at a position selected from the group consisting of positions 209, 211, and 213 of the TCR alpha chain.

Consequently, for instance, applying the alpha / beta constant region internal numbering, the insert position is located at a position selected from the group consisting of positions 65, 67, 68, 69, and 71 of the TCR beta constant chain. In some embodiments, the insert position is located at a position selected from the group consisting of positions 105, 107, 108, 109, 112, and 114 of the TCR beta constant chain. In some embodiments, the insert position is located at a position selected from the group consisting of positions 80, 82, and 84 of the TCR alpha constant chain.

For example, in some embodiments, the insert position is located at
- a position selected from the group consisting of positions 196, 198, 199, 200, and 202 of the TCR beta chain; and/or
- a position selected from the group consisting of positions 236, 238, 239, 240, 243, and 245 of the TCR beta chain; and/or
- a position selected from the group consisting of positions 209, 211, and 213 of the TCR alpha chain.

Consequently, for instance, applying the alpha / beta constant region internal numbering, the insert position is located at
- a position selected from the group consisting of positions 65, 67, 68, 69, and 71 of the TCR beta constant chain; and/or
- a position selected from the group consisting of positions 105, 107, 108, 109, 112, and 114 of the TCR beta constant chain; and/or
- a position selected from the group consisting of positions 80, 82, and 84 of the TCR alpha constant chain.

In some such embodiments, the insert position is located at
- a position selected from the group consisting of positions 196, 198, 199, 200, and 202 of the TCR beta chain; and
- a position selected from the group consisting of positions 236, 238, 239, 240, 243, and 245 of the TCR beta chain; and
- a position selected from the group consisting of positions 209, 211, and 213 of the TCR alpha chain.

In other embodiments, the insert position is located at
- a position selected from the group consisting of positions 196, 198, 199, 200, and 202 of the TCR beta chain; or
- a position selected from the group consisting of positions 236, 238, 239, 240, 243, and 245 of the TCR beta chain; or
- a position selected from the group consisting of positions 209, 211, and 213 of the TCR alpha chain.

In the context of the present invention, it is envisaged that, in some embodiments, the at least one epitope tag may be inserted by means of linker sequences. Generally, a "linker sequence" refers to a linker molecule linking the C terminus of a first amino acid segment to the N terminus of a second amino acid segment. Preferably, the linker sequences are selected from amino acid sequences, preferably from short glycine-rich linker sequences, more preferably glycine-rich linker sequences consisting of 2 to 6, preferably 3 to 5 amino acids. In some embodiments, said linker sequences comprise or consist of the amino acid sequence GSG. Generally, however, the linker sequence may be any sequence which does not impair the function of the TCR.

Generally, an epitope tag suitable for employment in the context of the present invention may be selected from any epitope tag known in the art. For instance, but without limitation, the at least one epitope tag may be selected from the group consisting of CD20 and Her2/neu tags and other conventional tags such as a myc-tag, FLAG-tag, T7-tag, HA (hemagglutinin)-tag, His-tag, S-tag, GST-tag, or GFP -tag. myc, T7, GST, GFP tags are epitopes derived from existing molecules. In contrast, FLAG is a synthetic epitope tag designed for high antigenicity (see, e.g., U.S. Pat. Nos. 4,703,004 and 4,851,341). The myc tag can preferably be used because high quality reagents are available to be used for its detection. Epitope tags can of course have one or more additional functions, beyond recognition by an antibody. The sequences of these tags are described in the literature and well known to the person of skill in art.

The epitope tag is, particularly, characterized in that it does not interfere with TCR functionality. To this end, in some embodiments, the epitope tag sequence is relatively short, and in some embodiments comprises 4 to 17 amino acids, such as 4, 5, 6, 7, 8, 9 10, 11, 12, 13, 14, 15, 16, or 17 amino acids, preferably 5 to 15 amino acids, more preferably 6 to 14 amino acids, for instance 6, 7, 8, 9, 10, 11 or 12 amino acids, such as 6, 9, 10, 11 or 12 amino acids.

Particularly, in some embodiments, the epitope tag can be recognized by a tagging antibody specific for the epitope tag. Antibodies specific for epitope tags are generally known in the art. A specific, non-limiting example of an epitope tag-specific antibody is disclosed herein below in the experimental section of the present invention.

In some embodiments, the epitope tag comprises an amino acid sequence having at least 80 %, such as 83 %, preferably at least 90 %, for instance 100 % sequence identity to the amino acid sequence set forth in SEQ ID NO: 25 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V). Accordingly, in other words, the antibody or binding fragment thereof in accordance with the present invention is capable of binding to an antigen comprising or consisting of an amino acid sequence having at least 80 %, such as 83 %, preferably at least 90 %, for instance 100 % sequence identity to the amino acid sequence set forth in SEQ ID NO: 25 (EVPKX₁R).

For instance, in some embodiments, the epitope tag comprises or consists of an amino acid sequence having at least about 60 %, for instance at least about 66 % or about 67 %, preferably at least about 80 %, such as about 83 % or about 84 %, preferably at least 90 %, for instance 100 % sequence identity to the amino acid sequence set forth in SEQ ID NO: 154.

In some embodiments, the epitope tag comprises an amino acid sequence having at least about 83 % or about 84 %, preferably 100 % sequence identity to the amino acid sequence set forth in SEQ ID NO: 154.

In some embodiments, the epitope tag consists of an amino acid sequence having at least about 83 % or about 84 %, preferably 100 % sequence identity to the amino acid sequence set forth in SEQ ID NO: 154.

In some embodiments, the epitope tag comprises or consists of an amino acid sequence having at least about 85 %, such as about 88 % or about 89 %, more preferably at least about 95 %, for instance 100 % sequence identity to the amino acid sequence set forth in SEQ ID NO: 26 (GEVPKX₁RFS), wherein X₁ is as defined herein above.

For instance, in some embodiments, the epitope tag comprises or consists of an amino acid sequence having at least about 85 %, such as about 88 % or about 89 %, more preferably at least about 95 %, for instance 100 % sequence identity to the amino acid sequence set forth in SEQ ID NO: 50.

In some embodiments, the epitope tag consists of an amino acid sequence having at least about 85 %, such as about 88 % or about 89 %, more preferably at least about 95 %, for instance 100 % sequence identity to the amino acid sequence set forth in SEQ ID NO: 50.

In some other embodiments, the epitope tag comprises or consists of an amino acid sequence having at least about 85 %, such as about 88 % or about 89 %, more preferably at least about 95 %, for instance 100 % sequence identity to the amino acid sequence set forth in SEQ ID NO: 52.

In some embodiments, the epitope tag consists of an amino acid sequence having at least about 85 %, such as about 88 % or about 89 %, more preferably at least about 95 %, for instance 100 % sequence identity to the amino acid sequence set forth in SEQ ID NO: 52.

In some other embodiments, the epitope tag comprises or consists of an amino acid sequence having at least about 80 %, more preferably at least about 90 %, for instance 100 % sequence identity to the amino acid sequence set forth in SEQ ID NO: 49.

In some embodiments, the epitope tag consists of an amino acid sequence having at least about 80 %, more preferably at least about 90 %, for instance 100 % sequence identity to the amino acid sequence set forth in SEQ ID NO: 49.

In some other embodiments, the epitope tag comprises or consists of an amino acid sequence having at least about 80 %, such as about 81 % or about 82 %, more preferably at least about 90 %, such as about 91 %, for instance 100 % sequence identity to the amino acid sequence set forth in SEQ ID NO: 48.

In some embodiments, the epitope tag consists of an amino acid sequence having at least about 80 %, such as about 81 % or about 82 %, more preferably at least about 90 %, such as about 91 %, for instance 100 % sequence identity to the amino acid sequence set forth in SEQ ID NO: 48.

In some other embodiments, the epitope tag comprises or consists of an amino acid sequence having at least about 80 %, such as about 81 % or about 82 %, more preferably at least about 90 %, such as about 91 %, for instance 100 % sequence identity to the amino acid sequence set forth in SEQ ID NO: 51.

In some embodiments, the epitope tag consists of an amino acid sequence having at least about 80 %, such as about 81 % or about 82 %, more preferably at least about 90 %, such as about 91 %, for instance 100 % sequence identity to the amino acid sequence set forth in SEQ ID NO: 51.

Further, in some embodiments, the epitope tag comprises or consists of the amino acid sequence set forth in SEQ ID NO: 27 (X₂X₃GEVPKX₁RFSX₄), wherein X₁ is as defined herein above; X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T). This consensus sequence is an artificial sequence and naturally not present in any TCR. As surprisingly found by the present inventors, such an epitope tag composition significantly diminishes the likelihood of eliciting immune responses when the epitope is introduced in a protein of interest, e.g., a TCR, ensuring heightened safety in patient applications.

For instance, in some embodiments, the epitope tag comprises an amino acid sequence selected from:
- DX₃GEVPKX₁RFSX₄ (SEQ ID NO: 28);
- EX₃GEVPKX₁RFSX₄ (SEQ ID NO: 29);
- X₂KGEVPKX₁RFSX₄ (SEQ ID NO: 30);
- X₂RGEVPKX₁RFSX₄ (SEQ ID NO: 31);
- X₂HGEVPKX₁RFSX₄ (SEQ ID NO: 32);
- X₂X₃GEVPKX₁RFSG (SEQ ID NO: 33);
- X₂X₃GEVPKX₁RFSL (SEQ ID NO: 34);
- X₂X₃GEVPKX₁RFSA (SEQ ID NO: 35);
- X₂X₃GEVPKX₁RFSV (SEQ ID NO: 36;
- X₂X₃GEVPKX₁RFSM (SEQ ID NO: 37);
- X₂X₃GEVPKX₁RFSI (SEQ ID NO: 38);
- X₂X₃GEVPKX₁RFSS (SEQ ID NO: 39);
- X₂X₃GEVPKX₁RFST (SEQ ID NO: 40),
wherein, where applicable, wherein X₁ is selected from A, R, N, D, C, Q, E, G, H, I, L, K, M, F, P, S, T, W, Y, V; X₂ is selected from D and E; X₃ is selected from K, R, and H; and X₄ is selected from G, L, A, V, M, I, S, and T.

In some embodiments, the epitope tag consists of an amino acid sequence selected from:
- DX₃GEVPKX₁RFSX₄ (SEQ ID NO: 28);
- EX₃GEVPKX₁RFSX₄ (SEQ ID NO: 29);
- X₂KGEVPKX₁RFSX₄ (SEQ ID NO: 30);
- X₂RGEVPKX₁RFSX₄ (SEQ ID NO: 31);
- X₂HGEVPKX₁RFSX₄ (SEQ ID NO: 32);
- X₂X₃GEVPKX₁RFSG (SEQ ID NO: 33);
- X₂X₃GEVPKX₁RFSL (SEQ ID NO: 34);
- X₂X₃GEVPKX₁RFSA (SEQ ID NO: 35);
- X₂X₃GEVPKX₁RFSV (SEQ ID NO: 36;
- X₂X₃GEVPKX₁RFSM (SEQ ID NO: 37);
- X₂X₃GEVPKX₁RFSI (SEQ ID NO: 38);
- X₂X₃GEVPKX₁RFSS (SEQ ID NO: 39);
- X₂X₃GEVPKX₁RFST (SEQ ID NO: 40),
wherein, where applicable, wherein X₁ is selected from A, R, N, D, C, Q, E, G, H, I, L, K, M, F, P, S, T, W, Y, V; X₂ is selected from D and E; X₃ is selected from K, R, and H; and X₄ is selected from G, L, A, V, M, I, S, and T.

In some embodiments, the epitope tag comprises an amino acid sequence selected from:
- DKGEVPKX₁RFSX₄ (SEQ ID NO: 41);
- DRGEVPKX₁RFSX₄ (SEQ ID NO: 42);
- DHGEVPKX₁RFSX₄ (SEQ ID NO: 43);
- EKGEVPKX₁RFSX₄ (SEQ ID NO: 44);
- ERGEVPKX₁RFSX₄ (SEQ ID NO: 45);
- EHGEVPKX₁RFSX₄ (SEQ ID NO: 46),
wherein X₁ and X₄ are as defined herein above.

In some embodiments, the epitope tag consists of an amino acid sequence selected from:
- DKGEVPKX₁RFSX₄ (SEQ ID NO: 41);
- DRGEVPKX₁RFSX₄ (SEQ ID NO: 42);
- DHGEVPKX₁RFSX₄ (SEQ ID NO: 43);
- EKGEVPKX₁RFSX₄ (SEQ ID NO: 44);
- ERGEVPKX₁RFSX₄ (SEQ ID NO: 45);
- EHGEVPKX₁RFSX₄ (SEQ ID NO: 46),
wherein X₁ and X₄ are as defined herein above.

For instance, in some embodiments, the epitope tag comprises or consists of an amino acid sequence having at least about 80 %, such as at least about 83 % or about 84 %, preferably at least about 90 %, such as about 91 % or about 92 %, for instance 100 % sequence identity to the amino acid sequence set forth in SEQ ID NO: 47.

In some such embodiments, the epitope tag consists of an amino acid sequence having at least about 80 %, such as at least about 83 % or about 84 %, preferably at least about 90 %, such as about 91 % or about 92 %, for instance 100 % sequence identity to the amino acid sequence set forth in SEQ ID NO: 47.

In some embodiments, the epitope tag comprises an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52.

In other embodiments, the epitope tag consists of an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52.

In various other embodiments, the epitope tag comprises an amino acid sequence having at least 80 %, preferably at least about 85 %, more preferably at least about 90 %, for instance 100 % sequence identity to an amino acid sequence selected from the group consisting of SEQ ID NO: 53. In some such embodiments, the epitope tag consists of an amino acid sequence having at least 80 %, preferably at least 85 %, more preferably at least 90 %, for instance 100 % sequence identity to an amino acid sequence selected from the group consisting of SEQ ID NO: 53.

In various embodiments, the epitope tag is flanked by linker sequences, wherein the combination of linker sequences and epitope tag comprises or consists of an amino acid sequence having at least about 80 %, preferably at least about 85 %, more preferably at least about 90 %, for instance 100 % sequence identity to an amino acid sequence selected from the group consisting of SEQ ID NO 54, SEQ ID NO: 55, SEQ ID NO: 56, SEQ ID NO: 57, SEQ ID NO: 58, and SEQ ID NO: 59.

In some embodiments, the epitope tag is flanked by linker sequences, wherein the combination of linker sequences and epitope tag consists of an amino acid sequence having at least about 80 %, preferably at least about 85 %, more preferably at least about 90 %, for instance 100 % sequence identity to an amino acid sequence selected from the group consisting of SEQ ID NO 54, SEQ ID NO: 55, SEQ ID NO: 56, SEQ ID NO: 57, SEQ ID NO: 58, and SEQ ID NO: 59.

In some embodiments, the epitope tag is flanked by linker sequences, wherein each linker sequence flanking the epitope tag has the amino acid sequence GSG and wherein the epitope tag comprises an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52.

In some embodiments, the epitope tag is flanked by linker sequences, wherein each linker sequence flanking the epitope tag has the amino acid sequence GSG and wherein the epitope tag consists of an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52.

In various other embodiments, the epitope tag is flanked by linker sequences, wherein the combination of linker sequences and epitope tag comprises or consists of an amino acid sequence having at least about 80 %, preferably at least about 85 %, more preferably at least about 90 %, for instance 100 % sequence identity to the amino acid sequence set forth in SEQ ID NO: 60.

In some such embodiments, the epitope tag is flanked by linker sequences, wherein each flanking linker sequence has the amino acid sequence GSG and wherein the epitope tag comprises an amino acid sequence having at least 80 %, preferably at least about 85 %, such as about 88 % or 89 %, more preferably at least about 90 %, for instance 100 % sequence identity to the amino acid sequence set forth in SEQ ID NO: 53. In some such embodiments, the epitope tag is flanked by linker sequences, wherein each flanking linker sequence has the amino acid sequence GSG and wherein the epitope tag consists of an amino acid sequence having at least 80 %, preferably at least 85 %, such as about 88 % or 89 %, more preferably at least 90 %, for instance 100 % sequence identity to the amino acid sequence set forth in SEQ ID NO: 53.

In various further embodiments, the epitope tag is flanked by linker sequences, wherein the combination of linker sequences and epitope tag comprises or consists of an amino acid sequence having at least about 80 %, such as about 83 % or about 84 %, preferably at least about 90 %, such as at least about 91 % or about 92 %, for instance 100 % sequence identity to the amino acid sequence set forth in SEQ ID NO: 163.

In various such embodiments, the epitope tag is flanked by linker sequences, wherein the combination of linker sequences and epitope tag consists of an amino acid sequence having at least about 80 %, such as about 83 % or about 84 %, preferably at least about 90 %, such as at least about 91 % or about 92 %, for instance 100 % sequence identity to the amino acid sequence set forth in SEQ ID NO 163.

In some embodiments, the epitope tag is flanked by linker sequences, wherein each flanking linker sequence has the amino acid sequence GSG and wherein the epitope tag comprises or consists of an amino acid sequence having at least about 60 % such as bout 66 % or 67 %, more preferably about 80 %, such as about 83 % or about 84 %, preferably at least 90 %, for instance 100 % sequence identity to the amino acid sequence set forth in SEQ ID NO: 154.

In some embodiments, the epitope tag is flanked by linker sequences, wherein each flanking linker sequence has the amino acid sequence GSG and wherein the epitope tag consists of an amino acid sequence having at least about 60 % such as bout 66 % or 67 %, more preferably about 80 %, such as about 83 % or about 84 %, preferably at least 90 %, for instance 100 % sequence identity to the amino acid sequence set forth in SEQ ID NO: 154.

Particularly, in various embodiments, integration of the epitope tag does not interfere with structural integrity and/or functionality of the TCR. In other words, in particular embodiments, presence of an epitope tag, as herein described and defined, within the TCR does not interfere with the respective TCR's biological activity/activities such that it remains functional. More particularly, integration of the epitope tag allows for natural TCR-pMHC contact. Furthermore, given the particular site of epitope integration in accordance with the present invention, integration of the epitope tag does not affect TCR specificity, affinity and avidity for target antigen since the introduced epitope tag does not lie within CDRs or in the surrounding framework region. As further surprisingly discovered, integration of the epitope tag in accordance with the present invention has no impact on expression of the TCR or co-receptor molecules.

In the context of the present invention, a "functional" TCR shall mean a TCR or TCR variant or TCR fragment, as herein described and defined, that maintains at least substantial biological activity.

In some embodiments, the TCR comprises two or more epitope-tags, as herein described and defined, either spaced apart or directly in tandem. Embodiments of such TCRs can contain 2, 3, 4, 5 or even more epitope-tags, as long as the TCR does not lose its biological activity/activities ("functional").

### Target

Typically, the TCR recognizes the peptide fragment of the antigen when it is presented by a major histocompatibility complex (MHC) molecule. However, the positioning of the epitope tag within the structural motif of solvent-exposed loops of the constant region of the TCR was found to be universally applicable irrespective of the target that a given TCR is specifically recognizing and binding to. Insertion of the epitope tag within the structural motif of a solvent-exposed loop of the constant region of the TCR allows for maintaining structural integrity and functionality as well as specificity of the TCR. Therefore, as experimentally demonstrated, epitope tagging in accordance with the present invention can be applied across different types of TCR independent of target specificity.

However, non-limiting examples of targets, which TCRs in accordance with the present invention may be specific for, include NYESO1, such as NYESO1-SSL; WT1, such as WT1-RMF; and PRAME, a preferentially expressed antigen in melanoma (PRAME) peptide.

The term "specific for" in the context of the invention means that the TCR is specifically binding to the target.

The terms "allelic variation" and "allelic version" are used herein are identical.

The recognition motif may be determined by serine substitution.

The recognition motif defines the amino acids of the epitope that influence the activation of the TCR, for example determined in an IFN-γ secretion assay. In particular, the influence of an amino acid residue in the binding epitope can be determined by an amino acid substitution scan, such as a serine scan.

In the serine scan, epitope peptides having each individual amino acid residue consecutively substituted by serine are used. If a peptide leads to a significant decrease of TCR activation, e.g. determined by IFN-g secretion indicates that the substituted position belongs to the recognition motif. A significant decrease may be a decrease of at least 3-fold, preferably at least 5-fold, more preferably of at least 10 fold IFN-g secretion compared to the unsubstituted peptide sequence, i.e. unsubstituted epitope.

### TCR sequence

As already detailed and experimentally demonstrated, epitope tagging in accordance with the present invention can be applied across different types of TCR independent of target specificity. Thus, in other words, the present invention is not particularly limited in terms of TCR sequence. However, in support of this finding and as further corroborated by the data presented in the experimental section of the present invention, non-limiting examples of TCR's are described herein below.

Specific yet non-limiting examples of TCR sequences suitable for epitope tagging in accordance with the present invention include TCRs characterized as follows:
In some embodiments, the alpha variable and joining region of a TCR may have the amino acid sequence selected from the group of amino acid sequences set forth in SEQ ID NO: 64, SEQ ID NO: 65, or SEQ ID NO: 66.

In some embodiments, the alpha constant region of the TCR may have the amino acid sequence selected from the group of amino acid sequences set forth in SEQ ID NO: 67, SEQ ID NO: 68 or SEQ ID NO 69.

In some embodiments, the beta variable and joining region of the TCR may have the amino acid sequence selected from the group of amino acid sequences set forth in SEQ ID NO: 70, SEQ ID NO: 71 or SEQ ID NO: 72.

In some embodiments, the beta constant region of the TCR may have the amino acid sequence set forth in SEQ ID NO: 141.

In some embodiments, the TCR may be characterized in comprising:
a) an alpha variable and joining region having the amino acid sequence set forth in SEQ ID NO: 64;
   an alpha constant region having the amino acid sequence set forth in SEQ ID NO: 67;
   a beta variable and joining region having the amino acid sequence set forth in SEQ ID NO: 70; and
   a beta constant region having the amino acid sequence set forth in SEQ ID NO: 141; or
b) an alpha variable and joining region having the amino acid sequence set forth in SEQ ID NO: 65;
   an alpha constant region having the amino acid sequence set forth in SEQ ID NO: 68;
   a beta variable and joining region having the amino acid sequence set forth in SEQ ID NO: 71; and
   a beta constant region having the amino acid sequence set forth in SEQ ID NO: 141; or
c) an alpha variable and joining region having the amino acid sequence set forth in SEQ ID NO: 66;
   an alpha constant region having the amino acid sequence selected set forth in SEQ ID NO 69;
   a beta variable and joining region having the amino acid sequence set forth in SEQ ID NO: 72; and
   a beta constant region having the amino acid sequence set forth in SEQ ID NO: 141.

In some embodiments, the TCR may be characterized in comprising
a) an alpha variable and joining region having an amino acid sequence which is at least 80% identical to SEQ ID NO: 64;
   an alpha constant region having an amino acid sequence which is at least 80% identical to SEQ ID NO: 67;
   a beta variable and joining region having an amino acid sequence which is at least 80% identical to SEQ ID NO: 70; and
   a beta constant region having an amino acid sequence which is at least 80% identical to SEQ ID NO: 141; or
b) an alpha variable and joining region having an amino acid sequence which is at least 80% identical to SEQ ID NO: 65;
   an alpha constant region having an amino acid sequence which is at least 80% identical to SEQ ID NO: 68;
   a beta variable and joining region having an amino acid sequence which is at least 80% identical to SEQ ID NO: 71; and
   a beta constant region having an amino acid sequence which is at least 80% identical to SEQ ID NO: 141; or
c) an alpha variable and joining region having an amino acid sequence which is at least 80% identical to SEQ ID NO: 65;
   an alpha constant region having an amino acid sequence which is at least 80% identical to SEQ ID NO 69;
   a beta variable and joining region having an amino acid sequence which is at least 80% identical to SEQ ID NO: 72; and
   a beta constant region having an amino acid sequence which is at least 80% identical to SEQ ID NO: 141.

"At least 80% identical", in particular "having an amino acid sequence which is at least 80% identical" as used herein includes that the amino acid sequence is at least 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence set out.

The determination of percent identity between multiple sequences is preferably accomplished using the AlignX application of the Vector NTI Advance^{™} 10 program (Invitrogen Corporation, Carlsbad CA, USA). This program uses a modified Clustal W algorithm (Thompson et al., 1994. Nucl Acids Res. 22: pp. 4673-4680; Invitrogen Corporation; Vector NTI Advance™ 10 DNA and protein sequence analysis software. User's Manual, 2004, pp.389-662). The determination of percent identity is performed with the standard parameters of the AlignX application.

Specific yet non-limiting examples of TCR sequences that comprise an epitope tag in accordance with the present invention include:
- SEQ ID NO: 82
   NYESO-SSL(T11.8-10-17) Beta Constant Loop 1_P198 with the epitope tag set forth in SEQ ID NO: 47 flanked by GSG linker sequences as set forth in SEQ ID NO: 54, which is also referred to herein as "V1"
- SEQ ID NO: 83
   NYESO-SSL(T11.8-10-17) Beta Constant Loop 1_A199 with the epitope tag set forth in SEQ ID NO: 47 flanked by GSG linker sequences as set forth in SEQ ID NO: 54, which is also referred to herein as "V1"
- SEQ ID NO: 84
   NYESO-SSL(T11.8-10-17) Beta Constant Loop 1_L200 with the epitope tag set forth in SEQ ID NO: 47 flanked by GSG linker sequences as set forth in SEQ ID NO: 54, which is also referred to herein as "V1"
- SEQ ID NO: 85
   NYESO-SSL(T11.8-10-17) Beta Constant Loop 1_D202 with the epitope tag set forth in SEQ ID NO: 47 flanked by GSG linker sequences as set forth in SEQ ID NO: 54, which is also referred to herein as "V1"
- SEQ ID NO: 86
   NYESO-SSL(T11.8-10-17) Beta Constant Loop 2_D238 with the epitope tag set forth in SEQ ID NO: 47 flanked by GSG linker sequences as set forth in SEQ ID NO: 54, which is also referred to herein as "V1"
- SEQ ID NO: 87
   NYESO-SSL(T11.8-10-17) Beta Constant Loop 2_W240 with the epitope tag set forth in SEQ ID NO: 47 flanked by GSG linker sequences as set forth in SEQ ID NO: 54, which is also referred to herein as "V1"
- SEQ ID NO: 88
   NYESO-SSL(T11.8-10-17) Beta Constant Loop 2_A245 with the epitope tag set forth in SEQ ID NO: 47 flanked by GSG linker sequences as set forth in SEQ ID NO: 54, which is also referred to herein as "V1"
- SEQ ID NO: 89
   NYESO-SSL(T11.8-10-17) Alpha Constant Loop 3_I209 with the epitope tag set forth in SEQ ID NO: 47 flanked by GSG linker sequences as set forth in SEQ ID NO: 54, which is also referred to herein as "V1"
- SEQ ID NO: 90
   NYESO-SSL(T11.8-10-17) Alpha Constant Loop 3_P211 with the epitope tag set forth in SEQ ID NO: 47 flanked by GSG linker sequences as set forth in SEQ ID NO: 54, which is also referred to herein as "V1"
- SEQ ID NO: 91
   NYESO-SSL(T11.8-10-17) Alpha Constant Loop 3_D213 with the epitope tag set forth in SEQ ID NO: 47 flanked by GSG linker sequences as set forth in SEQ ID NO: 54, which is also referred to herein as "V1"
- SEQ ID NO: 92
   NYESO-SSL(T11.8-10-17) Beta Constant Loop 2_A245 with the epitope tag set forth in SEQ ID NO: 48 flanked by GSG linker sequences as set forth in SEQ ID NO: 55, which is also referred to herein as "V2"
- SEQ ID NO: 93
   NYESO-SSL(T11.8-10-17) Beta Constant Loop 2_A245 with the epitope tag set forth in SEQ ID NO: 49 flanked by GSG linker sequences as set forth in SEQ ID NO: 56, which is also referred to herein as "V3"
- SEQ ID NO: 94
   NYESO-SSL(T11.8-10-17) Beta Constant Loop 2_A245 with the epitope tag set forth in SEQ ID NO: 50 flanked by GSG linker sequences as set forth in SEQ ID NO: 57, which is also referred to herein as "V4"
- SEQ ID NO: 95
   NYESO-SSL(T11.8-10-17) Beta Constant Loop 2_A245 with the epitope tag set forth in SEQ ID NO: 51 flanked by GSG linker sequences as set forth in SEQ ID NO: 58, which is also referred to herein as "V5"
- SEQ ID NO: 96
   NYESO-SSL(T11.8-10-17) Beta Constant Loop 2_A245 with the epitope tag set forth in SEQ ID NO: 52 flanked by GSG linker sequences as set forth in SEQ ID NO: 59, which is also referred to herein as "V6"
- SEQ ID NO: 97
   NYESO-SSL(T11.8-10-17) Beta Constant Loop 1_P198 with HA-Tag set forth in SEQ ID NO: 53 flanked by GSG linker sequences as set forth in SEQ ID NO: 60 and also referred to herein as "V7"
- SEQ ID NO: 98
   NYESO-SSL(T11.8-10-17) Beta Constant Loop 1_A199 with HA-Tag set forth in SEQ ID NO: 53 flanked by GSG linker sequences as set forth in SEQ ID NO: 60 and also referred to herein as "V7"
- SEQ ID NO: 99
   NYESO-SSL(T11.8-10-17) Beta Constant Loop 1_L200 with HA-Tag set forth in SEQ ID NO: 53 flanked by GSG linker sequences as set forth in SEQ ID NO: 60 and also referred to herein as "V7"
- SEQ ID NO: 100
   NYESO-SSL(T11.8-10-17) Beta Constant Loop 1_D202 with HA-Tag set forth in SEQ ID NO: 53 flanked by GSG linker sequences as set forth in SEQ ID NO: 60 and also referred to herein as "V7"
- SEQ ID NO: 101
   NYESO-SSL(T11.8-10-17) Beta Constant Loop 2_D238 with HA-Tag set forth in SEQ ID NO: 53 flanked by GSG linker sequences as set forth in SEQ ID NO: 60 and also referred to herein as "V7"
- SEQ ID NO: 102
   NYESO-SSL(T11.8-10-17) Beta Constant Loop 2_W240with HA-Tag set forth in SEQ ID NO: 53 flanked by GSG linker sequences as set forth in SEQ ID NO: 60 and also referred to herein as "V7"
- SEQ ID NO: 103
   NYESO-SSL(T11.8-10-17) Alpha Constant Loop 3_I209 with HA-Tag set forth in SEQ ID NO: 53 flanked by GSG linker sequences as set forth in SEQ ID NO: 60 and also referred to herein as "V7"
- SEQ ID NO: 104
   NYESO-SSL(T11.8-10-17) Alpha Constant Loop 3_P211 with HA-Tag set forth in SEQ ID NO: 53 flanked by GSG linker sequences as set forth in SEQ ID NO: 60 and also referred to herein as "V7"
- SEQ ID NO: 105
   PRAME-VLD(T4.8-1-29) Beta Constant Loop 1_A199 with the epitope tag set forth in SEQ ID NO: 47 flanked by GSG linker sequences as set forth in SEQ ID NO: 54, which is also referred to herein as "V1"
- SEQ ID NO: 106
   PRAME-VLD(T4.8-1-29) Beta Constant Loop 2_A245 with the epitope tag set forth in SEQ ID NO: 47 flanked by GSG linker sequences as set forth in SEQ ID NO: 54, which is also referred to herein as "V1"
- SEQ ID NO: 107
   WT1-RMF (T2.8-26-4) Beta Constant Loop 1_A199 with the epitope tag set forth in SEQ ID NO: 47 flanked by GSG linker sequences as set forth in SEQ ID NO: 54, which is also referred to herein as "V1"
- SEQ ID NO: 108
   WT1-RMF (T2.8-26-4) Beta Constant Loop 2_A245 with the epitope tag set forth in SEQ ID NO: 47 flanked by GSG linker sequences as set forth in SEQ ID NO: 54, which is also referred to herein as "V1"

As indicated herein above, Loop 1 refers to TCR beta full length chain amino acids D190 to R204, the amino acid sequence of which is set forth in SEQ ID NO: 146; Loop 2 refers to TCR beta full length chain amino acid Y232 to T249, the amino acid sequence of which is set forth in SEQ ID NO: 147; and Loop 3 refers to TCR alpha full length chain amino acid F205 to S218, the amino acid sequence of which is set forth in SEQ ID NO: 145.

TCRs in accordance with the invention may be isolated or purified. "Isolated" in the context of the invention means that the TCR is not present in the context in which it originally occurred in nature. "Purified" in the context of the invention means, e.g., that the TCR is free or substantially free of other proteins and non-protein parts of the cell it originally stems from.

In some embodiments, the amino acid sequence of the TCR may comprise one or more phenotypically silent substitutions.

"Phenotypically silent substitutions" are also named "conservative amino acid substitutions". The concept of "conservative amino acid substitutions" is understood by the skilled artisan, and preferably means that codons encoding positively-charged residues (H, K, and R) are substituted with codons encoding positively-charged residues, codons encoding negatively- charged residues (D and E) are substituted with codons encoding negatively-charged residues, codons encoding neutral polar residues (C, G, N, Q, S, T, and Y) are substituted with codons encoding neutral polar residues, and codons encoding neutral non-polar residues (A, F, I, L, M, P, V, and W) are substituted with codons encoding neutral non-polar residues. These variations can spontaneously occur, be introduced by random mutagenesis, or can be introduced by directed mutagenesis. Those changes can be made without destroying the essential characteristics of these polypeptides. The ordinarily skilled artisan can readily and routinely screen variant amino acids and/or the nucleic acids encoding them to determine if these variations substantially reduce or destroy the ligand binding capacity by methods known in the art.

The skilled person understands that also the nucleic acid encoding the TCR may be modified. Useful modifications in the overall nucleic acid sequence include codon optimization of the sequence. Alterations may be made which lead to conservative substitutions within the expressed amino acid sequence. These variations can be made in complementarity determining and non-complementarity determining regions of the amino acid sequence of the TCR chain that do not affect function. Usually, additions and deletions should not be performed in the CDR3 region.

According to some embodiments of the invention the amino acid sequence of the TCR is modified to comprise a detectable label, a therapeutic agent or pharmacokinetic modifying moiety. Modification of the TCR, in the context of the present invention, does not encompass epitope tagging, i.e., does not encompass the incorporation of at least one epitope tag, as herein defined and described. Therefore, in the context of the present invention, a TCR in accordance with the present invention may be modified, such that it comprises at least one epitope tag, as described and defined herein, and at least one modification, as described and defined herein.

Non-limiting examples for detectable labels are radiolabels, fluorescent labels, nucleic acid probes, enzymes and contrast reagents. Therapeutic agents which may be associated with the TCRs include radioactive compounds, immunomodulators, enzymes or chemotherapeutic agents. The therapeutic agents could be enclosed by a liposome linked to TCR so that the compound can be released slowly at the target site. This will avoid damaging during the transport in the body and ensure that the therapeutic agent, e.g., toxin, has maximum effect after binding of the TCR to the relevant antigen presenting cells.

Other examples for therapeutic agents are:
Peptide cytotoxins, i.e., proteins or peptides with the ability to kill mammalian cells, such as ricin, diphtheria toxin, pseudomonas bacterial exotoxin A, DNase and RNase. Small molecule cytotoxic agents, i.e., compounds with the ability to kill mammalian cells having a molecular weight of less than 700 Daltons. Such compounds could contain toxic metals capable of having a cytotoxic effect. Furthermore, it is to be understood that these small molecule cytotoxic agents also include pro-drugs, i.e. compounds that decay or are converted under physiological conditions to release cytotoxic agents. Such agents may for example include docetaxel, gemcitabine, cis-platin, maytansine derivatives, rachelmycin, calicheamicin, etoposide, ifosfamide, irinotecan, porfimer sodium photofrin II, temozolomide, topotecan, trimetrexate glucoronate, mitoxantrone, auristatin E, vincristine and doxorubicin; radionuclides, such as, iodine 131, rhenium 186, indium 111, yttrium 90. bismuth 210 and 213, actinium 225 and astatine 213. The association of the radionuclides with the TCRs or derivatives thereof may for example be carried out by chelating agents; immunostimulators, also known as immunostimulants, i.e., immune effector molecules which stimulate immune response. Exemplary immunstimulators are cytokines such as IL-2 and IFN-y, antibodies or fragments thereof, including anti-T cell or NK cell determinant antibodies (e.g anti-CD3, anti-CD28 or anti-CD16); alternative protein scaffolds with antibody like binding characteristics; Superantigens, i.e., antigens that cause non-specific activation of T cells resulting in polyclonal T cell activation and massive cytokine release, and mutants thereof; chemokines such as IL-8, platelet factor 4, melanoma growth stimulatory protein, etc. complement activators; xenogeneic protein domains, allogeneic protein domains, viral/bacterial protein domains, viral/bacterial peptides.

The antigen receptor molecules (T cell receptor molecules) on human T lymphocytes are non-covalently associated with the CD3 (T3) molecular complex on the cell surface. Perturbation of this complex with anti-CD3 monoclonal antibodies induces T cell activation. Thus, some embodiments refer to a TCR as described herein associated (usually by fusion to an N-or C-terminus of the alpha or beta chain) with an anti-CD3 antibody, or a functional fragment or variant of said anti-CD3 antibody. Antibody fragments and variants/analogues which are suitable for use in the compositions and methods described herein include minibodies, Fab fragments, F(ab<'>)2fragments, dsFv and scFv fragments, Nanobodies^{™} (Ablynx (Belgium), molecules comprising synthetic single immunoglobulin variable heavy chain domain derived from a camelid (e.g. camel or llama) antibody) and Domain Antibodies (comprising an affinity matured single immunoglobulin variable heavy chain domain or immunoglobulin variable light chain domain (Domantis (Belgium)) or alternative protein scaffolds that exhibit antibody-like binding characteristics such as Affibodies (comprising engineered protein A scaffold Affibody (Sweden)) or Anticalins (comprising engineered anticalins Pieris (Germany)).

The therapeutic agent may preferably be selected from the group consisting of an immune effector molecule, a cytotoxic agent and a radionuclide. Preferably, the immune effector molecule is a cytokine.

The pharmacokinetic modifying moiety may be for example at least one polyethylene glycol repeating unit, at least one glycol group, at least one sialyl group or a combination thereof. The association of at least one polyethylene glycol repeating unit, at least one glycol group, at least one sialyl group may be caused in a number of ways known to those skilled in the art. In a preferred embodiment the units are covalently linked to the TCR. The TCRs according to the invention can be modified by one or several pharmacokinetic modifying moieties. In particular, the soluble form of the TCR is modified by one or several pharmacokinetic modifying moieties. The pharmacokinetic modifying moiety may achieve beneficial changes to the pharamacokinetic profile of the therapeutic, for example improved plasma half-life, reduced or enhanced immunogenicity, and improved solubility.

The TCR according to the invention may be soluble or membrane bound. The term "soluble" refers to a TCR being in soluble form (i.e., having no transmembrane or cytoplasmic domains), for example for use as a targeting agent for delivering therapeutic agents to the antigen presenting cell. For stability, soluble αβ heterodimeric TCRs preferably have an introduced disulfide bond between residues of the respective constant domains, as described, for example, in WO 03/020763. One or both of the constant domains present in an αβ heterodimer of the invention may be truncated at the C terminus or C termini, for example by up to 15, or up to 10 or up to 8 or fewer amino acids. For use in adoptive therapy, an αβ heterodimeric TCR may, for example, be transfected as full-length chains having both cytoplasmic and transmembrane domains. TCRs may contain a disulfide bond corresponding to that found in nature between the respective alpha and beta constant domains, additionally or alternatively a non-native disulfide bond may be present.

The TCR, in particular a soluble form of the TCR according to the invention can thus be modified by attaching additional functional moieties, e.g., for reducing immunogenicity, increasing hydrodynamic size (size in solution) solubility and/or stability (e.g. by enhanced protection to proteolytic degradation) and/or extending serum half-life.

Other useful functional moieties and modifications include "suicide" or "safety switches" that can be used to shut off effector host cells carrying an inventive TCR in a patient's body. An example is the inducible Caspase 9 (iCasp9) "safety switch" described by Gargett and Brown Front Pharmacol. 2014; 5: 235. Briefly, effector host cells are modified by well-known methods to express a Caspase 9 domain whose dimerization depends on a small molecule dimerizer drug such as AP1903/CIP, and results in rapid induction of apoptosis in the modified effector cells. The system is for instance described in EP2173869 (A2). Examples for other "suicide" "safety switches" are known in the art, e.g. Herpes Simplex Virus thymidine kinase (HSV-TK), expression of CD20 and subsequent depletion using anti-CD20 antibody, expression of truncated EGFR and subsequent depletion using anti-EGFR antibody (Wang et al, Blood, 2011 Aug 4;118(5):1255-63) or myc tags (Kieback et al, Proc Natl Acad Sci U S A. 2008 Jan 15;105(2):623-8).

TCRs with an altered glycosylation pattern are also envisaged herein. As is known in the art, glycosylation patterns can depend on the amino acid sequence (e.g., the presence or absence of particular glycosylation amino acid residues, discussed below) and/or the host cell or organism in which the protein is produced. Glycosylation of polypeptides is typically either N-linked or O-linked. N-linked refers to the attachment of the carbohydrate moiety to the side chain of an asparagine residue. Addition of N-linked glycosylation sites to the binding molecule is conveniently accomplished by altering the amino acid sequence such that it contains one or more tri-peptide sequences selected from asparagine-X-serine and asparagine-X-threonine (where X is any amino acid except proline). O-linked glycosylation sites may be introduced by the addition of or substitution by, one or more serine or threonine residues to the starting sequence.

Another means of glycosylation of TCRs is by chemical or enzymatic coupling of glycosides to the protein. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. Similarly, deglycosylation (i.e., removal of carbohydrate moieties present on the binding molecule) may be accomplished chemically, e.g., by exposing the TCRs to trifluoromethanesulfonic acid, or enzymatically by employing endo- and exo-glycosidases.

It is also conceivable to add a drug such as a small molecule compound to the TCR, in particular a soluble form of the inventive TCR. Linkage can be achieved via covalent bonds, or non-covalent interactions such as through electrostatic forces. Various linkers, known in the art, can be employed in order to form the drug conjugates.

In general, the TCR can be modified in some instances with various mutations that modify the affinity and the off-rate of the TCR with the target antigen. In particular, the mutations may increase the affinity and/or reduce the off-rate. Thus, the TCR may be mutated in at least one CDR and the variable domain framework region thereof.

However, in some embodiments the CDR regions of the TCR are not modified or *in vitro* affinity maturated. This means that the CDR regions have naturally occurring sequences. This can be advantageous, since *in vitro* affinity maturation may lead to immunogenicity to the TCR molecule. This may lead to the production of anti-drug antibodies decreasing or inactivating the therapeutic effect and the treatment and /or induce adverse effects.

The mutation may be one or more substitution(s), deletion(s) or insertions(s). These mutations may be introduced by any suitable method known in the art, such as polymerase chain reaction, restriction enzyme-based cloning, ligation independent cloning procedures, which are described for Example in Sambrook, Molecular Cloning - 4th Edition (2012) Cold Spring Harbor Laboratory Press.

Theoretically, unpredictable TCR specificity with the risk for cross-reactivity can occur due to mispairing between endogenous and exogenous TCR chains. To avoid mispairing of TCR sequences, the recombinant TCR sequence may be modified to contain murinized Cα and Cβ regions, a technology that has been shown to efficiently enhance correct pairing of several different transduced TCR chains. Murinization of TCRs (i.e., exchanging the human constant regions in the alpha and beta chain by their murine counterparts) is a technique that is commonly applied in order to improve cell surface expression of TCRs in host cells. Without wishing to be bound by specific theory, it is thought that murinized TCRs associate more effectively with CD3 co-receptors; and/or that preferentially pair with each other and are less prone to form mixed TCRs on human T cells genetically modified ex *vivo* to express the TCRs of desired antigenic specificity, but still retaining and expressing their "original", i.e., endogenous, TCRs.

Nine amino acids responsible for the improved expression of murinized TCRs have been identified (Sommermeyer and Uckert, J Immunol. 2010 Jun 1; 184(11):6223-31) and it is envisaged to substitute at least one or all of the amino acid residues in the TCRs alpha and/or beta chain constant region for their murine counterpart residues. This technique is also referred to as "minimal murinization", and offers the advantage of enhancing cell surface expression while, at the same time, reducing the number of "foreign" amino acid residues in the amino acid sequence and, thereby, the risk of immunogenicity. Thus, in some embodiments the TCR sequence may be modified to contain minimal murinized Cα and Cβ regions.

Some embodiments refer to an isolated TCR as described herein, wherein the TCR is of the single chain type, wherein the TCR α chain and the TCR β chain are linked by a linker sequence.

A suitable single chain TCR form comprises a first segment constituted by an amino acid sequence corresponding to a variable TCR α region, a second segment constituted by an amino acid sequence corresponding to a variable TCR β region fused to the N terminus of an amino acid sequence corresponding to a TCR β chain constant region extracellular sequence, and a linker sequence linking the C terminus of the first segment to the N terminus of the second segment. Alternatively, the first segment may be constituted by an amino acid sequence corresponding to a TCR β chain variable region, the second segment may be constituted by an amino acid sequence corresponding to a TCR α chain variable region sequence fused to the N terminus of an amino acid sequence corresponding to a TCR α chain constant region extracellular sequence. The above single chain TCRs may further comprise a disulfide bond between the first and second chains, and wherein the length of the linker sequence and the position of the disulfide bond being such that the variable domain sequences of the first and second segments are mutually orientated substantially as in native TCRs. More specifically the first segment may be constituted by an amino acid sequence corresponding to a TCR α chain variable region sequence fused to the N terminus of an amino acid sequence corresponding to a TCR α chain constant region extracellular sequence, the second segment may be constituted by an amino acid sequence corresponding to a TCR β chain variable region fused to the N terminus of an amino acid sequence corresponding to TCR β chain constant region extracellular sequence, and a disulfide bond may be provided between the first and second chains. The linker sequence may be any sequence which does not impair the function of the TCR.

In accordance with the definition of the term "functional", as provided herein, a functional TCR α and/or β chain fusion protein shall mean that both chains remain able to form a T-cell receptor (either with a non- modified α and/or β chain or with another inventive fusion protein α and/or β chain) which exerts its biological function, in particular binding to the specific peptide-MHC complex of said TCR, and/or functional signal transduction upon specific peptide:MHC interaction.

### TCR fragments and variants

Another aspect of the invention refers to a polypeptide comprising a functional portion of the TCR of as described herein. The functional portion may comprise at least one of the amino acid sequences selected from the group consisting of SEQ ID NOs: SEQ ID NO: 64-72 and 104, so long as it comprises at least one epitope tag located within the TCR, as herein described and defined.

The TCR variant molecule, *i.e.,* a molecule combining a polypeptide comprising a functional portion of the TCR with other domains, may have the binding properties of the TCR receptor but may be combined with signaling domains of effectors cells (other than T cells), in particular with signaling domains of NK cells. Therefore, some embodiments refer to a protein comprising a functional portion of the TCR as described herein in combination with the signaling domains of an effector cell, such as a NK cell.

"Binding" refers to the ability to specifically and non-covalently associate, unite or bind with the target.

Another aspect of the invention refers to a multivalent TCR complex comprising at least two TCRs in accordance with the present invention. In one embodiment of this aspect, at least two TCR molecules are linked *via* linker moieties to form multivalent complexes. Preferably, the complexes are water soluble, so the linker moiety should be selected accordingly. It is preferable that the linker moiety is capable of attaching to defined positions on the TCR molecules, so that the structural diversity of the complexes formed is minimized. One embodiment of the present aspect is provided by a TCR complex of the invention wherein the polymer chain or peptidic linker sequence extends between amino acid residues of each TCR which are not located in a variable region sequence of the TCR. Since the complexes of the invention may be for use in medicine, the linker moieties should be chosen with due regard to their pharmaceutical suitability, for example their immunogenicity. Examples of linker moieties which fulfil the above desirable criteria are known in the art, for example the art of linking antibody fragments.

Examples for linkers are hydrophilic polymers and peptide linkers. An example for hydrophilic polymers are polyalkylene glycols. The most commonly used of this class are based on polyethylene glycol or PEG. However, others are based on other suitable, optionally substituted, polyalkylene glycols which include polypropylene glycol, and copolymers of ethylene glycol and propylene glycol. Peptide linkers are comprised of chains of amino acids, and function to produce simple linkers or multimerization domains onto which TCR molecules can be attached.

One embodiment refers to a multivalent TCR complex, wherein at least one of said TCRs is associated with a therapeutic agent.

### Nucleic Acids, Vectors

Another aspect of the invention refers to a nucleic acid encoding a TCR as described herein or encoding the polynucleotide encoding a TCR as described herein.

The following table indicates exemplary nucleotide sequences encoding the exemplary respective peptide sequences, as referred to herein in the context of the present invention:

**Table 1: exemplary TCR sequences**

| **TCR** | **Amino acid sequence** | **Nucleic acid sequence** |
|---|---|---|
| NYESO-SSL(T11.8-10-17) Alpha Variable & Joining region | SEQ ID NO: 64 | SEQ ID NO: 73 |
| NYESO-SSL(T11.8-10-17) Alpha Constant region | SEQ ID NO: 67 | SEQ ID NO: 76 |
| NYESO-SSL (T11.8-10-17) Beta variable & joining region | SEQ ID NO: 70 | SEQ ID NO: 79 |
| NYESO-SSL (T11.8-10-17) Beta constant region | SEQ ID NO: 141 | SEQ ID NO: 142 |
| PRAME-VLD (T4.8-1-29) Alpha variable & joining region | SEQ ID NO: 65 | SEQ ID NO: 74 |
| PRAME-VLD (T4.8-1-29) Alpha constant region | SEQ ID NO: 68 | SEQ ID NO: 77 |
| PRAME-VLD (T4.8-1-29) Beta variable & joining region | SEQ ID NO: 71 | SEQ ID NO: 80 |
| PRAME-VLD (T4.8-1-29) Beta constant region | SEQ ID NO: 141 | SEQ ID NO: 142 |
| WT1-RMF (T2.8-26-4) Alpha variable & joining region | SEQ ID NO: 66 | SEQ ID NO: 75 |
| WT1-RMF (T2.8-26-4) Alpha constant region | SEQ ID NO: 69 | SEQ ID NO: 78 |
| WT1-RMF (T2.8-26-4) Beta variable & joining region | SEQ ID NO: 72 | SEQ ID NO: 81 |
| WT1-RMF (T2.8-26-4) Beta constant region | SEQ ID NO: 141 | SEQ ID NO: 142 |

"Nucleic acid molecule" and generally means a polymer of DNA or RNA, which can be single-stranded or double-stranded, synthesized or obtained (e.g., isolated and/or purified) from natural sources, which can contain natural, non-natural or altered nucleotides, and which can contain a natural, non-natural or altered internucleotide linkage, such as a phosphoroamidate linkage or a phosphorothioate linkage, instead of the phosphodiester found between the nucleotides of an unmodified oligonucleotide. Preferably, the nucleic acids described herein are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be *in vitro* replication or *in vivo* replication. The nucleic acids can be constructed based on chemical synthesis and/or enzymatic ligation reactions using procedures known in the art or commercially available (e.g. from Genscript, Thermo Fisher and similar companies). See, for example Sambrook et al., a nucleic acid can be chemically synthesized using naturally occurring nucleotides or variously modified nucleotides designed to increase the biological stability of the molecules or to increase the physical stability of the duplex formed upon hybridization (e.g., phosphorothioate derivatives and acridine substituted nucleotides). The nucleic acid can comprise any nucleotide sequence which encodes any of the recombinant TCRs, polypeptides, or proteins, or functional portions or functional variants thereof.

The present disclosure also provides variants of the isolated or purified nucleic acids wherein the variant nucleic acids comprise a nucleotide sequence that has at least 75%, 80%, 81%, 82%, 83%, 84%, 85%, 86%, 87%, 88%, 89%, 90%, 91%, 92%, 93%, 94%, 95%, 96%, 97%, 98%, or 99% identical to the nucleotide sequence encoding the TCR described herein.

The disclosure also provides an isolated or purified nucleic acid comprising a nucleotide sequence which is complementary to the nucleotide sequence of any of the nucleic acids described herein or a nucleotide sequence which hybridizes under stringent conditions to the nucleotide sequence of any of the nucleic acids described herein.

The nucleotide sequence which hybridizes under stringent conditions preferably hybridizes under high stringency conditions. By "high stringency conditions" is meant that the nucleotide sequence specifically hybridizes to a target sequence (the nucleotide sequence of any of the nucleic acids described herein) in an amount that is detectably stronger than non-specific hybridization. High stringency conditions include conditions which would distinguish a polynucleotide with an exact complementary sequence, or one containing only a few scattered mismatches from a random sequence that happened to have a few small regions (e.g., 3-10 bases) that matched the nucleotide sequence. Such small regions of complementarity are more easily melted than a full-length complement of 14-17 or more bases, and high stringency hybridization makes them easily distinguishable. Relatively high stringency conditions would include, for example, low salt and/or high temperature conditions, such as provided by about 0.02-0.1 M NaCl or the equivalent, at temperatures of about 50-70° C. Such high stringency conditions tolerate little, if any, mismatch between the nucleotide sequence and the template or target strand, and are particularly suitable for detecting expression of any of the TCRs described herein. It is generally appreciated that conditions can be rendered more stringent by the addition of increasing amounts of formamide.

As already described elsewhere herein, the nucleic acid encoding the TCR may be modified. Useful modifications in the overall nucleic acid sequence may be codon optimization. Alterations may be made which lead to conservative substitutions within the expressed amino acid sequence. These variations can be made in complementarity determining and non-complementarity determining regions of the amino acid sequence of the TCR chain that do not affect function of the TCR in accordance with the present invention. Usually, additions and deletions should not be performed in the CDR3 region.

Another embodiment refers to a vector comprising the nucleic acid encoding the TCR as described herein.

The vector is preferably a plasmid, shuttle vector, phagemide, cosmid, expression vector, retroviral vector, adenoviral vector or particle and/or vector to be used in gene therapy.

A "vector" is any molecule or composition that has the ability to carry a nucleic acid sequence into a suitable host cell where synthesis of the encoded polypeptide can take place. Typically, and preferably, a vector is a nucleic acid that has been engineered, using recombinant DNA techniques that are known in the art, to incorporate a desired nucleic acid sequence (e.g., a nucleic acid of the invention). The vector may comprise DNA or RNA and/or comprise liposomes. The vector may be a plasmid, shuttle vector, phagemide, cosmid, expression vector, retroviral vector, lentiviral vector, adenoviral vector or particle and/or vector to be used in gene therapy. A vector may include nucleic acid sequences that permit it to replicate in a host cell, such as an origin of replication. A vector may also include one or more selectable marker genes and other genetic elements known to those of ordinary skill in the art. A vector preferably is an expression vector that includes a nucleic acid according to the present invention operably linked to sequences allowing for the expression of said nucleic acid.

Preferably, the vector is an expression vector. More preferably, the vector is a retroviral, more specifically a gamma-retroviral or lentiviral vector.

### Cells, Cell lines

Another aspect of the invention refers to a cell expressing the TCR as described herein.

In some embodiments, the cell is isolated or non-naturally occurring.

In specific embodiments, the cell may comprise the nucleic acid encoding the TCR as described herein or the vector comprising said nucleic acid.

In the cell the above-described vector comprising a nucleic acid sequence coding for the above described TCR may be introduced or *ivt*RNA coding for said TCR may be introduced. The cell may be a peripheral blood lymphocyte such as a T cell. The method of cloning and exogenous expression of the TCR is for example described in Engels et al. (Relapse or eradication of cancer is predicted by peptide-major histocompatibility complex affinity. Cancer Cell, 23(4), 516-26. 2013). The transduction of primary human T cells with a lentiviral vector is, for example, described in Cribbs "simplified production and concentration of lentiviral vectors to achieve high transduction in primary human T cells" BMC Biotechnol. 2013; 13: 98.

The term "transfection" and "transduction" are interchangeable and refer to the process by which an exogenous nucleic acid sequence is introduced in a host cell, e.g. in a eukaryotic host cell. It is noted that introduction or transfer of nucleic acid sequences is not limited to the mentioned methods but can be achieved by any number of means including electroporation, microinjection, gene gun delivery, lipofection, superfection and the mentioned infection by retroviruses or other suitable viruses for transduction or transfection.

Some embodiments refer to a cell comprising:
a) an expression vector which comprises at least one nucleic acid as described herein, or
b) a first expression vector which comprises a nucleic acid encoding the alpha chain of the TCR as described herein, and a second expression vector which comprises a nucleic acid encoding the beta chain of a TCR as described herein.

In such embodiments b), the at least one epitope tag may be present in said alpha chain and/or in said beta chain.

In some embodiments, the cell is a peripheral blood lymphocyte (PBL) or a peripheral blood mononuclear cell (PBMC). The cell may be a natural killer cell or a T cell. Preferably, the cell is a T cell. The T cell may be a CD4+ or a CD8+ T cell. In some embodiments, the cell is selected from NK cells and NK-like T cells.

In some embodiments the cell is a stem cell like memory T cell.

Stem cell-like memory T cells (TSCM) are a less-differentiated subpopulation of CD8+ T cells, which are characterized by the capacity of self-renewal and to persist long-term. Once these cells encounter their antigen *in vivo,* they differentiate further into central memory T cells (TCM), effector memory T cells (TEM) and terminally differentiated effector memory T cells (TEMRA) with some TSCM remaining quiescent (Flynn et al., Clinical & Translational Immunology (2014). These remaining TSCM cells show the capacity to build a durable immunological memory *in vivo* and therefore are considered an important T cell subpopulation for adoptive T cell therapy (Lugli et al., Nature Protocols 8, 33-42 (2013) Gattinoni et al., Nat. Med. 2011 Oct; 17(10): 1290-1297). Immune-magnetic selection can be used in order to restrict the T cell pool to the stem cell memory T cell subtype see (Riddell et al. 2014, Cancer Journal 20(2): 141-44)

### Pharmaceutical compositions, medical treatments and kits

Another aspect of the invention refers to pharmaceutical composition comprising the TCR as described herein, the polypeptide comprising a functional portion of said TCR, the multivalent TCR complex as described herein, the nucleic acid encoding the TCR, the vector comprising said nucleic acid, the cell comprising said TCR as described herein.

Those active components of the present invention are preferably used in such a pharmaceutical composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutically acceptable" defines a non-toxic material, which does not interfere with effectiveness of the biological activity of the active component. The choice of the carrier is dependent on the application.

The pharmaceutical composition may contain additional components which enhance the activity of the active component or which supplement the treatment. Such additional components and/or factors can be part of the pharmaceutical composition to achieve synergistic effects or to minimize adverse or unwanted effects.

Techniques for the formulation or preparation and application/medication of active components of the present invention are published in "Remington's Pharmaceutical Sciences", Mack Publishing Co., Easton, PA, latest edition. An appropriate application is a parenteral application, for example intramuscular, subcutaneous, intramedular injections as well as intrathecal, direct intraventricular, intravenous, intranodal, intraperitoneal or intratumoral injections. The intravenous injection is the preferred treatment of a patient.

According to a preferred embodiment, the pharmaceutical composition is an infusion or an injection.

An injectable composition is a pharmaceutically acceptable fluid composition comprising at least one active ingredient, e.g. an expanded T cell population (for example autologous or allogenic to the patient to be treated) expressing a TCR. The active ingredient is usually dissolved or suspended in a physiologically acceptable carrier, and the composition can additionally comprise minor amounts of one or more non-toxic auxiliary substances, such as emulsifying agents, preservatives, and pH buffering agents and the like. Such injectable compositions that are useful for use with the fusion proteins of this disclosure are conventional; appropriate formulations are well known to those of ordinary skill in the art.

Typically, the pharmaceutical composition comprises at least one pharmaceutically acceptable carrier.

Accordingly, in another aspect the present invention refers to the epitope-tagged TCRs of the present invention, corresponding polypeptides of the present invention, the multivalent TCR complexes of the present invention, the nucleic acids of the present invention, the vectors of the present invention, or the cells of the present invention, particularly the epitope-tagged TCRs of the present invention, for use as a medicament, for use as a screening agent, or for the use in the therapy of malignancies.

Thus, in some embodiments, the invention refers to the TCR as described herein, the polypeptide comprising described herein, the multivalent TCR complex according as described herein, the nucleic acid encoding said TCR, the vector comprising said nucleic acid, the cell comprising said TCR as described herein for use as a medicament.

Some embodiments refer to the TCR as described herein, the polypeptide described herein, the multivalent TCR complex according as described herein, the nucleic acid encoding said TCR, the vector comprising said nucleic acid, the cell comprising said TCR for use in the treatment of cancer.

In one embodiment the cancer is a hematological cancer or a solid tumor.

Hematological cancers also called blood cancers which do not form solid tumors and therefore are primarily dispersed in the body. Examples of hematological cancers are leukemia, lymphoma or multiple myeloma.

There are two major types of solid tumors, sarcomas and carcinomas. Sarcomas are for example tumors of the blood vessel, bone, fat tissue, ligament, lymph vessel, muscle or tendon.

In various embodiments, the cancer is selected from the group consisting of prostate cancer, uterine cancer, thyroid cancer, testicular cancer, renal cancer, pancreatic cancer, ovarian cancer, esophageal cancer, non-small-cell lung cancer, lung adenocarcinoma, squamous cell carcinoma, non-Hodgkin's lymphoma, multiple myeloma, melanoma, hepatocellular carcinoma, head and neck cancer, gastric cancer, endometrial cancer, cervical cancer, colorectal cancer, stomach adenocarcinoma, cholangiocarcinoma, breast cancer, bladder cancer, myeloid leukemia and acute lymphoblastic leukemia, carcinoma, acute myeloid leukemia, myelodysplastic syndromes and multiple myeloma, sarcoma or osteosarcoma.

In various embodiments, the hematological cancer may be selected from the group consisting of, non-Hodgkin's lymphoma (NHL), Hodgkin's lymphoma (HL), multiple myeloma, acute myeloid leukemia (AML) and acute lymphoblastic leukemia (ALL), mixed phenotype acute leukemia (MPAL), chronic myeloid leukemia (CML), B cell polymorphic lymphoma, hairy cell leukemia, chronic lymphocytic leukemia (CLL), small lymphocytic lymphoma (SLL), central nervous system lymphoma, CD37+ dendritic cell lymphoma, lymphoplasmatic lymphoma, splenic marginal zone lymphoma, plasma cell myeloma, extraosseuos plasmacytoma, extra-nodal marginal zone B-cell lymphoma of mucosa-associated lymphoid tissue (MALT tissue), nodal marginal zone B-cell lymphoma, follicular lymphoma, mantel cell lymphoma, diffuse large B-cell lymphoma, mediastinal (thymic) large B-cell lymphoma, precursor B-lymphoblastic lymphoma, immunoblastic large cell lymphoma, intravascular large B-cell lymphoma, primary effusion lymphoma, Burkitt's lymphoma/leukemia, B-cell proliferations of uncertain malignant potential, lymphomatoid granulomatosis, post-transplant lymphoproliferative disorder. Other hematological cancers or disorders include myelodysplastic disorder.

Also contemplated herein are pharmaceutical compositions and kits containing one or more of (i) an isolated TCR as described herein; (ii) viral particles comprising a nucleic acid encoding a recombinant TCR; (iii) immune cells, such as T cells or NK cells, modified to express a recombinant TCR as described herein; (iv) nucleic acids encoding a recombinant TCR as described herein. In some embodiments, the present disclosure provides compositions comprising lentiviral vector particles comprising a nucleotide sequence encoding a recombinant TCR described herein (or T cells that have been modified using the vector particles described herein to express a recombinant TCR). Such compositions can be administered to subjects in the methods of the present disclosure as described further herein.

Compositions comprising the modified T cells as described herein can be utilized in methods and compositions for adoptive immunotherapy in accordance with known techniques, or variations thereof that will be apparent to those skilled in the art based on the instant disclosure.

In some embodiments, the cells are formulated by first harvesting them from their culture medium, and then washing and concentrating the cells in a medium and container system suitable for administration (a "pharmaceutically acceptable" carrier) in a treatment-effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin.

The number of cells for an effective treatment in the composition is typically greater than 10 cells, and up to 10⁶, up to and including 10⁸ or 10⁹ cells and can be more than 10¹⁰ cells. The number of cells will depend upon the ultimate use for which the composition is intended as will the type of cells included therein. For example, if cells that are specific for a particular antigen are desired, then the population will contain greater than 70%, generally greater than 80%, 85% and 90-95% of such cells. For uses provided herein, the cells are generally in a volume of a liter or less, can be 500 ml or less, even 250 ml or 100 ml or less. Hence the density of the desired cells is typically greater than 10⁶ cells/ml and generally is greater than 10⁷ cells/ml, generally 10⁸ cells/ml or greater. The clinically relevant number of immune cells can be apportioned into multiple infusions that cumulatively equal or exceed 10⁹, 10¹⁰ or 10¹¹ cells. Pharmaceutical compositions provided herein can be in various forms, e.g., in solid, liquid, powder, aqueous, or lyophilized form. Examples of suitable pharmaceutical carriers are known in the art. Such carriers and/or additives can be formulated by conventional methods and can be administered to the subject at a suitable dose. Stabilizing agents such as lipids, nuclease inhibitors, polymers, and chelating agents can preserve the compositions from degradation within the body. In a composition intended to be administered by injection, one or more of a surfactant, preservative, wetting agent, dispersing agent, suspending agent, buffer, stabilizer and isotonic agent may be included.

The recombinant TCRs as described herein, or the viral vector particles comprising a nucleotide sequence encoding a recombinant TCR provided herein, can be packaged as kits. Kits can optionally include one or more components such as instructions for use, devices, and additional reagents, and components, such as tubes, containers and syringes for practice of the methods. Exemplary kits can include the nucleic acids encoding the recombinant TCRs, the recombinant TCR polypeptides, or viruses provided herein, and can optionally include instructions for use, a device for detecting a virus in a subject, a device for administering the compositions to a subject, and a device for administering the compositions to a subject.

Kits comprising polynucleotides encoding a gene of interest (e.g., a recombinant TCR) are also contemplated herein. Kits comprising a viral vector encoding a sequence of interest (e.g., a recombinant TCR) and optionally, a polynucleotide sequence encoding an immune checkpoint inhibitor are also contemplated herein.

Kits contemplated herein also include kits for carrying out the methods for detecting the presence of polynucleotides encoding any one or more of the TCRs disclosed herein. In particular, such diagnostic kits may include sets of appropriate amplification and detection primers and other associated reagents for performing deep sequencing to detect the polynucleotides encoding TCRs disclosed herein. In further embodiments, the kits herein may comprise reagents for detecting the TCRs disclosed herein, such as antibodies or other binding molecules. Diagnostic kits may also contain instructions for determining the presence of the polynucleotides encoding the TCRs disclosed herein or for determining the presence of the TCRs disclosed herein. A kit may also contain instructions. Instructions typically include a tangible expression describing the components included in the kit, and methods for administration, including methods for determining the proper state of the subject, the proper dosage amount, and the proper administration method. Instructions can also include guidance for monitoring the subject over the duration of the treatment time.

Kits provided herein also can include a device for administering a composition described herein to a subject. Any of a variety of devices known in the art for administering medications or vaccines can be included in the kits provided herein. Exemplary devices include, but are not limited to, a hypodermic needle, an intravenous needle, a catheter, a needle-less injection device, an inhaler, and a liquid dispenser, such as an eyedropper. Typically, the device for administering a virus of the kit will be compatible with the virus of the kit; for example, a needle-less injection device such as a high pressure injection device can be included in kits with viruses not damaged by high pressure injection, but is typically not included in kits with viruses damaged by high pressure injection.

Kits provided herein also can include a device for administering a compound, such as a T cell activator or stimulator, or a TLR agonist, such as a TLR4 agonist to a subject. Any of a variety of devices known in the art for administering medications to a subject can be included in the kits provided herein. Exemplary devices include a hypodermic needle, an intravenous needle, a catheter, a needle-less injection, but are not limited to, a hypodermic needle, an intravenous needle, a catheter, a needle-less injection device, an inhaler, and a liquid dispenser such as an eyedropper. Typically, the device for administering the compound of the kit will be compatible with the desired method of administration of the compound.

### Method of modifying a TCR

In a further aspect, the present invention also relates to a method for modifying a TCR or a fragment thereof comprising the constant region of the TCR, the method comprising introduction, into the constant region, at least one epitope tag, wherein the insert position of the epitope tag is located in the alpha constant region (TRAC) and/or the beta constant region (TRBC).

Methods and means for introducing epitope tags into TCRs are generally known and can be applied accordingly in the context of the present invention.

In various embodiments, the method further comprises at least one step of modifying the amino acid sequence of the TCR for the TCR to comprise a detectable label, a therapeutic agent or pharmacokinetic modifying moiety. In accordance with the present invention, said step of modifying the amino acid sequence of the TCR does not encompass modifying of the epitope tag introduced, in accordance with the present invention, into the alpha constant region (TRAC) and/or the beta constant region (TRBC) of the TCR.

In various such embodiments, the TCR α chain and/or the TCR β chain is modified.

In some embodiments, the method comprises at least one step of modifying the TCR such that the recombinant TCR sequence may contain minimal murinized Cα and Cβ regions.

All embodiments described herein above with regard to the epitope-tagged TCRs of the present invention as well as related aspects apply *mutatis mutandis.*

### Experiments

### Examples:

### Example 1: Analysis of expression and functionality of UNI-TAB-TCR inserted with the epitope set forth in SEQ ID NO: 47 in Jurkat 76 -/-CD8⁺ ieGFP at various specific positions

To ensure that the constructs in accordance with the present invention were properly expressed, folded, and functional, rTCR-deficient ieGFP Jurkat-76 -/- CD8+ cells (also known as biosensors) were transduced with the corresponding NY-ESO-UNI-TABS constructs. Then, antibody staining (using MDG827 and Vβ-specific antibody as control) was performed and eGFP expression analyzed using fluorescence analysis. To this end, a binding analysis of anti-TRBC MDG827 and anti-TRBV12-3/4 (Beckman Coulter; #Cat PN IM1148) antibody to UNITABS carrying NY-ESO specific TCR (T11.8-10-17) at position A199, A198, L200, & D202 in loop 1 of TRBC, at position A245, D238, & W240 in loop 2 of TRBC, and at position D213, I209, & P211 in loop 3 of TRAC on Jurkat biosensor cells was carried out.

For the experiment, Jurkat biosensor cells were co-cultured with (T2_Ld_rel_Pep) or without relevant peptide-loaded (T2 unl.) APC (T2) along with controls (negative control: Jurkat only; positive control for complete activation: PMA/lonomycin treated) and subsequently eGFP signal was analyzed by flow cytometry as a read for the TCR activation.

### Method:

To ensure the expression of rTCR, the first step involved transfecting the gamma viral vector (pES12-6) containing the NY-ESO specific TCR (T11.8-10-17) or the NY-ESO specific TCR (T11.8-10-17) with UniTABS epitope (epitope det forth in SEQ ID NO: 47 and flanked with GSG linker sequences as set forth in SEQ ID NO: 54 herein denoted "V1") at positions A199, A198, L200, & D202 in loop 1 of TRBC, at position A245, D238, & W240 in loop 2 of TRBC, and at position D213, I209, & P211 in loop 3 of TRAC were transfected into HEK293FT cells using Mirus transfection reagent (Cat # MIR2305, Lot: 02083632) to generate virus particles. Briefly, a mixture of Mirus transfection reagent and DMEM III was prepared, to which DNA was added for each construct. After an incubation period of about 48 hours at 37°C and 5% CO₂, the virus supernatant (VSN) was collected for transduction.

For transduction, the respective TCR construct VSN was centrifuged to eliminate residual HEK cells, and 1.5 ml/well (24 well plate) of VSN was added to retronectin (Cat # T201, Lot. AGLG003, Takara/Clontech) coated wells. Subsequently, 0.25 Million ieGFP Jurkat-76 -/- CD8+ biosensors cells per/well were seeded onto the well and incubated overnight at 37°C, 5% CO₂. After 72 hours, the transduced cells were used for further analysis (expression analysis via antibody staining and functionality via reading out IFN-γ levels in supernatants when co-cultured with relevant peptide loaded APCs).

For expression analysis, the transduced cells were stained with anti-TRBC MDG827 or anti-TRBV12-3/4 (Beckman Coulter; #Cat PN IM1148) antibody. For anti-TRBC MDG827 antibody staining, briefly, 100k transduced cells were carefully transferred into a well of a 96-well u-bottom plate. The plate was then subjected to centrifugation at 450xg for 5 minutes, and the supernatant was discarded. Following this, the cell pellets were resuspended in 50µl of anti-TRBC MDG827 primary antibody and left to incubate for 30 minutes at 4°C. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Subsequently, 50µl of anti-Rat IgG-FITC labelled secondary antibody, prepared at a 1:20 dilution, was added to the samples and incubated with the samples for 30 minutes in the dark at room temperature. After the incubation, 150µl of FACS buffer was added to all samples, followed by centrifugation at 450xg for 5 minutes and discarding of the supernatant. Finally, 100µl per sample of FACS buffer with 7AAD, diluted at 1:100, was prepared, and the cell pellets were resuspended in FACS-7AAD buffer for subsequent FACS analysis. Similarly, for anti-TRBV12-3/4 (Beckman Coulter; #Cat PN IM1148) antibody staining, 100000 transduced cells were incubated with 50µl of 1:20 diluted PE labeled anti-TRBV12-3/4 (Beckman Coulter; #Cat PN IM1148) antibody and incubated for 30 minutes at 4°C in the dark. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Finally, 100µl per sample of FACS buffer with 7AAD, diluted at 1:100, was prepared, and the cell pellets were resuspended in FACS-7AAD buffer for subsequent FACS analysis. For functional analysis, ieGFP Jurkat-76 -/- CD8+ biosensor cells were transduced with either the NY-ESO specific TCR (T11.8-10-17) or the NY-ESO specific TCR (T11.8-10-17) incorporating Uni-TABS epitope at positions mentioned above. These biosensor cells were then co-cultured with T2 cells loaded with the relevant peptide (T2_Ld_rel_Pep) or without peptide (T2 unl.), alongside controls (negative control: Jurkat only; positive control for complete activation: PMA/lonomycin treated). After a 16-hour incubation period, eGFP signal was assessed via flow cytometry to quantify TCR activation.

### Results:

Both WT and epitope-tagged TCRs were successfully expressed in the Jurkat cells and functionally surface-presented by the cells, as shown in **Figs. 2****,** **3****, and** **4****.** Thus, correct expression, folding, and functionality of the epitope-tagged TCRs were verified. As demonstrated, epitope tags in accordance with the present invention can be successfully inserted while maintaining biological viability and activity/functionality of thusly tagged TCRs.

### Example 2: Analysis of expression and functionality of UNI-TAB-TCR in PBMC

To confirm the UNI-TABS system's universal utility with any rTCR, three additional TCRs (specifically targeting KRASmut G12V (not shown), PRAME-VLD, and WT1 were equipped with the UNITABs epitope in loop1 at position TRBC_A199 and loop 2 at position TRBC_A245 and tested in CD8⁺ T cells.

### Method:

To ensure the expression of rTCR, the first step involved transfecting the gamma viral vector (pES12-6) containing the wildtype PRAME-VLD *(T4.8-1-29)* **(Example2a)** or WT1 **(Example2b)** specific TCR or respective specific TCR with Uni-TABS epitope of the present invention at positions A199 in loop1 or A245 in loop2 of TRBC were transfected into HEK293FT cells using Mirus transfection reagent (Cat # MIR2305, Lot: 02083632) to generate virus particles. Briefly, a mixture of Mirus transfection reagent and DMEM III was prepared, to which DNA was added for each construct. After an incubation period of about 48 hours at 37°C and 5% CO₂, the virus supernatant (VSN) was collected for transduction.

For transduction, the respective TCR construct VSN was centrifuged to eliminate residual HEK cells, and 1.5 ml/well (24 well plate) of VSN was added to retronectin (Cat # T201, Lot. AGLG003, Takara/Clontech) coated wells. Subsequently, 0.25 Million CD8+ cells per/well were seeded onto the well and incubated overnight at 37°C, 5% CO₂. After 72 hours, the transduced cells were used for further analysis (expression analysis via antibody staining and functionality via reading out IFN-γ levels in supernatants when co-cultured with relevant peptide loaded APCs).

For expression analysis, the transduced cells were stained with anti-TRBC MDG827 (PRAME-VLD or WT1 specific TCR) and anti-TRBV9 (Beckman Coulter; #Cat IM2002) (PRAME-VLD specific TCR) antibody. For anti-TRBC MDG827 antibody staining, briefly, 100000 respective transduced CD8+ cells were carefully transferred into a well of a 96-well u-bottom plate. The plate was then subjected to centrifugation at 450xg for 5 minutes, and the supernatant was discarded. Following this, the cell pellets were resuspended in 50µl of anti-TRBC MDG827 primary antibody and left to incubate for 30 minutes at 4°C. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Subsequently, 50µl of anti-Rat IgG-FITC labelled secondary antibody, prepared at a 1:20 dilution, was added to the samples and incubated with the samples for 30 minutes in the dark at room temperature. After the incubation, 150µl of FACS buffer was added to all samples, followed by centrifugation at 450xg for 5 minutes and discarding of the supernatant. Finally, 100µl per sample of FACS buffer with 7AAD, diluted at 1:100, was prepared, and the cell pellets were resuspended in FACS-7AAD buffer for subsequent FACS analysis. Similarly, for anti- TRBV9 (Beckman Coulter; #Cat IM2002) antibody staining (only CD8+ cells transduced with PRAME-VLD specific TCR), 100k transduced cells were incubated with 50µl of 1:20 diluted PE labelled anti- TRBV9 (Beckman Coulter; #Cat IM2002) antibody and incubated for 30 minutes at 4°C in the dark. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Finally, 100µl per sample of FACS buffer with 7AAD, diluted at 1:100, was prepared, and the cell pellets were resuspended in FACS-7AAD buffer for subsequent FACS analysis.

For functional analysis, CD8+ cells transduced with either the PRAME-VLD *(T4.8-1-29)* (Example3a) or WT1 (Example3b) specific TCR or respective specific TCR with Uni-TABS epitope of the present invention at positions A199 in loop1 or A245 in loop2 of TRBC were then co-cultured with T2 cells loaded with the relevant peptide (T2_Ld_rel_Pep) or without peptide (T2 unl.), alongside controls (negative control: CD8+ cells only; positive control for complete activation: PMA/lonomycin treated). After a 16-hour incubation period, the supernatant was collected and 50µl of supernatant was subjected to IFN-γ ELISA assay (BD OptEIA^{™} Human IFN-γ ELISA Set; Cat #555142) to measure the IFN-γ level using the manufacture protocol.

### Example 2a: Binding analysis of anti-TRBC MDG827 (A) and anti-TRBV9 (Beckman Coulter; #Cat IM2002) (B) antibody to PRAME specific TCR (T4.8-1-29) on PRAME-Uni-TABS-TCR transduced PBMCs

A flow cytometric evaluation of PBMCs, transduced with various WT or Uni-TABS epitope V1 (epitope set forth in SEQ ID NO: 47 flanked by GSG linker sequences as set forth in SEQ ID NO: 54) -modified TCRs in loop1 (A199) & loop2 (A245) and a functional evaluation of CD8-enriched PBMCs expressing wildtype or Uni-TABS epitope V1 (epitope set forth in SEQ ID NO: 47 flanked by GSG linker sequences as set forth in SEQ ID NO: 54) -modified PRAME specific TCR (T4.8-1-29) were performed. For the experiment, CD8+ cells were co-cultured with (T2_Ld_rel_Pep) or without (T2 unl.) relevant peptide-loaded APC (T2) in an E:T ratio of 1:2 along with controls (Negative control: CD8 only; Positive control for complete activation: PMA/lonomycin treated) and subsequently INF-γ release was determined using ELISA.

### Example 2b: Binding analysis of anti-TRBC MDG827 (A) antibody to WT1 specific TCR on WT1-Uni-TABS-TCR transduced PBMCs of three donors

A flow cytometric evaluation of PBMCs, transduced with various WT or Uni-TABS (V1) WT1 specific TCR modified in loop1 (A199) & loop2 (A245) and functional evaluation of CD8-enriched PBMCs expressing wildtype or Uni-TABS epitope V1 (epitope tag set forth in SEQ ID NO: 47 flanked by GSG linker sequences as set forth in SEQ ID NO: 54) -modified WT1 specific TCR were performed. For the experiment, CD8+ cells were co-cultured with (T2_Ld_rel_Pep) or without (T2 unl.) relevant peptide-loaded APC (T2) in an E:T ratio of 1:2 along with controls (negative control: CD8 only; positive control for complete activation: PMA/lonomycin treated) and subsequently INF-γ release was determined using ELISA.

### Results:

Exemplary outcomes were achieved for the PRAME-VLD specific TCR (wildtype or-Uni-TABS modified TCR) when introduced into peripheral blood mononuclear cells (PBMCs) derived CD8+ cells from a single donor, as illustrated in Fig. 5. The results depict comparable transduction efficiency (>95%) (Fig. 5a) in CD8+ cells, as determined by TRBV9 (Beckman Coulter; #Cat IM2002) antibody staining, for both the wildtype PRAME-VLD and the UniTABS modified PRAME-VLD specific TCR. Specifically, the UniTABS modified PRAME-VLD transduced CD8+ cells were distinguishable using the anti-TRBC MDG827 antibody (Fig. 5b). Furthermore, both the wildtype and UniTABS modified PRAME-VLD specific TCR exhibited similar levels of IFN-γ in the cocultured supernatant, as assessed via IFN-γ ELISA assay showing similar functionality in CD8+ cells.

Results as obtained in three donors for a WT1 specific TCR are shown in **Fig. 6****.**

Exemplary outcomes were achieved for the WT1 specific TCR (wildtype or -Uni-TABS modified TCR) when introduced into peripheral blood mononuclear cells (PBMCs) derived CD8+ cells from three donors, as illustrated in **Fig. 6****.** The results depict the exclusivity of only anti-TRBC MDG827 staining only in UniTABS modified WT1 specific TCR transduced CD8+ cells **(****Fig. 6a****).** Furthermore, both the wildtype WT1 and UniTABS modified WT1 specific TCR exhibited similar levels of IFN-y in the cocultured supernatant, as assessed via IFN-γ ELISA assay showing similar functionality in CD8+ cells **(****Fig. 6b****).**

To summarize, in both experiments, transduction efficiency, expression of the respective recombinant T cell receptors (rTCRs), and functionality (assessed *via* ELISA) exhibited no significant differences between wildtype and UNI-TABS modified rTCR-transduced PBMCs. The proper expression, folding, and functionality of the epitope-tagged TCRs were thereby confirmed, indicating that epitope tags, as per the present invention, can be effectively incorporated while preserving biological viability and activity/functionality across various TCRs.

### Example 3: Analysis of applicability of further epitope tags

To confirm that the chosen positions are of general utility for a UNI-TABs system, the HA-Tag (epitope tag set forth in SEQ ID NO: 53 flanked by GSG linker sequences as set forth in SEQ ID NO: 60 herein denoted "V7"); commercially antibody available (Biolegend; #cat 901517 or R&D Systems; cat #IC6875G or abcam # ab72479) was placed in integration sites in loop 1 (position: A198, A199, L200, & D202) and loop 2 (position: D238 & W240) of NY-ESO specific TCR using same GSG flexible linker. To ensure that the constructs were properly expressed, folded, and functional, rTCR-deficient ieGFP Jurkat-76 -/- CD8⁺ cells were transduced with the corresponding NY-ESO-HA-Tag constructs. Then, antibody staining (using HA.11-PE antibody and TRBV12.6-specific antibody [control]) was performed and eGFP expression was analyzed using fluorescence analysis.

### Method:

To ensure the expression of rTCR, the first step involved transfecting the gamma viral vector (pES12-6) containing the NY-ESO specific TCR (T11.8-10-17) or the NY-ESO specific TCR (T11.8-10-17) with HA-Tag epitope V7 at positions A199, A198, L200, & D202 in loop 1 of TRBC, at position D238 & W240 in loop 2 of TRBC, and at position I209, & P211 in loop 3 of TRAC were transfected into HEK293FT cells using Mirus transfection reagent (Cat # MIR2305, Lot: 02083632) to generate virus particles. Briefly, a mixture of Mirus transfection reagent and DMEM III was prepared, to which DNA was added for each construct. After an incubation period of about 48 hours at 37°C and 5% CO₂, the virus supernatant (VSN) was collected for transduction.

For transduction, the respective TCR construct VSN was centrifuged to eliminate residual HEK cells, and 1.5 ml/well (24 well plate) of VSN was added to retronectin (Cat # T201, Lot. AGLG003, Takara/Clontech) coated wells. Subsequently, 0.25 Million ieGFP Jurkat-76 -/- CD8+ biosensors cells per/well were seeded onto the well and incubated overnight at 37°C, 5% CO₂. After 72 hours, the transduced cells were used for further analysis (expression analysis via antibody staining and functionality via reading out IFN-γ levels in supernatants when co-cultured with relevant peptide loaded APCs).

For expression analysis, the transduced cells were stained with anti-HA-Tag (Biolegend; #cat 901517) or anti-TRBV12-3/4 (Beckman Coulter; #Cat PN IM1148) antibody. For anti-HA-Tag or anti-TRBV12-3/4 antibody staining, 100000 transduced cells were incubated with 50µl of 1:20 diluted PE labelled anti-HA-Tag or anti-TRBV12-3/4 antibody and incubated for 30 minutes at 4°C in the dark. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Finally, 100µl per sample of FACS buffer with 7AAD, diluted at 1:100, was prepared, and the cell pellets were resuspended in FACS-7AAD buffer for subsequent FACS analysis.

For functional analysis, ieGFP Jurkat-76 -/- CD8+ biosensor cells were transduced with either the NY-ESO specific TCR (T11.8-10-17) or the NY-ESO specific TCR (T11.8-10-17) incorporating HA-Tag epitope at positions mentioned above. These biosensor cells were then co-cultured with T2 cells loaded with the relevant peptide (T2_Ld_rel_Pep) or without peptide (T2 unl.), alongside controls (negative control: Jurkat only; positive control for complete activation: PMA/lonomycin treated). After a 16-hour incubation period, eGFP signal was assessed via flow cytometry to quantify TCR activation.

### Results:

The results are depicted in **Figs. 7-9****.** Correct expression, folding, and functionality for the NY-ESO1 TCR inserted with HA-TAG at positions A199, A198, L200, & D202 in loop 1 of TRBC, at position D238 & W240 in loop 2 of TRBC, and at position I209, & P211 in loop 3 of TRAC, and it was demonstrated that the selected loops in TCR constant region (loop1 , loop2 & loop 3) could be universally used for the insertion of an arbitrary epitope for tagging of TCRs.

### Example 4: insertion of short epitope tags and corresponding analysis

Additionally, to establish the usability of the epitope tag set forth in SEQ ID NO: 47 in shorter versions keeping the important amino acids binding the antibody (EVP), various shorter versions of the epitope set forth in SEQ ID NO: 47 were placed similarly using flexible flanking linker (GSG) in TRBC loop 2 at position A245 in NYESO1 specific TCR and subsequently anti-TRBC MDG827 antibody binding & functionality of rTCR were tested.

### Method:

To ensure the usability of UniTABS epitope set forth in SEQ ID NO: 47 in its shorter version, multiple shorter versions of original UNITABS epitope (epitope tag set forth in SEQ ID NO 47) was inserted in NY-ESO specific TCR at position A245 in loop2, with linker sequence GSG flanking the epitope sequence. For testing the expression of rTCR, the first step involved transfecting the gamma viral vector (pES12-6) containing the NY-ESO specific TCR (T11.8-10-17) or the NY-ESO specific TCR (T11.8-10-17) with shorter versions of UNITABS epitope (epitope tags set forth in SEQ ID 48-52 and flanked by GSG linker sequences as set forth in SEQ ID NOs 55-59 herein denoted V2-V6) at position A245 in loop 2 of TRBC were transfected into HEK293FT cells using Mirus transfection reagent (Cat # MIR2305, Lot: 02083632) to generate virus particles. Briefly, a mixture of Mirus transfection reagent and DMEM III was prepared, to which DNA was added for each construct. After an incubation period of about 48 hours at 37°C and 5% CO₂, the virus supernatant (VSN) was collected for transduction.

For transduction, the respective TCR construct VSN was centrifuged to eliminate residual HEK cells, and 1.5 ml/well (24 well plate) of VSN was added to retronectin (Cat # T201, Lot. AGLG003, Takara/Clontech) coated wells. Subsequently, 0.25 Million ieGFP Jurkat-76 -/- CD8+ biosensors cells per/well were seeded onto the well and incubated overnight at 37°C, 5% CO₂. After 72 hours, the transduced cells were used for further analysis (expression analysis via antibody staining and functionality via reading out IFN-γ levels in supernatants when co-cultured with relevant peptide loaded APCs).

For expression analysis, the transduced cells were stained with anti-TRBC MDG827 or anti-TRBV12-3/4 (Beckman Coulter; #Cat PN IM1148) antibody. For anti-TRBC MDG827 antibody staining, briefly, 100000 transduced cells were carefully transferred into a well of a 96-well u-bottom plate. The plate was then subjected to centrifugation at 450xg for 5 minutes, and the supernatant was discarded. Following this, the cell pellets were resuspended in 50µl of anti-TRBC MDG827 primary antibody and left to incubate for 30 minutes at 4°C. After the incubation period, 150µl of FACS buffer was added to all samples, and the plate was centrifuged again at 450xg for 5 minutes, with the supernatant being discarded afterward. The samples underwent two washes with 150µl of FACS buffer each time, and the supernatant was again discarded after each wash. Subsequently, 50µl of anti-Rat IgG-FITC labelled secondary antibody, prepared at a 1:20 dilution, was added to the samples and incubated with the samples for 30 minutes in the dark at room temperature. After the incubation, 150µl of FACS buffer was added to all samples, followed by centrifugation at 450xg for 5 minutes and discarding of the supernatant. Finally, 100µl per sample of FACS buffer with 7AAD, diluted at 1:100, was prepared, and the cell pellets were resuspended in FACS-7AAD buffer for subsequent FACS analysis.

For functional analysis, ieGFP Jurkat-76 -/- CD8+ biosensor cells were transduced with either the NY-ESO specific TCR (T11.8-10-17) or the NY-ESO specific TCR (T11.8-10-17) incorporating shorter UNITABS epitope at position A245 in TRBC loop2. These biosensor cells were then co-cultured with T2 cells loaded with the relevant peptide (T2_Ld_rel_Pep) or without peptide (T2 unl.), alongside controls (negative control: Jurkat only; positive control for complete activation: PMA/lonomycin treated). After a 16-hour incubation period, eGFP signal was assessed via flow cytometry to quantify TCR activation.

### Results:

The TCRs containing shortened epitopes were able to be stained with the MDG827 antibody (as shown in **Fig. 10A****)** and demonstrated functionality in co-culture tests that was comparable to TCRs equipped with the full-length epitope (set forth in SEQ ID NO: 47) as well as wild type non-modified TCR (as shown in **Figure 10B****).** Applicability and workability of shortened epitope tags was thus also confirmed.

### Example 5: Evaluation of functional abilities of epitope-tagged TCRs

To certify that the insertion of the uniTABS epitope does not change the specificity of the TCR, a mismatch-library assay and an LCL-library assay were performed. The aim was to further compare the WT NY-ESO-1 with two UNI-TABS NY-ESO-TCRs (Position A199 in loop 1 and A245 in loop 2) in combination with PD1_41 BB regarding allogeneic HLA cross-recognition and HLA fine typing.

To test a peptide mismatched library, 336 different mismatched peptides, differing from 1-4 amino acids from the wild-type peptide recognized by the WT TCR were loaded on T2 cells and co-cultured with CD8⁺ T cell transduced with either wt NY-ESO_PD1-41 BB TCR, NY-ESO-UniTABS-A199_PD1-41 BB TCR or NY-ESO-UniTABS-A245_PD1-41 BB TCR. The culture supernatant was collected, and an IFN-γ-ELISA performed.

### Results:

From the tested 336 mismatched peptides, only the 32 peptides listed in Table 2 gave an IFN-γ response to one of the tested constructs. Except for peptides #47, #84 and #280, the peptides recognized by one of the UNI-TAB-PD1-41BB variant TCRs were also recognized by the WT TCR-PD1-41BB construct which shows higher co-relation between wild type NY-ESO specif TCR and uniTABS modified NYESO TCR (UniTABS-A199 & uniTABS-A245). Regarding peptide #47, this is recognized by NY-ESO-A199_PD1-41BB TCR transduced T cells of only one donor, same is true for #280. Peptide #84 is recognized by NY-ESO-A245_PD1-41 BB TCR transduced T cells.

Taking into account that some peptides are only recognized by WT TCR transduced T cells of one donor (peptides #7, #8, #13, #24, #314), it is unlikely that the recognition of the peptides #47, #84 and #280 is really due to a change in the specificity of the UNI-TABS TCRs but rather shows the limitations of the test system.

Table 2 shows the IFNg-ELISA results of co-cultures of CD8⁺ T cells transduced with WT NY-ESO_PD1-41 BB TCR ("SWAT"), NY-ESO-A199_PD1-41 BB TCR (UniTAB1) and NY-ESO-A245_PD1-41 BB TCR (UniTAB2) results in two donors (MIT038 and MIT045). The sequences and the numbers of the respective mismatch peptides are listed. The cells contain the values of IFN-γ [pg/ml production]. BetaA199: UniTAB epitope set forth in SEQ ID NO: 47 inserted in loop1 at position A199 flanked by GSG linker sequences, BetaA245: UniTAB epitope set forth in SEQ ID NO: 47 inserted in loop 2 at position A245 flanked by GSG linker sequences.

**Table 2: Mismatched peptides recognized by NY-ESO WT and NY-ESO variants**

| **MM Screen** | | | | | | | |
|---|---|---|---|---|---|---|---|
| **MIT038 SWAT** | **MIT038 UniTABs-A199** | **MIT 045 SWAT** | **MIT 045 UniTABs-A199** | **MIT 045 UniTABs-A245** | # | **peptide sequence** | **SEQ ID NO** |
| | | 24.0**** | | | 7**** | YLNMWITTC**** | 109**** |
| | | 24.7**** | | | 8**** | ICLQWITQC**** | 110**** |
| | 257.6*** | 26.5*** | | | 9*** | **SLLFWILIC***** | **111***** |
| | | 20.8 | | | 13 | SLCMVITIY | 112 |
| 282.0* | 520.4* | 47.1* | 306.3* | 0.0* | 15* | SLLLWPTRL* | 113* |
| | | 24.3 | | | 24 | GLLNWITGA | 114 |
| 2126.8* | 2419.6* | 886.8* | 1556.6* | 0.0* | 31* | SLLMSIHNI* | 115* |
| 297.8* | 555.0* | 55.6* | 680.4* | 0.0* | 41* | SLLMTISLL* | 116* |
| 1502.6* | 1407.2* | 176.7* | 1515.5* | 0.0* | 45* | SILMYITSL* | 117* |
| | | | 530.1 | | 47 | SLLQWVTSR | 118 |
| 819.0* | 942.2* | 88.3* | 918.4* | 0.0* | 62* | SLLMWMLRL* | 119* |
| 768.9* | 824.7* | 149.7* | 938.8* | 0.0* | 76* | PLLVWITTL* | 120* |
| 292.3* | 369.6* | 40.3* | | 0.0* | 79* | **LLLMIITFW*** | **121*** |
| | | | | 0.0**** | 84**** | SLLYVIHTC**** | 122**** |
| | | 32.4*** | 257.0*** | | 116*** | **YLLYGITHC***** | **123***** |
| 3622.0* | 2804.4* | 1289.9* | 2839.1* | 0.0* | 160* | SLMYWITIQ* | 124* |
| 788.9* | 900.2* | 100.6* | 889.3* | 0.0* | 221* | NLLFWICGC* | 125* |
| 234.5* | 484.5* | 35.4* | 1200.3* | 0.0* | 228* | **NLKMWIQEC*** | **126*** |
| 255.6* | 476.1* | 32.3* | 400.5* | | 236* | **YLKMSIAQC*** | **127*** |
| 228.6** | 312.2** | 32.1** | | | 239** | **SLLCYIKHC**** | **128**** |
| 236.7** | 386.0** | 23.4** | | | 240** | **SLQMGYTQG**** | **129**** |
| 380.2* | 498.7* | 47.2* | 355.7* | 0.0* | 243* | SLLSSVTQG* | 130* |
| 441.4* | 387.4* | 45.4* | 261.1* | 0.0* | 244* | SLLSHITSS* | 131* |
| 321.2* | 344.8* | 40.9* | | 0.0* | 246* | SLLMLIAMS* | 132* |
| 681.2* | 536.8* | 80.5* | 372.5* | 0.0* | 247* | SLDMWRLGC* | 133* |
| 383.1* | 407.2* | 57.9* | | 0.0* | 257* | SILVWTTQP* | 134* |
| 366.5** | 400.3** | 43.2** | | | 265** | SLLMKIFHS** | 135** |
| 463.7* | 441.8* | 50.8* | | 0.0* | 278* | SLLSKITFD* | 136* |
| | 255.7**** | | | | 280**** | SLLEVLTQD**** | 137**** |
| | | 26.7**** | | | 314**** | ELLMWPLQE**** | 138**** |
| 3699.1* | 3165.9* | 658.2* | 2995.1* | 0.0* | 323* | GLLQWITNL* | 139* |
| 431.9* | 756.9* | | 627.6* | 0.0* | 324* | **SLLMPWTSL*** | **140*** |
| *recognition by 4-5 TCRs | | | | 19 | | | |
| **recognition by 3 TCRs | | | | 3 | | | |
| ***recognition by 2 TCRs | | | | 2 | | | |
| ****recognition by 1 TCR | | | | 8 | | | |

### Example 6: Testing of cross-recognition

To test the cross-recognition of allogeneic HLA molecules, 54 different LCL cell lines (either unloaded or loaded with relevant peptide) were co-cultured with CD8⁺T cell transduced with either wt NY-ESO_PD1-41BB TCR, NY-ESO-A199_PD1-41BB TCR and NY-ESO-A245_PD1-41BB TCR. The culture supernatant was collected and an IFN-γ-ELISA performed.

The analysis of the LCL-library (consisting of 54 different cell lines) revealed no change in allogeneic MHC cross-recognition and MHC fine typing compared to WT NY-ESO. An example is given in **Fig. 11****.**

### Results:

Cell lines #12 and #21 are recognized by WT TCR and UNI-TAB-TCR transduced CD8⁺ T cells of one donor (MIT_45) **(****Fig.11A****)** to the same extent. The loaded cell lines **(****Fig.11B****)** are equally well recognized by WT TCR and UNI-TAB-TCR transduced CD8⁺ T cells of both donors. Since there are no significant differences in the recognition, the statement that the insertion of the UNI-TABS epitope set forth in SEQ ID NO: 47 does not change the fine specificity of restriction is valid.

### Example 7: Evaluation of functional abilities of further epitope-tagged TCRs according to the invention (Fig. 12 - 16)

### Viral Transduction of Jurkat 76 -/-CD8+ ieGFP (Jurkat Biosensor) cells

HEK293FT cells were transfected with DNA encoding the respective transgenes (epitope tag-modified TCR inserted with either 9-mer UniTope tag with linker (GSGGEVPKDRFSGSG) **(****Fig.12-14****)** or 6-mer UniTope tag with linker (GSGEVPKDRGSG) **(****Fig.15****)** or 12-mer UniTope tag without linker (DKGEVPKDRFSA) **(****Fig.16****)** at different positions in the beta constant chain of KRASG12V specific TCR (T47.8-041-071)), Gag/pol (packaging plasmid pcDNA3.1(+)_gag/pol encoding gag and pol gene), and Mirus transfection reagent (manufacturer: Mirus Bio LLC). Jurkat biosensor cells were collected and transduced with the HEL293FT-derived viral supernatant on retronectin-coated plates at 32 °C *via* spinoculation.

### Assessment of transduction efficiency by antibody staining and flow cytometry

Transduced Jurkat biosensor cells were collected, washed with FACS buffer (D-PBS + 1% FCS), and labelled with anti-CD8 antibodies in combination with either anti-UniTope MDG827 antibody (also referred to herein as "anti-TRBC MDG827"), anti-TRBV5-5 antibody (Beckman Coulter, #cat IM2002) or Tetramer (Multimer) (PE KRAS G12V7-16 Tetramer (VWGAVGVGK/A1101, ImmunAware)), respectively. Samples were measured with a Fortessa LSR II and data were analyzed with FlowJo v10.8 (both BD Biosciences, Franklin Lakes, USA).

### Assessment of functionality of tagged TCR by flow cytometry

Analysis of expression and functionality of epitope-tag modified TCR inserted with either 9-mer UniTope tag with linkers (GSGGEVPKDRFSGSG) **(****Fig. 12-14****)** or 6-mer UniTope tag with linkers (GSGEVPKDRGSG)(**Fig.15**) or 12-mer UniTope tag without linker (DKGEVPKDRFSA) (Fig.16) at different position in beta constant chain of KRASG12V specific TCR1 (T47.8-041-071) in Jurkat biosensor.

To ensure that the constructs in accordance with the present invention were properly expressed, folded, and functional, rTCR-deficient ieGFP Jurkat-76 -/- CD8+ cells (also known as biosensors) were transduced with the corresponding epitope-tag modified KRASG12V specific TCR1 (T47.8-041-071) constructs. Then, antibody staining (using MDG827, Multimer and anti-TRBV5-5 antibody (Beckman Coulter, #cat IM2002) was performed and eGFP expression analyzed using fluorescence analysis. To this end, a binding analysis of anti-TRBC MDG827 (also referred to herein as "anti-TRBC MDG827"), Multimer and anti-TRBV5-5 antibody to epitope tag-modified KRASG12V specific TCR1 (T47.8-041-071) inserted with either 9-mer epitope tag with linkers (GSGGEVPKDRFSGSG) **(****Fig. 12****,** **13** **&** **14****)** or 6-mer epitope tag with linker (GSGEVPKDRGSG) (Fig. 15) or 12-mer epitope tag without linker (DKGEVPKDRFSA) (Fig. 16) at different positions in beta constant chain of KRASG12V specific TCR1 (T47.8-041-071) in Jurkat 76 -/-CD8⁺ ieGFP.

For the experiment, Jurkat biosensor cells were co-cultured with K562-A1 1 loaded with relevant peptide (K562-A11_Ld_rel_Pep) or without relevant peptide-loaded (K562-A11 unl.) along with controls (negative control: Jurkat only; positive control for complete activation: PMA/lonomycin treated) and subsequently eGFP signal was analyzed by flow cytometry as a read for the TCR activation.

### Example 8: Evaluation of functional abilities of further epitope-tagged TCRs according to the invention

### Viral Transduction of CD8+ T cells

HEK293FT cells were transfected with DNA encoding the respective transgenes, Gag/pol (packaging plasmid pcDNA3.1(+) gag/pol encoding gag and pol gene), and Mirus transfection reagent (manufacturer: Mirus Bio LLC). PBMC-derived human CD8⁺ T were activated with anti-CD3/anti-CD28-targeting Dynabeads (Dynabeads human T-Activator CD3/CD28; manufacturer: Gibco) and 200 U/ml IL-2 (Proleukin-S, manufacturer: Clinigen) for 48 h. Activated CD8⁺ T cells were collected and transduced with the HEL293FT-derived viral supernatant on retronectin-coated plates at 32 °C *via* spinoculation.

### Assessment of transduction efficiency by antibody staining and flow cytometry

CD8⁺ T cells were collected, washed with FACS buffer (D-PBS + 1% FCS), and labelled with a viability dye, anti-CD3, and anti-CD8 antibodies in combination with either anti-TRBC MDG827 antibody (also referred to herein as "anti-TRBC MDG827"), anti-PD-1 antibody or Tetramer, respectively. Samples were measured with a Fortessa LSR II and data were analyzed with FlowJo v10.8 (both BD Biosciences, Franklin Lakes, USA).

### Assessment of transduction efficiency by antibody staining and flow cytometry

Specific IFN-γ release was determined *via* IFN-γ enzyme-linked immunosorbent assay (ELISA). Tumor cell lines derived from various tumor indications expressing either endogenous levels of mKRAS G12V antigen (DAN-G, NCI-H441, SW480) or KRAS wild type (DU145) were selected for this co-culture experiment. After 20 h of co-culture, supernatant samples were collected and stored at -20 °C until measurement. IFN-γ ELISA was performed according to the manufacturer's instructions. Optical density was measured using the Multiskan^{™} FC Microplate Photometer and data were evaluated by the Skanlt^{™} Software (both Thermo Scientific^{™}, Waltham, MA, USA). Additional calculations were performed using Microsoft Excel^{®} and GraphPad^{®} Prism 8 software.

### Results for TCR 1 / TCR 3 / TCR 6 tagging

### Ex. I.1: Specific and reliable detection of the epitope tag in KRASG12V specific TCRs (TCR 1/ TCR 3 / TCR 6 + epitope tag) in transduced T cells

A potential benefit of the epitope tag of the present invention is the reliable and specific detection of transduced T cells containing the epitope tag. Labelling with the anti-TRBC MDG827 antibody and analysis by flow cytometry **(****Fig. 17****)** detects T cells bearing TCR 1 (reference in the context of TCR1 being made to EP23197780.2) / TCR 3 (reference in the context of TCR3 being made to EP23197778.6) / TCR 6 (reference in the context of TCR6 being made to EP23197786.9) containing the 9mer-UniTope with 'GSG' linker (GSGGEVPKDRFSGSG) at position D202 in loop1 of TCR beta constant chain but not T cells bearing TCR 1 / TCR 3 / TCR 6 without the epitope tag or untransduced T cells.

### Ex. I.2: Expression of the transgenic KRASG12V specific TCRs (TCR 1 / TCR 3 / TCR 6) in transduced T cells

Labelling with the mKRAS7-16 G12V (10-mer) HLA-A*11:01 tetramer (PE KRAS G12V₇₋₁₆ Tetramer (VWGAVGVGK/A1101, ImmunAware)) and analysis by flow cytometry confirmed expression of TCR 1 / TCR 3 / TCR 6 by T cells transduced with TCR 1 / TCR 3 / TCR 6 or TCR 1 / TCR 3 / TCR 6 + 9mer-UniTope with 'GSG' linker (GSGGEVPKDRFSGSG) at position D202 in loop1 of TCR beta constant chain **(****Fig. 18****).** Almost all untransduced T cells (< 0.5 %) were not labelled by tetramer staining.

### Ex. I.3: Comparably high and specific IFN-γ secretion in response to tumor cells in KRASG12V specific TCRs (TCR 1 / TCR 3 / TCR 6 and TCR 1 / TCR 3 / TCR 6 + 9mer-UniTope) transduced T cells.

To be potentially useful in a clinical setting, it is essential that T cells expressing the candidate TCR, can specifically recognize tumor cells expressing the target antigen. A standard way to assess tumor cell recognition by TCR-transgenic T cells is the evaluation of IFN-γ release by T cells upon target antigen-specific stimulation. The maintenance of a high and specific IFN-γ response is a key parameter for successful insertion of the 9mer- UniTope with 'GSG' linker (GSGGEVPKDRFSGSG) sequence into the TCR. Tumor cell lines derived from various cancers (pancreatic, lung and colorectal tumors) expressing mKRAS endogenous levels of G12V were selected as target cells for this co-culture experiment. In addition, one tumor cell line expressing only KRAS wild type served as mKRAS G12V negative tumor target cell and therefore negative control. All tumor cell lines were transduced with HLA-A*11:01-encoded molecules to allow surface expression of the desired HLA molecules. The co-culture experiment was set up by using the described tumor cell lines and TCR-transduced CD8⁺ T cells or TCR + 9mer- UniTope (GSGGEVPKDRFSGSG) - transduced CD8⁺ T cells. The 9mer- UniTope sequence with 'GSG' linker was inserted at position D202 in TCR beta constant chain. Untransduced CD8⁺ T cells served as negative control. IFN-γ secretion was assessed 20 h after setting up the co-culture. IFN-γ release was observed only when TCR-transduced CD8⁺ T cells were stimulated with KRAS G12V-positive tumor cells **(****Fig. 19****).** IFN-γ release was comparable in T cells expressing TCR 1 / TCR 3 / TCR 6 alone and T cells expressing TCR 1 / TCR 3 / TCR 6 + epitope tag, respectively. No recognition of the KRAS wild type tumor cells was observed with both T cell populations. These findings confirmed the maintenance of specificity of KRASG12V specific TCRs (TCR 1 / TCR 3 / TCR 6) after insertion of the UniTope sequence and its capacity to be activated and subsequently drive release of IFN-γ in response to tumor cells expressing endogenous levels of mKRAS G12V.

Overall, the presented invention is universally applicable, allowing tagging of TCRs without changing their functionality or specificity.

The description further comprises the following embodiments:
Embodiment 1: T cell receptor or fragment thereof comprising the constant region of the TCR, and further comprising, in said constant region, at least one epitope tag, wherein the insert position of the epitope tag is located in the alpha constant region (TRAC) and/or the beta constant region (TRBC).
Embodiment 2. TCR according to embodiment 1, wherein the TCR is isolated and/or purified, possibly soluble or membrane bound.
Embodiment 3. TCR according to any one of the preceding embodiments, wherein the insert position of the at least one epitope tag is comprised in the alpha constant region (TRAC) and the beta constant region (TRBC).
Embodiment 4. TCR according to any one of embodiments 1 to 3, wherein the insert position of the at least one epitope tag is comprised in the alpha constant region (TRAC) or the beta constant region (TRBC).
Embodiment 5. TCR according to any one of the preceding embodiments, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and/or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and/or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 6. TCR according to any one of the preceding embodiments, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 7. TCR according to any one of embodiments 1 to 5, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 8. TCR according to any one of the preceding embodiments, wherein
   - the insert position is located at any position within the amino acid sequence set forth in SEQ ID NO: 145 and is located within the alpha constant region of the TCR; and/or
   - the insert position is located at any position within the amino acid sequence set forth in SEQ ID NO: 146 and is located within the beta constant region of the TCR; and/or
   - the insert position is located at any position within the amino acid sequence set forth in SEQ ID NO: 147 and is located within the beta constant region of the TCR.
Embodiment 9. TCR according to embodiment 8, wherein
   - the insert position is located at any position within the amino acid sequence set forth in SEQ ID NO: 145 and is located within the alpha constant region of the TCR; or
   - the insert position is located at any position within the amino acid sequence set forth in SEQ ID NO: 146 and is located within the beta constant region of the TCR; or
   - the insert position is located at any position within the amino acid sequence set forth in SEQ ID NO: 147 and is located within the beta constant region of the TCR.
Embodiment 10. TCR according to embodiment 8, wherein
   - the insert position is located at any position within the amino acid sequence set forth in SEQ ID NO: 145 and is located within the alpha constant region of the TCR; and
   - the insert position is located at any position within the amino acid sequence set forth in SEQ ID NO: 146 and is located within the beta constant region of the TCR; and
   - the insert position is located at any position within the amino acid sequence set forth in SEQ ID NO: 147 and is located within the beta constant region of the TCR.
Embodiment 11. TCR according to any one of the preceding embodiments, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 148 and/or
   - followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149.
Embodiment 12. TCR according to any one of the preceding embodiments, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 150 and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151.
Embodiment 13. TCR according to any one of the preceding embodiments, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 152 and/or
   - followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.
Embodiment 14. TCR according to any one of embodiments 11 to 13, wherein the amino acid sequence that is the preceded by an amino acid sequence as defined in embodiments 11 to 13 is from 12 to 20, preferably from 14 to 18, such as 14, 15, 16, 17, or 18 amino acids of length, most preferably 14, 15, or 18 amino acids of length.
Embodiment 15. TCR according to any one of embodiments 11 to 14, wherein the amino acid sequence that is followed by an amino acid sequence as defined in embodiments 11 to 13 is from 12 to 20, preferably from 14 to 18, such as 14, 15, 16, 17, or 18 amino acids of length, most preferably 14, 15, or 18 amino acids of length.
Embodiment 16. TCR according to embodiment 14 or 15, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 148, wherein the amino acid sequence that is preceded is 12 to 16 amino acids of length, preferably 14 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 149, wherein the amino acid sequence that is followed is 12 to 16 amino acids of length, preferably 14 amino acids of length.
Embodiment 17. TCR according to any one of embodiments 14 to 16, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 150, wherein the amino acid sequence that is preceded and/or followed is 13 to 17 amino acids of length, preferably 15 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151, wherein the amino acid sequence that is followed is 13 to 17 amino acids of length, preferably 15 amino acids of length.
Embodiment 18. TCR according to any one of embodiments 14 to 17, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 152, wherein the amino acid sequence that is preceded is 16 to 20 amino acids of length, preferably 18 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 153, wherein the amino acid sequence that is followed is 16 to 20 amino acids of length, preferably 18 amino acids of length.
Embodiment 19. TCR according to any one of the preceding embodiments, wherein the epitope tag is inserted by means of linker sequences, preferably short glycine-rich linker sequences, more preferably glycine-rich linker sequences consisting of 2 to 6, preferably 3 to 5 amino acids, more preferably comprising or consisting of the amino acid sequence GSG.
Embodiment 20. TCR according to any one of the preceding embodiments, wherein the epitope tag comprises 4 to 17 amino acids, preferably 5 to 15 amino acids, more preferably 6 to 14 amino acids.
Embodiment 21. TCR according to any one of the preceding embodiments, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 155, SEQ ID NO: 1, SEQ ID NO: 2, SEQ ID NO: 3, and SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 159, SEQ ID NO: 5, SEQ ID NO: 6, SEQ ID NO: 7, and SEQ ID NO: 8 of the TCR beta constant region.
Embodiment 22. TCR according to embodiment 21, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 155 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 159 of the TCR beta constant region.
Embodiment 23. TCR according to any one of embodiments 1 to 22, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 1 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 5 of the TCR beta constant region.
Embodiment 24. TCR according to embodiment 21, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 2 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 6 of the TCR beta constant region.
Embodiment 25. TCR according to embodiment 21, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 3 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 7 of the TCR beta constant region.
Embodiment 26. TCR according to embodiment 21, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 8 of the TCR beta constant region.
Embodiment 27. TCR according to any one of embodiments 1 to 26, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 156, SEQ ID NO: 9, SEQ ID NO: 157, SEQ ID NO: 10, SEQ ID NO: 158, and SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 160, SEQ ID NO: 12, SEQ ID NO: 161, SEQ ID NO: 13, SEQ ID NO: 162, and SEQ ID NO: 14 of the TCR beta constant region.
Embodiment 28. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 156 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 160 of the TCR beta constant region.
Embodiment 29. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 9 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 12 of the TCR beta constant region.
Embodiment 30. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 157 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 161 of the TCR beta constant region.
Embodiment 31. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 10 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 13 of the TCR beta constant region.
Embodiment 32. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 158 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 162 of the TCR beta constant region.
Embodiment 33. TCR according to embodiment 27, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 14 of the TCR beta constant region.
Embodiment 34. TCR according to any one of embodiments 1 to 33, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15, SEQ ID NO: 16, and SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18, SEQ ID NO: 19, and SEQ ID NO: 20 of the TCR alpha constant region.
Embodiment 35. TCR according to embodiment 34, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18 of the TCR alpha constant region.
Embodiment 36. TCR according to embodiment 34, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 16 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 19 of the TCR alpha constant region.
Embodiment 37. TCR according to embodiment 34, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 20 of the TCR alpha constant region.
Embodiment 38. TCR according to any one of embodiments 1 to 37, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 21 and SEQ ID NO: 22 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 23 and SEQ ID NO: 24 of the TCR alpha constant region.
Embodiment 39. TCR according to any one of the preceding embodiments, wherein the insert position is located
   - within the amino acid sequence ranging from positions 190 to 204, preferably positions 192 to 203 of the TCR beta chain; and/or
   - within the amino acid sequence ranging from positions 232 to 249, preferably positions 234 to 247 of the TCR beta chain; and/or
   - within the amino acid sequence ranging from positions 205 to 218, preferably positions 207 to 215 of the TCR alpha chain.
Embodiment 40. TCR according to embodiment 39, wherein the insert position is located
   - within the amino acid sequence ranging from positions 190 to 204, preferably positions 192 to 203 of the TCR beta chain; and
   - within the amino acid sequence ranging from positions 232 to 249, preferably positions 234 to 247 of the TCR beta chain; and
   - within the amino acid sequence ranging from positions 205 to 218, preferably positions 207 to 215 of the TCR alpha chain.
Embodiment 41. TCR according to embodiment 39, wherein the insert position is located
   - within the amino acid sequence ranging from positions 190 to 204, preferably positions 192 to 203 of the TCR beta chain; or
   - within the amino acid sequence ranging from positions 232 to 249, preferably positions 234 to 247 of the TCR beta chain; or
   - within the amino acid sequence ranging from positions 205 to 218, preferably positions 207 to 215 of the TCR alpha chain.
Embodiment 42. TCR according to embodiment 39, wherein the insert position is located
   - within the amino acid sequence ranging from positions 196 to 202 of the TCR beta chain; and/or
   - within the amino acid sequence ranging from positions 236 to 245 of the TCR beta chain; and/or
   - within the amino acid sequence ranging from positions 209 to 213 of the TCR alpha chain.
Embodiment 43. TCR according to embodiment 42, wherein the insert position is located
   - within the amino acid sequence ranging from positions 196 to 202 of the TCR beta chain; and
   - within the amino acid sequence ranging from positions 236 to 245 of the TCR beta chain; and
   - within the amino acid sequence ranging from positions 209 to 213 of the TCR alpha chain.
Embodiment 44. TCR according to embodiment 42, wherein the insert position is located
   - within the amino acid sequence ranging from positions 196 to 202 of the TCR beta chain; or
   - within the amino acid sequence ranging from positions 236 to 245 of the TCR beta chain; or
   - within the amino acid sequence ranging from positions 209 to 213 of the TCR alpha chain.
Embodiment 45. TCR according to any one of the preceding embodiments, wherein the insert position is located at
   - a position selected from the group consisting of positions 196, 198, 199, 200, and 202 of the TCR beta chain; and/or
   - a position selected from the group consisting of positions 236, 238, 239, 240, 243, and 245 of the TCR beta chain; and/or
   - a position selected from the group consisting of positions 209, 211, and 213 of the TCR alpha chain.
Embodiment 46. TCR according to embodiment 45, wherein the insert position is located at
   - a position selected from the group consisting of positions 196, 198, 199, 200, and 202 of the TCR beta chain; and
   - a position selected from the group consisting of positions 236, 238, 239, 240, 243, and 245 of the TCR beta chain; and
   - a position selected from the group consisting of positions 209, 211, and 213 of the TCR alpha chain.
Embodiment 47. TCR according to embodiment 45, wherein the insert position is located at
   - a position selected from the group consisting of positions 196, 198, 199, 200, and 202 of the TCR beta chain; or
   - a position selected from the group consisting of positions 236, 238, 239, 240, 243, and 245 of the TCR beta chain; or
   - a position selected from the group consisting of positions 209, 211, and 213 of the TCR alpha chain.
Embodiment 48. TCR according to any one of the preceding embodiments, wherein the epitope tag sequence comprises 4 to 17 amino acids, such as 4, 5, 6, 7, 8, 9 10, 11, 12, 13, 14, 15, 16, or 17 amino acids, preferably 5 to 15 amino acids, more preferably 6 to 14 amino acids, for instance 6, 7, 8, 9, 10, 11 or 12 amino acids, such as 6, 9, 10, 11 or 12 amino acids.
Embodiment 49. TCR according to any one of the preceding embodiments, wherein the epitope tag comprises an amino acid sequence having at least 80 %, preferably at least 85 %, more preferably at least 90 %, for instance 100 % sequence identity to the amino acid sequence set forth in SEQ ID NO: 25 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 50. TCR according to any one of the preceding embodiments, wherein the epitope tag comprises an amino acid sequence having at least 80 %, preferably at least 85 %, more preferably at least 90 %, for instance 100 % sequence identity to the amino acid sequence set forth in SEQ ID NO: 26 (GEVPKX₁RFS), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 51. TCR according to any one of the preceding embodiments, wherein the epitope tag consists of an amino acid sequence having at least 80 %, preferably at least 85 %, more preferably at least 90 %, for instance 100 % sequence identity to the amino acid sequence set forth in SEQ ID NO: 26 (GEVPKX₁RFS), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 52. TCR according to any one of the preceding embodiments, wherein the epitope tag comprises or consists of the amino acid sequence set forth in SEQ ID NO: 27 (X₂X₃GEVPKX₁RFSX₄), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).
Embodiment 53. TCR according to any one of the preceding embodiments, wherein the epitope tag comprises an amino acid sequence selected from:
   - DX₃GEVPKX₁RFSX₄ (SEQ ID NO: 28);
   - EX₃GEVPKX₁RFSX₄ (SEQ ID NO: 29);
   - X₂KGEVPKX₁RFSX₄ (SEQ ID NO: 30);
   - X₂RGEVPKX₁RFSX₄ (SEQ ID NO: 31);
   - X₂HGEVPKX₁RFSX₄ (SEQ ID NO: 32);
   - X₂X₃GEVPKX₁RFSG (SEQ ID NO: 33);
   - X₂X₃GEVPKX₁RFSL (SEQ ID NO: 34);
   - X₂X₃GEVPKX₁RFSA (SEQ ID NO: 35);
   - X₂X₃GEVPKX₁RFSV (SEQ ID NO: 36;
   - X₂X₃GEVPKX₁RFSM (SEQ ID NO: 37);
   - X₂X₃GEVPKX₁RFSI (SEQ ID NO: 38);
   - X₂X₃GEVPKX₁RFSS (SEQ ID NO: 39);
   - X₂X₃GEVPKX₁RFST (SEQ ID NO: 40),
   wherein, where applicable, wherein X₁ is selected from A, R, N, D, C, Q, E, G, H, I, L, K, M, F, P, S, T, W, Y, V; X₂ is selected from D and E; X₃ is selected from K, R, and H; and X₄ is selected from G, L, A, V, M, I, S, and T.
Embodiment 54. TCR according to any one of the preceding embodiments, wherein the epitope tag comprises an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52 or wherein the epitope tag comprises an amino acid sequence having at least about 60 %, for instance at least about 66 % or about 67 %, preferably at least about 80 %, such as about 83 % or about 84 %, preferably at least 90 %, for instance 100 % sequence identity to the amino acid sequence set forth in SEQ ID NO: 154.
Embodiment 55. TCR according to any one of the preceding embodiments, wherein the epitope tag consists of an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52 or wherein the epitope tag comprises or consists of an amino acid sequence having at least about 60 %, for instance at least about 66 % or about 67 %, preferably at least about 80 %, such as about 83 % or about 84 %, preferably at least 90 %, for instance 100 % sequence identity to the amino acid sequence set forth in SEQ ID NO: 154.
Embodiment 56. TCR according to any one of embodiments 1 to 48, wherein the epitope tag comprises an amino acid sequence having at least 80 %, preferably at least 85 %, more preferably at least 90 %, for instance 100 % sequence identity to an amino acid sequence selected from the group consisting of SEQ ID NO: 53.
Embodiment 57. TCR according to embodiment 56, wherein the epitope tag consists of an amino acid sequence having at least 80 %, preferably at least 85 %, more preferably at least 90 %, for instance 100 % sequence identity to an amino acid sequence selected from the group consisting of SEQ ID NO: 53.
Embodiment 58. TCR according to any one of the preceding embodiments, wherein integration of the epitope tag does not interfere with structural integrity and/or functionality of the TCR.
Embodiment 59. TCR according to any one of the preceding embodiments, wherein its amino acid sequence is modified to comprise a detectable label, a therapeutic agent or pharmacokinetic modifying moiety.
Embodiment 60. TCR according to embodiment 59, wherein the therapeutic agent is selected from the group consisting of an immune effector molecule, a cytotoxic agent and a radionuclide.
Embodiment 61. TCR according to embodiment 60, wherein the immune effector molecule is a cytokine.
Embodiment 62. TCR according to embodiment 59, wherein the pharmacokinetic modifying moiety is at least one polyethylene glycol repeating unit, at least one glycol group, at least one sialyl group or a combination thereof.
Embodiment 63. TCR according to any one of the preceding embodiments, wherein the TCR is of the single chain type, wherein the TCR α chain and the TCR β chain are linked by a linker sequence.
Embodiment 64. TCR according to any one of the preceding embodiments, wherein the TCR is modified, preferably wherein the TCR α chain and/or the TCR β chain is modified.
Embodiment 65. TCR according to any one of the preceding embodiments, wherein the recombinant TCR sequence may be modified to contain minimal murinized Cα and Cβ regions.
Embodiment 66. Isolated polypeptide comprising at least one epitope tag and a functional portion of the TCR or fragment thereof according to any one of embodiments 1 to 65.
Embodiment 67. Multivalent TCR complex, comprising a least two TCRs as embodied in any one of embodiments 1 to 65.
Embodiment 68. Multivalent TCR complex, wherein at least one of said TCRs is associated with a therapeutic agent.
Embodiment 69. Nucleic acid encoding a TCR according to any one of embodiments 1 to 65 or encoding the polypeptide according to embodiment 66.
Embodiment 70. Nucleic acid according to embodiment 69, wherein the nucleic acid is codon optimized.
Embodiment 71. Vector comprising the nucleic acid of embodiment 69 or 70.
Embodiment 72. Vector according to embodiment 71, wherein the vector is an expression vector.
Embodiment 73. Vector according to embodiment 71 or 72, wherein the vector is a retroviral vector.
Embodiment 74. Vector according to embodiment 71 or 72, wherein the vector is a lentiviral vector.
Embodiment 75. Cell expressing the TCR according to embodiments 1 to 65.
Embodiment 76. Cell according to embodiment 75, wherein the cell is isolated or non-naturally occurring.
Embodiment 77. Cell comprising the nucleic acid according to embodiments 69 or 70 or the vector according to embodiments 71 to 74.
Embodiment 78. Cell according to embodiments 75 to 77, wherein the cell comprises:
   a) an expression vector which comprises at least one nucleic acid as embodied in embodiment 69 or 70.
   b) a first expression vector which comprises a nucleic acid encoding the alpha chain of the TCR as embodied in any one of the embodiments 1 to 65, and a second expression vector which comprises a nucleic acid encoding the beta chain of a TCR as embodied in any one of the embodiments 1 to 65.
Embodiment 79. Cell according to any one of embodiments 75 to 78, wherein the cell is a peripheral blood lymphocyte (PBL) or a peripheral blood mononuclear cell (PBMC).
Embodiment 80. Cell according to any one of embodiments 75 to 78, wherein the cell is a T cell.
Embodiment 81. Pharmaceutical composition comprising the TCR according to embodiments 1 to 65, the polypeptide according to embodiment 66, the multivalent TCR complex according to embodiment 67 or 68, the nucleic acid according to embodiment 69 or 70, the vector according to embodiments 71 to 74, or the cell according to any one of embodiments 75 to 80.
Embodiment 82. Pharmaceutical composition according to embodiment 81, wherein the pharmaceutical composition comprises at least one pharmaceutically acceptable carrier.
Embodiment 83. The TCR according to embodiments 1 to 65, the polypeptide according to embodiment 66, the multivalent TCR complex according to embodiment 67 or 68, the nucleic acid according to embodiment 69 or 70, the vector according to any one of embodiments 71 to 74, or the cell according to any one of embodiments 75 to 80 for use as a medicament.
Embodiment 84. The TCR according to embodiments 1 to 65, the polypeptide according to embodiment 66, the multivalent TCR complex according to embodiment 67 or 68, the nucleic acid according to embodiment 69 or 70, the vector according to any one of embodiments 71 to 74, or the cell according to any one of embodiments 75 to 80 for use in the treatment of cancer.
Embodiment 85. The TCR, the polypeptide, the multivalent TCR complex, the nucleic acid or the cell for use according to embodiment 84, wherein the cancer is a hematological cancer.
Embodiment 86. The TCR, the polypeptide, the multivalent TCR complex, the nucleic acid or the cell for use according to embodiment 80, wherein the hematological cancer is selected from the group consisting of non-Hodgkin's lymphoma (NHL), Hodgkin's lymphoma (HL), multiple myeloma, acute myeloid leukemia (AML), acute lymphoblastic leukemia (ALL), mixed phenotype acute leukemia (MPAL), chronic myeloid leukemia (CML), B cell polymorphic lymphoma, hairy cell leukemia, chronic lymphocytic leukemia (CLL), small lymphocytic lymphoma (SLL), central nervous system lymphoma, CD37+ dendritic cell lymphoma, lymphoplasmatic lymphoma, splenic marginal zone lymphoma, plasma cell myeloma, extraosseuos plasmacytoma, extra-nodal marginal zone B-cell lymphoma of mucosa-associated lymphoid tissue (MALT tissue), nodal marginal zone B-cell lymphoma, follicular lymphoma, mantel cell lymphoma, diffuse large B-cell lymphoma, mediastinal (thymic) large B-cell lymphoma, precursor B-lymphoblastic lymphoma, immunoblastic large cell lymphoma, intravascular large B-cell lymphoma, primary effusion lymphoma, Burkitt's lymphoma/leukemia, B-cell proliferations of uncertain malignant potential, lymphomatoid granulomatosis, post-transplant lymphoproliferative disorder and myelodysplastic disorder.
Embodiment 87: Method of modifying a TCR or a fragment thereof comprising the constant region of the TCR, the method comprising introduction, into the constant region, of at least one epitope tag, wherein the insert position of the epitope tag is located in the alpha constant region (TRAC) and/or the beta constant region (TRBC).
Embodiment 88. Method according to embodiment 87, wherein the TCR is isolated and/or purified, possibly soluble or membrane bound.
Embodiment 89. Method according to embodiment 87 or 88, wherein the insert position of the at least one epitope tag is comprised in the alpha constant region (TRAC) and the beta constant region (TRBC).
Embodiment 90. Method according to any one of embodiments 87 or 88, wherein the insert position of the at least one epitope tag is comprised in the alpha constant region (TRAC) or the beta constant region (TRBC).
Embodiment 91. Method according to any one of embodiments 87 to 90, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and/or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and/or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 92. Method according to any one of embodiments 87 to 91, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; or
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 93. Method according to any one of embodiments 87 to 91, wherein
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and
   - the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.
Embodiment 94. Method according to any one of embodiments 87 to 93, wherein
   - the insert position is located at any position within the amino acid sequence set forth in SEQ ID NO: 145 and is located within the alpha constant region of the TCR; and/or
   - the insert position is located at any position within the amino acid sequence set forth in SEQ ID NO: 146 and is located within the beta constant region of the TCR; and/or
   - the insert position is located at any position within the amino acid sequence set forth in SEQ ID NO: 147 and is located within the beta constant region of the TCR.
Embodiment 95. Method according to embodiment 94, wherein
   - the insert position is located at any position within the amino acid sequence set forth in SEQ ID NO: 145 and is located within the alpha constant region of the TCR; or
   - the insert position is located at any position within the amino acid sequence set forth in SEQ ID NO: 146 and is located within the beta constant region of the TCR; or
   - the insert position is located at any position within the amino acid sequence set forth in SEQ ID NO: 147 and is located within the beta constant region of the TCR.
Embodiment 96. Method according to embodiment 94, wherein
   - the insert position is located at any position within the amino acid sequence set forth in SEQ ID NO: 145 and is located within the alpha constant region of the TCR; and
   - the insert position is located at any position within the amino acid sequence set forth in SEQ ID NO: 146 and is located within the beta constant region of the TCR; and
   - the insert position is located at any position within the amino acid sequence set forth in SEQ ID NO: 147 and is located within the beta constant region of the TCR.
Embodiment 97. Method according to any one of embodiments 87 to 96, wherein the insertion position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 148 and/or
   - followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 149.
Embodiment 98. Method according to any one of embodiments 87 to 97, wherein the insertion position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 150 and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151.
Embodiment 99. Method according to any one of embodiments 87 to 98, wherein the insertion position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 152 and/or
   - followed by an amino acid sequence comprising or consisting of an amino acid sequence having at least 80 %, preferably at least 85 %, more preferably at least 90 %, such as about 100 % sequence identity to the amino acid sequence set forth in SEQ ID NO: 153.
Embodiment 100. Method according to any one of embodiments 97 to 99, wherein the amino acid sequence that is the preceded by an amino acid sequence as defined in embodiments 97 to 99 is from 12 to 20, preferably from 14 to 18, such as 14, 15, 16, 17, or 18 amino acids of length, most preferably 14, 15, or 18 amino acids of length.
Embodiment 101. Method according to any one of embodiments 92 to 95, wherein the amino acid sequence that is followed by an amino acid sequence as defined in embodiments 97 to 99 is from 12 to 20, preferably from 14 to 18, such as 14, 15, 16, 17, or 18 amino acids of length, most preferably 14, 15, or 18 amino acids of length.
Embodiment 102. Method according to embodiment 100 or 101, wherein the insertion position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 148, wherein the amino acid sequence that is preceded is 12 to 16 amino acids of length, preferably 14 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 149, wherein the amino acid sequence that is followed is 12 to 16 amino acids of length, preferably 14 amino acids of length.
Embodiment 103. Method according to any one of embodiments 100 to 102, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 150, wherein the amino acid sequence that is preceded and/or followed is 13 to 17 amino acids of length, preferably 15 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151, wherein the amino acid sequence that is followed is 13 to 17 amino acids of length, preferably 15 amino acids of length.
Embodiment 104. Method according to any one of embodiments 100 to 103, wherein the insert position is located within an amino acid sequence that is
   - preceded by an amino acid sequence comprising or consisting of an amino acid sequence having at least 80 %, preferably at least 85 %, more preferably at least 90 % sequence identity to the amino acid sequence set forth in SEQ ID NO: 152, wherein the amino acid sequence that is preceded is 16 to 20 amino acids of length, preferably 18 amino acids of length; and/or
   - followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 153, wherein the amino acid sequence that is followed is 16 to 20 amino acids of length, preferably 18 amino acids of length.
Embodiment 105. Method according to any one of embodiments 87 to 104, wherein the insert position is located
   - within the amino acid sequence ranging from positions 190 to 204, preferably positions 192 to 203 of the TCR beta chain of the TCR beta chain; and/or
   - within the amino acid sequence ranging from positions 232 to 249, preferably positions 234 to 247 of the TCR beta chain; and/or
   - within the amino acid sequence ranging from positions 205 to 218, preferably positions 207 to 215 of the TCR alpha chain.
Embodiment 106. Method according to embodiment 105, wherein the insert position is located
   - within the amino acid sequence ranging from positions 190 to 204, preferably positions 192 to 203 of the TCR beta chain of the TCR beta chain; and
   - within the amino acid sequence ranging from positions 232 to 249, preferably positions 234 to 247 of the TCR beta chain; and
   - within the amino acid sequence ranging from positions 205 to 218, preferably positions 207 to 215 of the TCR alpha chain.
Embodiment 107. Method according to embodiment 105, wherein the insert position is located
   - within the amino acid sequence ranging from positions 190 to 204, preferably positions 192 to 203 of the TCR beta chain of the TCR beta chain; or
   - within the amino acid sequence ranging from positions 232 to 249, preferably positions 234 to 247 of the TCR beta chain; or
   - within the amino acid sequence ranging from positions 205 to 218, preferably positions 207 to 215 of the TCR alpha chain.
Embodiment 108. Method according to embodiment 105, wherein the insert position is located
   - within the amino acid sequence ranging from positions 196 to 202 of the TCR beta chain; and/or
   - within the amino acid sequence ranging from positions 236 to 245 of the TCR beta chain; and/or
   - within the amino acid sequence ranging from positions 209 to 213 of the TCR alpha chain.
Embodiment 109. Method according to embodiment 108, wherein the insert position is located
   - within the amino acid sequence ranging from positions 196 to 202 of the TCR beta chain; and
   - within the amino acid sequence ranging from positions 236 to 245 of the TCR beta chain; and
   - within the amino acid sequence ranging from positions 209 to 213 of the TCR alpha chain.
Embodiment 110. Method according to embodiment 108, wherein the insert position is located
   - within the amino acid sequence ranging from positions 196 to 202 of the TCR beta chain; or
   - within the amino acid sequence ranging from positions 236 to 245 of the TCR beta chain; or
   - within the amino acid sequence ranging from positions 209 to 213 of the TCR alpha chain.
Embodiment 111. Method according to embodiment 105, wherein the insert position is located at
   - a position selected from the group consisting of positions 196, 198, 199, 200, and 202 of the TCR beta chain; and/or
   - a position selected from the group consisting of positions 236, 238, 239, 240, 243, and 245 of the TCR beta chain; and/or
   - a position selected from the group consisting of positions 209, 211, and 213 of the TCR alpha chain.
Embodiment 112. Method according to embodiment 111, wherein the insert position is located at
   - a position selected from the group consisting of positions 196, 198, 199, 200, and 202 of the TCR beta chain; and
   - a position selected from the group consisting of positions 236, 238, 239, 240, 243, and 245 of the TCR beta chain; and
   - a position selected from the group consisting of positions 209, 211, and 213 of the TCR alpha chain.
Embodiment 113. Method according to embodiment 111, wherein the insert position is located at
   - a position selected from the group consisting of positions 196, 198, 199, 200, and 202 of the TCR beta chain; or
   - a position selected from the group consisting of positions 236, 238, 239, 240, 243, and 245 of the TCR beta chain; or
   - a position selected from the group consisting of positions 209, 211, and 213 of the TCR alpha chain.
Embodiment 114. Method according to any one of embodiments 87 to 113, wherein the epitope tag is inserted by means of linker sequences, preferably short glycine-rich linker sequences, more preferably glycine-rich linker sequences consisting of 2 to 6, preferably 3 to 5 amino acids, more preferably comprising or consisting of the amino acid sequence GSG.
Embodiment 115. Method according to any one of embodiments 87 to 114, wherein the epitope tag comprises 4 to 17 amino acids, preferably 5 to 15 amino acids, more preferably 6 to 14 amino acids.
Embodiment 116. Method according to any one of embodiments 87 to 115, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 155, SEQ ID NO: 1, SEQ ID NO: 2, SEQ ID NO: 3, and SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 159, SEQ ID NO: 5, SEQ ID NO: 6, SEQ ID NO: 7, and SEQ ID NO: 8 of the TCR beta constant region.
Embodiment 117. Method according to embodiment 116, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequence set forth in SEQ ID NO: 155 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 159 of the TCR beta constant region.
Embodiment 118. Method according to embodiment 116, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 1 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 5 of the TCR beta constant region.
Embodiment 119. Method according to embodiment 116, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 2 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 6 of the TCR beta constant region.
Embodiment 120. Method according to embodiment 116, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 3 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 7 of the TCR beta constant region.
Embodiment 121. Method according to embodiment 116, wherein the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 8 of the TCR beta constant region.
Embodiment 122. Method according to any one of embodiments 87 to 121, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 156, SEQ ID NO: 9, SEQ ID NO: 157, SEQ ID NO: 10, SEQ ID NO: 158, and SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 160, SEQ ID NO: 12, SEQ ID NO: 161, SEQ ID NO: 13, SEQ ID NO: 162, and SEQ ID NO: 14 of the TCR beta constant region.
Embodiment 123. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 156 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 160 of the TCR beta constant region.
Embodiment 124. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 9 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 12 of the TCR beta constant region.
Embodiment 125. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 157 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 161 of the TCR beta constant region.
Embodiment 126. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 10 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 13 of the TCR beta constant region.
Embodiment 127. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 158 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequence set forth in SEQ ID NO: 162 of the TCR beta constant region.
Embodiment 128. Method according to embodiment 122, wherein the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 14 of the TCR beta constant region.
Embodiment 129. Method according to any one of embodiments 87 to 128, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15, SEQ ID NO: 16, and SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18, SEQ ID NO: 19, and SEQ ID NO: 20 of the TCR alpha constant region.
Embodiment 130. Method according to embodiment 129, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18 of the TCR alpha constant region.
Embodiment 131. Method according to embodiment 129, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 16 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 19 of the TCR alpha constant region.
Embodiment 132. Method according to embodiment 129, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 20 of the TCR alpha constant region.
Embodiment 133. Method according to any one of embodiments 87 to 132, wherein the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 21 and SEQ ID NO: 22 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 23 and SEQ ID NO: 24 of the TCR alpha constant region.
Embodiment 134. Method according to any one of embodiments 87 to 133, wherein the epitope tag comprises an amino acid sequence having at least 80 %, preferably at least 85 %, more preferably at least 90 %, for instance 100 % sequence identity to the amino acid sequence set forth in SEQ ID NO: 25 (EVPKX₁R), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 135. Method according to any one of embodiments 87 to 134, wherein the epitope tag comprises an amino acid sequence having at least 80 %, preferably at least 85 %, more preferably at least 90 %, for instance 100 % sequence identity to the amino acid sequence set forth in SEQ ID NO: 26 (GEVPKX₁RFS), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 136. Method according to any one of embodiments 87 to 135, wherein the epitope tag consists of an amino acid sequence having at least 80 %, preferably at least 85 %, more preferably at least 90 %, for instance 100 % sequence identity to the amino acid sequence set forth in SEQ ID NO: 26 (GEVPKX₁RFS), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V).
Embodiment 137. Method according to any one of embodiments 87 to 136, wherein the epitope tag comprises or consists of the amino acid sequence set forth in SEQ ID NO: 27 (X₂X₃GEVPKX₁RFSX₄), wherein X₁ is selected from Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V); X₂ is selected from aspartate (D) and glutamate (E); X₃ is selected from lysine (K), arginine (R), and histidine (H); and X₄ is selected from glycine (G), leucine (L), alanine (A), valine (V), methionine (M), isoleucine (I), serine (S), and threonine (T).
Embodiment 138. Method according to any one of embodiments 87 to 137, wherein the epitope tag comprises an amino acid sequence selected from:
   - DX₃GEVPKX₁RFSX₄ (SEQ ID NO: 28);
   - EX₃GEVPKX₁RFSX₄ (SEQ ID NO: 29);
   - X₂KGEVPKX₁RFSX₄ (SEQ ID NO: 30);
   - X₂RGEVPKX₁RFSX₄ (SEQ ID NO: 31);
   - X₂HGEVPKX₁RFSX₄ (SEQ ID NO: 32);
   - X₂X₃GEVPKX₁RFSG (SEQ ID NO: 33);
   - X₂X₃GEVPKX₁RFSL (SEQ ID NO: 34);
   - X₂X₃GEVPKX₁RFSA (SEQ ID NO: 35);
   - X₂X₃GEVPKX₁RFSV (SEQ ID NO: 36;
   - X₂X₃GEVPKX₁RFSM (SEQ ID NO: 37);
   - X₂X₃GEVPKX₁RFSI (SEQ ID NO: 38);
   - X₂X₃GEVPKX₁RFSS (SEQ ID NO: 39);
   - X₂X₃GEVPKX₁RFST (SEQ ID NO: 40),
   wherein, where applicable, wherein X₁ is selected from A, R, N, D, C, Q, E, G, H, I, L, K, M, F, P, S, T, W, Y, V; X₂ is selected from D and E; X₃ is selected from K, R, and H; and X₄ is selected from G, L, A, V, M, I, S, and T.
Embodiment 139. Method according to any one of embodiments 87 to 138, wherein the epitope tag comprises an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52 or wherein the epitope tag comprises an amino acid sequence having at least about 60 %, for instance at least about 66 % or about 67 %, preferably at least about 80 %, such as about 83 % or about 84 %, preferably at least 90 %, for instance 100 % sequence identity to the amino acid sequence set forth in SEQ ID NO: 154.
Embodiment 140. Method according to any one of embodiments 87 to 139, wherein the epitope tag consists of an amino acid sequence having at least 80 %, preferably at least about 81, 82, 83, 84 or 85 %, for instance at least about 88 % or about 89 %, more preferably at least about 90 %, such as about 91 % or 92 %, for instance 100 % sequence identity to an amino acid sequence selected from the group consisting of SEQ ID NO 47, SEQ ID NO: 48, SEQ ID NO: 49, SEQ ID NO: 50, SEQ ID NO: 51, and SEQ ID NO: 52 or wherein the epitope tag comprises an amino acid sequence having at least about 60 %, for instance at least about 66 % or about 67 %, preferably at least about 80 %, such as about 83 % or about 84 %, preferably at least 90 %, for instance 100 % sequence identity to the amino acid sequence set forth in SEQ ID NO: 154.
Embodiment 141. Method according to any one of embodiments 87 to 133, wherein the epitope tag comprises an amino acid sequence having at least 80 %, preferably at least 85 %, more preferably at least 90 %, for instance 100 % sequence identity to an amino acid sequence selected from the group consisting of SEQ ID NO: 53.
Embodiment 142. TCR according to embodiment 141, wherein the epitope tag consists of an amino acid sequence having at least 80 %, preferably at least 85 %, more preferably at least 90 %, for instance 100 % sequence identity to an amino acid sequence selected from the group consisting of SEQ ID NO: 53.
Embodiment 143. Method according to any one of embodiments 87 to 142, wherein integration of the epitope tag does not interfere with structural integrity and/or functionality of the TCR.
Embodiment 144. Method according to any one of embodiments 87 to 143, wherein the method further comprises at least one step of modifying the amino acid sequence of the TCR for the TCR to comprise a detectable label, a therapeutic agent or pharmacokinetic modifying moiety.
Embodiment 145. Method according to embodiment 144, wherein the therapeutic agent is selected from the group consisting of an immune effector molecule, a cytotoxic agent and a radionuclide.
Embodiment 146. Method according to embodiment 145, wherein the immune effector molecule is a cytokine.
Embodiment 147. Method according to embodiment 144, wherein the pharmacokinetic modifying moiety is at least one polyethylene glycol repeating unit, at least one glycol group, at least one sialyl group or a combination thereof.
Embodiment 148. Method according to any one of embodiments 87 to 147, wherein the TCR is of the single chain type, wherein the TCR α chain and the TCR β chain are linked by a linker sequence.
Embodiment 149. Method according to any one of embodiments 87 to 148, wherein the method comprises at least one step of modifying the TCR, such that the recombinant TCR sequence may contain minimal murinized Cα and Cβ regions.

## Claims

1. T cell receptor (TCR) or a fragment thereof comprising the constant region of the TCR, and further comprising, in said constant region, at least one epitope tag, wherein the insert position of the epitope tag is located in the alpha constant region (TRAC) and/or the beta constant region (TRBC).

2. The TCR according to claim 1, wherein
- the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 145 and is located within the alpha constant region of the TCR; and/or
- the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 146 and is located within the beta constant region of the TCR; and/or
- the insert position is located at any position within an amino acid sequence having at least 80, preferably at least 85, more preferably at least 90, even more preferably at least 95, such as at least 96, 97, 98, 99 or 100 % sequence identity to the amino acid sequence set forth in SEQ ID NO: 147 and is located within the beta constant region of the TCR.

3. The TCR according to claim 1 or claim 2, wherein
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 148 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 149; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 150 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 151; and/or
- the insertion position is located within an amino acid sequence that is preceded by an amino acid sequence comprising or consisting of an amino acid sequence having at least 80 %, preferably at least 85 %, more preferably at least 90 % sequence identity to the amino acid sequence set forth in SEQ ID NO: 152 and/or followed by an amino acid sequence comprising or consisting of an amino acid sequence having at least 80 %, preferably at least 85 %, more preferably at least 90 %, such as about 100 % sequence identity to the amino acid sequences set forth in SEQ ID NO: 153.

4. The TCR according to any one of the preceding claims, wherein
- the epitope tag is preceded by an amino acid sequence comprising or consisting of an amino acid sequence having at least 80 %, preferably at least 85 %, more preferably at least 90 % sequence identity to an amino acid sequence selected from the amino acid sequences set forth in SEQ ID NO: 1, SEQ ID NO: 2, SEQ ID NO: 3, and SEQ ID NO: 4 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 5, SEQ ID NO: 6, SEQ ID NO: 7, and SEQ ID NO: 8 of the TCR beta constant region; and/or
- the epitope tag is preceded by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 9, SEQ ID NO: 10, and SEQ ID NO: 11 and/or followed by an amino acid sequence comprising or consisting of an amino acid sequence having at least 80 %, preferably at least 85 %, more preferably at least 90 %, such as about 100 % sequence identity to an amino acid sequence selected from the amino acid sequences set forth in SEQ ID NO: 12, SEQ ID NO: 13, and SEQ ID NO: 14 of the TCR beta constant region; and/or
- the epitope tag is preceded by an amino acid sequence comprising or consisting of an amino acid sequence having at least 80 %, preferably at least 85 %, more preferably at least 90 % sequence identity an amino acid sequence selected from the amino acid sequences set forth in SEQ ID NO: 15, SEQ ID NO: 16, and SEQ ID NO: 17 and/or followed by an amino acid sequence comprising or consisting of an amino acid sequence having at least 80 %, preferably at least 85 %, more preferably at least 90 %, such as about 100 % sequence identity an amino acid sequence selected from the amino acid sequences set forth in SEQ ID NO: 18, SEQ ID NO: 19, and SEQ ID NO: 20 of the TCR alpha constant region.

5. The TCR or fragment thereof according to any one of the preceding claims, wherein
- the epitope tag is inserted by means of linker sequences, preferably short glycine-rich linker sequences, more preferably glycine-rich linker sequences consisting of 2 to 6, preferably 3 to 5 amino acids, more preferably comprising or consisting of the amino acid sequence GSG; and/or
- the epitope tag comprises 4 to 17 amino acids, preferably 5 to 15 amino acids, more preferably 6 to 14 amino acids; and/or
- integration of the epitope tag does not interfere with structural integrity and/or functionality of the TCR; and/or
- the epitope tag, which can be recognized by a tagging antibody specific for the epitope tag, comprises an amino acid sequence having at least 80 %, preferably at least 85 %, more preferably at least 90 % sequence identity to an amino acid sequence selected from the group consisting of SEQ ID NO 47-53 and 154.

6. Isolated polypeptide comprising at least one epitope tag and a functional portion of the TCR or fragment thereof of any of claims 1 to 5.

7. Multivalent TCR complex, comprising a least two TCRs as claimed in any one of claims 1 to 5.

8. Nucleic acid encoding a TCR or fragment thereof according to any one of claims 1 to 5 or encoding the polypeptide according to claim 6.

9. Vector comprising the nucleic acid of claim 8.

10. Cell expressing the TCR or fragment thereof according to claims 1 to 5.

11. The TCR or fragment thereof according to claims 1 to 5, the polypeptide according to claim 6, the multivalent TCR complex according to claim 7, the nucleic acid according to claim 8, the vector according to claim 9, or the cell according to claim 10 for use as a medicament.

12. The TCR or fragment thereof according to claims 1 to 5, the polypeptide according to claim 6, the multivalent TCR complex according to claim 7, the nucleic acid according to claim 8, the vector according to claim 9, or the cell according to claim 10 for use as screening agent.

13. The TCR or fragment thereof according to claims 1 to 5, the polypeptide according to claim 6, the multivalent TCR complex according to claim 7, the nucleic acid according to claim 8, the vector according to claim 9, or the cell according to claim 10 for use in the treatment of cancer.

14. Pharmaceutical composition comprising at least one TCR or fragment thereof according to claims 1 to 5, the polypeptide according to claim 6, the multivalent TCR complex according to claim 7, the nucleic acid according to claim 8, the vector according to claim 9, or the cell according to claim 10.

15. Method for modifying a TCR or a fragment thereof comprising the constant region of the TCR, the method comprising introduction, into the constant region, of at least one epitope tag, wherein the insert position of the epitope tag is located in the alpha constant region (TRAC) and/or the beta constant region (TRBC).
